(19) Europäisches Patentamt
European Patent Office
Office européen des brevets

(11) EP 1 088 233 B1

(12) EUROPEAN PATENT SPECIFICATION

(45) Date of publication and mention of the grant of the patent: **09.11.2005 Bulletin 2005/45**

(21) Application number: **99957096.3**

(22) Date of filing: **16.06.1999**

(51) Int Cl.[7]: **G01N 33/58**, C12Q 1/66, G01N 33/50, C12N 15/62, G01N 33/542

(86) International application number: **PCT/CA1999/000561**

(87) International publication number: **WO 1999/066324 (23.12.1999 Gazette 1999/51)**

# (54) A BIOLUMINESCENCE RESONANCE ENERGY TRANSFER (BRET) SYSTEM AND ITS USE

BIOLUMINESZENTES RESONANZ-ENERGIE-TRANSFERSYSTEM (BRET) UND SEINE ANWENDUNG

SYSTEME DE TRANSFERT D'ENERGIE DE RESONANCE PAR BIOLUMINESCENCE ET UTILISATION DUDIT SYSTEME

(84) Designated Contracting States:
**CH DE FR GB IE LI NL SE**

(30) Priority: **16.06.1998 US 89623 P**

(43) Date of publication of application:
**04.04.2001 Bulletin 2001/14**

(73) Proprietors:
- **Biosignal Packard Inc.**
  **Montreal, Quebec H3J 1R4 (CA)**
- **VANDERBILT UNIVERSITY**
  **Nashville, TN 37212 (US)**

(72) Inventors:
- **JOLY, Erik**
  **Blainville, Quebec J7C 4R7 (CA)**
- **JOHNSON, Carl, H.**
  **Nashville, TN 37204 (US)**
- **PISTON, David, W.**
  **Nashville, TN 37205 (US)**

(74) Representative: **Bassil, Nicholas Charles et al**
**Kilburn & Strode**
**20 Red Lion Street**
**London WC1R 4PJ (GB)**

(56) References cited:
- **WU P ET AL: "RESONANCE ENERGY TRANSFER: METHODS AND APPLICATIONS" ANALYTICAL BIOCHEMISTRY,US,ACADEMIC PRESS, SAN DIEGO, CA, vol. 218, page 1-13 XP002033685 ISSN: 0003-2697 cited in the application**
- **XU, YAO ET AL: "A bioluminescence resonance energy transfer ( BRET ) system: application to interacting circadian clock proteins" PROC. NATL. ACAD. SCI. U. S. A. (1999), 96(1), 151-156 , XP002127270 cited in the application**
- **KOJIMA S ET AL: "Mechanism of the Redox Reaction of the Aequorea Green Fluorescent Protein (GFP)" TETRAHEDRON LETTERS,NL,ELSEVIER SCIENCE PUBLISHERS, AMSTERDAM, vol. 38, no. 16, page 2875-2878 XP004058517 ISSN: 0040-4039**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 088 233 B1

Printed by Jouve, 75001 PARIS (FR)

## Description

### FIELD OF THE INVENTION

This invention pertains to the field of molecular interactions.

### BACKGROUND OF THE INVENTION

Interactions between proteins andothermolecules play a key regulatory role in almost every bio logical process. Thus, many techniques havebeen developed to identify and characterize these interactions. These tools range from in vitro binding assays to library-based methods and include genetic methods such as searching for extragenic suppressors (Phizicky, E. M. & Fields, S., (1995) *Microbiol. Rev.,* **59**,94-123). Currently, the most popular library-based method is the yeast two-hybrid system, which allows the selection of yeast strains that contain two interacting peptides, the target peptide and another from the library (Fields, S. & Song. O.K., (1989) *Nature (London)* **340**, 245-246). This method has proven itself to be a valuable technique for identifying potential protein interactions, but it has several liabilities, foremost of which is that the interaction is tested only in the nucleus of a yeast cell. This means interactions that depend upon cell-type specific processing and/or compartmentalization will not be detected by the two-hybrid system.

Another technique for assessing protein-protein interaction is based on fluorescence resonance energy transfer (FRET). In this process, one fluorophore (the "donor") transfers its excited-state energy to anotherfluorophore (the "acceptor") which usually emits fluorescence of a different color. FRET efficiency depends on three parameters: (i) the overlap between the absorption spectrum of the second fluorphore and the emission spectrum of the first fluorophore, (ii) the relative orientation between the emission dipole of the donor and the absorption dipole of the acceptor, and (iii) the distance between the fluorophores. Depending on the spectral overlap and dipole orientations, the distance over which FRET occurs can be between 10-100 Å (Wu, P. & Brand, L., (1994) *Anal. Biochem.,* **218**, 1-13). FRET has been used to assay protein-protein proximity in vitro and in vivo by chemically attaching fluorophores such as fluorescein and rhodamine to pairs of purified proteins and measuring fluorescence spectra of protein mixtures or cells that were microin jected with the labeled proteins (Adams, S. R. *et al*., (1991) *Nature*, **349,** 694-7). The cloning and expression of Green Fluorescent Protein (GFP) in heterologous systems opened the possibility of genetic attachment of fluorophores to proteins. In addition, the availability of GFP mutants with altered wavelengths (Heim *et al*., (1994) *Proc. Natl. Acad. Sci. USA*., **91,** 12501-12504) allowed their use as FRET pairs. For example, GFP and its blue-fluorescent mutant, BFP were first used to demonstrate FRET between GFP molecules (Heim, R. & Tsien, R. Y., (1996) *Curr. Biol.,* **6**, 178-182). Other GFP pairs such as the cyan CFP, and yellow-green YFP considerably extended the applications in which FRET can be used to measure protein-protein interactions (Miyawaki *et al*., (1997) *Nature* (*London*), **388**, 882-887).

An attractive application allowed by GFP-based FRET is the in vivo assay of protein interactions in organisms other than yeast. For example, fusion of GFP and BFP to the mammalian transcriptional factor Pit-1, showed homodimerization of Pit-1 in live HeLa cells (Penasamy, A. and Day, R. N., (1998) *J. Biamed. Opt*., **3**, 1-7). In this type of assay, interactions can be examined in the proteins' native organism, such that cell-type specific modifications and/or compartmentalization of the proteins are preserved. Additionally, compartmentalization of these interacting proteins is potentially visible in the microscope. FRET, however, has several limitations. As with any fluorescence technique, photobleaching of the fluorophore and autofluorescence of the cells/tissue can significantly restrict the usefulness of FRET, and in highly autofluorescent tissues, FRET is essentially unusable. Also, if the tissue is easily damaged by the excitation light, the technique may be unable to give a value for healthy cells. Finally, if the cells/tissues to be tested are photoresponsive (e.g., retina), FRET may be impractical because as soon as a measurement is taken, the photoresponse may be triggered.

European Patent Application No. EP 0 070 686 provides a non-radiative energy transfer immunochemical technique in which antigen assays were performed by introducing an antigen-containing sample into a reagent solution comprising an absorber-emitter conjugated antibody and a chemiluminescent catalyst conjugated antibody, wherein the conjugated antibodies bound to the antigen in close proximity to enable transfer of energy to the absorber-emitter. The resulting emission of light from the absorber-emitter is related to the amount of antigen present in the sample.

Green Fluorescent Protein (GFP) is a light emitting protein purified from the jellyfish Aequorea victoria. GFP can emit green light by accepting energy transfer from sources that include exogenous blue light and Renilla luciferase catalyzed reactions. The gene for GFP was cloned and its cDNA is a powerful reporter gene in a variety of living systems, including bacteria, fungi, and mammalian tissues. The UV light stimulated GFP fluorescence does not require cofactors and the gene product alone can be sufficient to allow detection of living cells under the light microscope.

Renilla luciferase is an enzyme purified from Renilla reniformis. The enzyme catalyzes the oxidative decarboxylation of coelenterazine in the presence of oxygen to produce blue light with an emission wavelength maximum of 478 nm. In Renilla reniformis cells, however, this reaction is shifted toward the green with a wavelength maximum of 510 nm due to an energy transfer to a Green Fluorescent Protein.

The gene for Renilla luciferase (ruc) was cloned and its cDNA was shown to be useful as a reporter gene in various living systems. D. C. Prasher, *et al*. Gene 1992; 111:229-33. By providing appropriate promoters to the cDNA as gene cassettes, the gene was expressed in bacteria, transformed plant cells, and mammalian cells. The high efficiency of Renilla luciferase is a useful trait as a marker enzyme for gene expression studies. International patent application WO 98/14605 discloses fusion gene constructs comprising the cDNA of Renilla luciferase and the cDNA of the "humanized" Aequorea green fluorescent protein.

A bioluminescence resonance energy transfer (BRET) system and its application to interacting circadian clock proteins has been described in Yao *et al*, Proc. Natl. Acad. Sci. USA (1999),151-156.

## SUMMARY OF THE INVENTION

This invention provides a bioluminescence resonance energy transfer (BRET) system that comprises four parts: 1) a bioluminescent protein that has luciferase activity; 2) an acceptor fluorophore that can accept the energy from the bioluminescent protein when they are associated, in the presence of the appropriate substrate; 3) a modulator that influences the proximity or the orientation of the bioluminescent protein and the fluorophore, and 4) an appropriate substrate to activate the luciferase activity of the bioluminescent protein. The components of this system interact to influence the spatial relationship between the bioluminescent protein and the fluorophore, that is demonstrated by the light emission from the system.

## BRIEF DESCRIPTION OF THE FIGURES

Figure 1 is a pictorial description of a BRET system wherein the luciferase and the fluorophore are attached to different proteins or peptides.

Figure 2 is a pictorial description of a BRET wherein the luciferase and the fluorophore are attached to the same protein or peptide.

Figure3 shows *in vivo* bioluminescence resonance energy transfer (BRET) ofRLUC in E. coli cells expressing the RLUC•EYFP fusion protein. (a) Diagrams of the expression cassettes of pT7/Eyfp, pT7/Rluc, and pT7/Rluc•Eyfp. Abbreviations:$P_{T7}$ =T7 promoter region; Ter = transcription terminator region. Antibiotic resistances are indicated within parentheses. The linker sequence between Rluc and Eyfp in the Rluc•Eyfp fusion construct is shown below the pT7/Rluc•Eyfp diagram. (b) Luminescence (1um.) or fluorescence (fluor.) emission spectra from transformed strains expressing the proteins RLUC or EYFP or the fusion protein RLUC•EYFP. All spectra were normalized.

Figure 4 demonstrates an *in vivo* BRET assay for circadian clock protein interaction. (a) Diagrams of the gene fusion expression cassettes of pT7/Eyfp•kaiB, pT7/Rluc•kaiB, and pT7/Eyfp•kaiA. (b) Luminescence emission spectra from the transformed strains expressing RLUC & EYFP (b1), RLUC•KaiB & EYFP•KaiB (b2), and RLUC•KaiB & EYFP•KaiA (b3).

Figure 5 shows clock protein KaiB-KaiB association in vitro revealed by BRET. Extracts containing fusion proteins EYFP•KaiB were mixed in equal proportions with those containing RLUC•KaiB. Bioluminescence emission profiles were measured at the indicated times after the extracts were combined and incubated at room temperature.

Figure 6 demonstrates imaging of biolummescence from transformed E. coli strains through interference bandpass filters transmitting light of 480 ± 5 nm (left panels) or 530 ± 5 nm (right panels). Row (a) is the strain producing only RLUC: row (b) is the strain expressing the RLUC•EYFP fusion protein; row (c) is the strain expressing the fusion proteins RLUC•KaiB and EYFP•KaiA; and row (d) is the strain producing the fusion proteins RLUC•KaiB and EYFP•KaiB. (Blue color is pseudo-color indicating the intensity of the light emission; it does not indicate the actual color of the light emitted).

Figure 7 shows quantification of BRET intensity and ratios in the images from Figure 6. Upper panel, quantification of luminescence intensity (in relative light units) at 480 nm and 530 nm of the cultures imaged in Figure 6. Bottompanel, calculation of the 530 nm/480 nm ratios. Strains are labelled in Figure 6: (a) RLUC, (b) RLUC•EYFP, (c) RLUC•KaiB and EYFP•KaiA, and (d) RLUC•KaiB and EYFP•KaiB.

Figure 8 shows the results of an *in vitro* enterokinase assay using the B RET Count, demonstrating that only the ratio for the construct Rluc:enterokinase:EYFP decreases over time in the presence of the enterokinase enzyme.

Figure 9 represents the timecourse of interaction of the different kai proteins at 25 °C using the BRET Count. Only the ratio for the Rluc-kaiB EYFP-kaiB increase over time for both transfection condition (using 4 or 0.5μg vector DNA per well). Ratios for all the negative controls Rluc-kaiB and Rluc-kaiB +EYFP-kaiA , do not increase with time. This means that energy transfer occurs only for the Rluc-kaiB+EYFP-kaiB which is as expected since kaiB binds only with itself and not with kaiA.

Figure 10 depicts a timecourse of induction of CAMP sensor by calcitonin and forskolin.

Figure 11 shows a dose responseof calcitoninusing CHO-K1 cell line transfected with CAMP sensor.

Figure 12 shows a timecourse of induction of $\beta_2$ receptor by isoproterenol.

Figure 13 shows a dose response of isoproterenol on the cells transfected with cAMP sensor CBP:CREB. Data where taking at 45 minutes after addition of the isoproterenol and the coelenterazine.

Figure 14 demonstrates the results of a timecourseexperimenton the activation of CCR5 receptorusing forskolin and MIP-1β, a selective agonist for CCR5.

Figure 15 demonstrates the results of a time course experiment on the activation of CCR5 receptor using forskolin and MIP-1β.

Figure 16 shows an EGF dose response on the GH4-C1 cells transfected with the TKR sensor.

Figure 17 depicts a timecourse experiment on cells transfected with the different β2 constructs.

Figure 18 shows a comparison of the BRET ratio at 20 minutes after addition of coelenterazine.

Figure 19 presents the results of experiments demonstrating the kinetics of β2 dimerization.

Figure 20 shows a dose response analysis using cells transfected with the different β2 constructs.

Figure 21 shows the results of a BRET assay performed using the BRETCount.

Figure 22 presents a spectral analysis of BRETreaction at different time points after coelenterazine addition using the Spex. The Y axis represents the CPS and X axis represents the wavelength (nm).

Figure 23 demonstrates the effect of cell number per well. (cont (+) represents cells transformed with fusion Rluc•EYSP gene; cont (-) represents cells transformed with Rluc gene alone).

Figure 24 shows the results of experiments determining Rluc luminescence in PBS Ca2+/Mg2+ in the presence or absence of DTT.

Figure 25 shows the results of experiments determining Rluc luminescence in PBS Ca2+/Mg2++ glucose in the presence or absence of DTT.

Figure 26 shows the results of experiments determining Rluc luminescence in DMEM without phenol red in the presence or absence of DTT.

Figure 27 demonstrates the effect of pH on the BRET ratio.

Figure 28 shows the addition of Hepes-based buffer on Rluc construct ratio.

Figure 29 shows the addition of Hepes-based buffer on Rluc::EYFP construct ratio.

Figure 30 shows the addition of DMSO on the energy transfer.

Figure 31 shows apoptosis induction using staurosporine in HeLa cells transfected with the apoptosis sensor.

Figure 32 shows results from the MiniScreen Assay

Figure 33 presents the results of an experiment demonstrating a calcitonin dose response on CHO-K1 cell transfected with the cAMP sensor (CBP:CREB constructs).

Figure 34 shows an isoproterenol dose response on CHO-K1 cell transfected with the cAMP sensor (CBP:CREB constructs). In this case, the ratio has been corrected using the alogrithm formula presented in Example XIV.

Figure 35 presents results from a cell linearity experiment.

Figure 36 shows results from a time-course study of Rluc luminescence.

Figure 37 shows a dose response of coelenterazine derivatives.

Figure 38 presents results from a study comparing lysed and non-lysed cells.

Figure 39 shows LucLite stabilization of Rluc luminescence in live cells.

Figure 40 presents results of a ratio stability study when using coelenterazine h in LucLite.

Figure 41 shows results of a ratio stability study when using native colenterazine in LucLite.

Figure 42 shows BRET detection in cells transfected with 10 times less DNA.

Figure 43 presents results demonstrating BRET detection in cells transfected with 20 times less DNA.

Figure 44 presents resultsof a study of BRET detection when cells are lysed. Transfections were done using the "normal" amount of DNA.

Figure 45 presents results of a comparison study between PBS and DMEM/F12 (without phenol red) for assaying BRET.

Figure 46 shows DTT effects on the Rluc emission stability.

Figure 47 demonstrates Rluc emission stability over time.

Figure 48 shows Rluc emission levels vs. cell number at fixed time.

Figure 49 presents Rluc emission stability in the presence of a fixed number of cells.

Figure 50 shows a demonstrationof Rluc emission levels vs. cell number atfixed times after the addition of coelenterazine.

Figure 51 shows a demonstration of the effect of temperature on the Rluc emission.

Figure 52 shows a demonstration of the effect of the temperature on the BRET ratio (LumiCount).

Figure 53 shows a demonstration of the effect of the temperature on the BRET ratio (BRETCount).

Figure 54 shows a demonstration of the effect of DTT on the Rluc emission.

Figure 55 shows a demonstration of the effect of DTT on the Rluc emission. And the BRET ratio (BRETCount). These results were obtained with the stable cell lines expressing Rluc only (negative control).

Figure 56 shows a demonstration of the effect of DTT on the Rluc emission. And the BRET ratio (BRETCount). These results were obtained with the stable cell lines expressing the fusion Rluc::EYFP (positive control).

Figure 57 shows the DNA sequence for the Rluc-EYFP construct (SEQ ID NO:26).

Figure 58 shows the DNA sequence for the Rluc-enterokinase-EYFP construct (SEQ ID NO:27).

Figure 59 shows the DNA sequence for the Rluc-caspase-EYFP construct (SEQ ID NO:28).

Figure 60 shows the DNA sequence for the EYSP-CREB construct (SEQ ID NO:29).

Figure 61 shows the DNA sequence for the RLUC-CBP construct (SEQ ID NO:30).

Figure 62 shows the DNA sequence for the EYFP-SH2 construct (SEQ ID NO:31).

Figure 63 shows the DNA sequence for the Rluc-PTB construct (SEQ ID NO:32).

## DETAILED DESCRIPTION OF THE INVENTION

Definitions

Unless defined otherwise, all technical and scientific terms used herein have the same meaning as commonly understood by one of ordinary skill in the art to which this invention belongs. Generally, the nomenclature used herein and the laboratory procedures in spectroscopy, drug discovery, cellculture, molecular genetics, plastic manufacture, polymer chemistry, diagnostics, amino acid and nucleic acid chemistry, and sugar chemistry described below are those well known and commonly employed in the art. Standard techniques are typically used for preparation of plastics, signal detection, recombinant nucleic acid methods, polynucleotide synthesis, and microbial culture and transformation (e.g., electroporation, lipofection).

The techniques and procedures are generally performed according to conventional methods in the art and various general references (see generally, Sambrook et al. Molecular Cloning: A Laboratory Manual, 2d ed. (1989) Cold Spring Harbor Laboratory Press, Cold Spring Harbor, N.Y., and Lakowicz, J. R. Principles of Fluorescence Spectroscopy, New York: Plenum Press (1983) for fluorescence techniques) which are provided throughout this document. Standard techniques are used for chemical syntheses, chemical analyses, and biological assays. As employed throughout the disclosure, the following terms, unless otherwise indicated, shall be understood to have the following meanings:

"Bioluminescent protein" refers to a form of chemiluminescence which arises as the result of an energy-yielding chemical reaction in which a specific biochemical substance, a luciferin (a naturally occuring fluorophore), is oxidized by an enzyme having a luciferase activity. A great diversity of organisms, both prokaryotic and eukaryotic, including species of bacteria, algae, fungi, insects, fish and other marine forms can emit light energy in this manner and each has specific luciferase activities and luciferins which are chemically distinct from those of other organisms. Luciferin/luciferase systems are very diverse in form, chemistry and function. For example, there are luciferase activities which facilitate continuous chemiluminescence, as exhibited by some bacteria and mushrooms, and those which are adapted to facilitate sporadic or stimuli induced emissions, as in the case of dinoflagellate algae. As a phenomenon which entails the transformation of chemical energy into light energy, bioluminescence is not restricted to living organisms, nor does it require the presence of living organisms. It is simply a type of chemiluminescent reaction that requires a luciferase activity which at one stage or another had its origins from a biological catalyst. Hence the preservation or construction of the essential activities and chemicals suffices to have the means to give rise to bioluminescent phenomena. Bioluminescent proteins with luciferase activity are thus available from a variety of sources or by a variety of means. Examples of bioluminescent proteins with luciferase activity may be found in U.S. Patent Nos 52229285, 5219737, 5843746, 5196524, 5670356.

"Fluorophore" refers to any molecule which can accept energy from the bioluminescent protein with luciferase activity, and re-emit it as lightenergy. There are a number of different fluorophores thatcan be employed in this invention. One very well known groupof fluorophores includes the green fluorescent protein from the jellyfish *Aequorea victoria* and numerous other variants (GFPs) arising from the application of molecular biology, eg. mutagenesis and chimeric protein technologies (R.Y. Tsien, (1998) *Ann. Rev. Biochem*. 63:509-544). GFPs are classified based on the distinctive component of their chromophores, eachclass having distinct excitation and emission wavelengths: class 1, wild-type mixture of neutral phenol and anionic phenolate: class 2, phenolate anion: class 3, neutral phenol: class 4, phenolate anion with stacked $\pi$-electron system: class 5, indole: class 6, imidazole: and class 7, phenyl (R.Y. Tsien, (1998), *supra*).

GFPs orother fluorophores, bioluminescent proteins with luciferase activities and the appropriate substrates have been combined into luminescence based assay, tagging, screening and protein-protein interaction detection systems: for example see U.S. Patent Nos. 5786162, 4614712, 5650289, 5837465, 5897990, 57002883, 54016229, 5221623, 5854010, 5770391, 4665022, 5854004.

"Substrate" refers to any means for activating the bioluminescent protein with luciferase activity. In a preferred embodiment *Renilla* luciferase (Rluc) (Cormier, (1978), *Methods Enzymol*., 57, 237-244), or one of its derivatives is used as the donor fluorophorebecause its emission spectrum is similar to the cyan ECFP (1 max Å 480 nm) which has been shown to exhibit FRET with EYFP (Miyawaki *et al*., (1997) *Nature (London)* 388, 882-887). The coelenterazine substrate for RLUC is a hydrophobic molecule that is permeable to most cell types.

"Protein" refers to include a whole protein, or fragment thereof, such as a protein domain or a binding site for a second messenger, co-factor, ion, etc. It can be a peptide or an amino acid sequence that functions as a signal for another protein in the system, such as a proteolytic cleavage site.

"Functional genomics" refers to the field pertaining to the determination of cellular function of a new gene. One manner of determining the function of a protein is to determine the identity of its binding partner. One application of the BRET system is to enable the identification of a gene product, or its target location within a cell, or the proteins, nucleic acid sequences, or other biomolecules with which it interacts.

"Binding pair" refers to two moieties (e.g. chemical or biochemical) that have an affinity for one another. Examples of binding pairs include homo-dimers, hetero-dimers, antigen/antibodies, lectin/avidin, target polynucleotide/probe, oligonucleotide, antibody/anti-antibody, receptor/ligand, enzyme/ligand and the like "One member of a binding pair" refers to one moiety of the pair, such as an antigen or ligand.

"Membrane-permeant derivative" refers a chemical derivative of a compound that has enhanced membrane permeability compared to an underivativized compound. Examples include ester, ether and carbamate derivatives. These derivatives are made better able to cross cell membranes, i.e. membrane permeant, because hydrophilic groups are masked to provide more hydrophobic derivatives. Also, masking groups are designed to be cleaved from a precursor (e.g.. fluorogenic substrate precursor) within the cell to generate the derived substrate intracellularly. Because the substrate is more hydrophilic than the membrane permeant derivative it is now trapped within the cells.

"Modulator" means a molecule or molecules that will undergo aconformational change in response to an interaction with another molecule, thereby affecting the proximity and/or orientation of the bioluminescent protein and the fluorophore. A modulator can be peptide, protein, nucleic acid or other synthetic or natural molecule. Examples of modulators for this invention include, a protease site, a second messenger binding site, an ion binding molecule, a homo-dimer or hetero-dimer molecule, a nucleic acid sequence, a kinase substrate or other enzyme substrate, a receptor or a receptor ligand.

"Isolated polynucleotide" refers a polynucleotide of genomic, cDNA, or synthetic origin or some combination there of, which by virtue of its ori gin the "isolated polynucleotide" (1) is not associated with the cell in which the "isolated polynucleotide" is found in nature, or (2) is operably linked to a polynucleotide which it is not linked to in nature.

"Isolated protein" refers a protein of cDNA, recombinant RNA, or synthetic origin or some combination thereof, which by virtue of its origin the "isolatedprotein" (1) is not associated with proteins found it is normally found with in nature, or (2) is isolated from the cell in which it normally occurs or (3) is isolated free of other proteins from the same cellular source, e.g. free of human proteins, or (4) is expressed by a cell from a different species, or (5) does not occur in nature. "Isolated naturally occurring protein" refers to a protein which by virtue of its origin the "isolated naturally occurring protein" (1) is not associated with proteins that it is normally found with in nature, or (2) is isolated from the cell in which it normally occurs or (3) is isolated free of other proteins from the same cellular source, e.g. free of human proteins.

"Polypeptide" as used herein as a generic term to refer to native protein, fragments, or analogs of a polypeptide sequence. Hence, native protein, fragments, and analogs are species of the polypeptide genus.

"Naturally-occurring" as used herein, as applied to an object, refers to the fact that an object can be found in nature. For example, a polypeptide or polynucleotide sequence that is present in an organism (including viruses) that can be isolated from a source in nature and which has not been intentionally modified by man in the laboratory is naturally-occurring.

"Operably linked" refers to ajuxtaposition wherein the components so described are in a relationship permitting them to function in their intended manner. A control sequence "operably linked" to a coding sequence is ligated in such a way that expression of the coding sequence is achieved under conditions compatible with the control sequences.

"Control sequence" refers to polynucleotide sequences which are necessary to effect the expression of coding and non-coding sequences to which they are ligated. The natureof such control sequences differs depending upon the host organism; in prokaryotes, such control sequences generally include promoter, ribosomal binding site, and transcription termination sequence; in eukaryotes, generally, such control sequences include promoters and transcription termination sequence. The term "control sequences" is intended to include, at a minimum, components whose presence can influence expression, and can also include additional components whose presence is advantageous, for example, leader sequences and fusion partner sequences.

"Polynucleotide" refers to a polymeric form of nucleotides of at least 10 bases in length, either ribonucleotides or deoxynucleotides or a modified form of either type of nucleotide. The term includes single and double stranded forms of DNA.

"Corresponds to" refers to a polynucleotide sequence is homologous (i.e., is identical, not strictly evolutionarily related) to all or a portion of a reference polynucleotide sequence, or that a polypeptide sequence is dentical to a reference polypeptide sequence. In contradistinction, the term "complementary to" is used herein to mean that the complementary sequence is homologous to all or a portion of a reference polynucleotide sequence. For illustration, the nucleotide sequence "TATAC" corresponds to a reference sequence "TATAC" and is complementary to a reference sequence "GTATA".

"Polypeptide fragment" refers to a polypeptide that has an amino-terminal and/or carboxy-terminal deletion, but where the remaining amino acid sequence is usually identical to the corresponding positions in the naturally-occurring sequence deduced, for example, from a fall-length cDNA sequence. Fragments typically are at least 5, 6, 8 or 10 amino acids long, preferably at least 14 amino acids long, more preferably at least 20 amino acids long, usually at least50 amino acids long, and even more preferably at least 70 amino acids long.

"Plate" refers to a multi-well plate, unless otherwise modified in the context of its.

The term "test chemical" refers to a chemical to be tested by one or more screening method(s) of the invention as a putative candidate.

The terms "label" or "labeled" refers to incorporation of a detectable marker, e.g., by incorporation of a radiolabeled amino acid or attachment to a polypeptide of biotinyl moieties that can be detected by marked avidin (e.g., streptavidin containing a fluorescent marker or enzymatic activity that can be detected by optical or colorimetric methods). Various methods of labeling polypeptides and glycoproteins are known in the art and may be used. Examples of labels for po lypeptides include, but arenotlimited to, the following: radioisotopes (e.g., .$^{3}$ H, .$^{14}$ C, $^{35}$ S,$^{125}$ I, $^{131}$ I), fluorescent labels (e.g., FITC, rhodamine, lanthanide phosphors), enzymatic labels (or reporter genes) (e.g., horseradish peroxidase, .beta.-galactosidase, .beta.-latamase, luciferase, alkaline phosphatase), chemiluminescent, biotinyl groups, predetermined polypeptide epitopes recognized by a secondary reporter (e.g., leucine zipper pair sequences, binding sites for secondary antibodies, metal binding domains, epitope tags). In some embodiments, labels are attached by spacer arms of various lengths to reduce potential steric hindrance.

"Genotoxicity" refers to the effect of a toxic compound on the stability of genes or genomes.

Common examples of second messengers include cAMP, cGMP, Ca2+, IP3, NO, DAG, ceramide and derivatives thereof, and arachidonic acid and derivatives thereof.

The terms, analyte, refers to a substance for which the presence, absence, or quantity is to be determined. An analyte is typically a small molecule or ionic solute such as $K^+$, $H^+$, $Ca^{2+}$, $CO_2$, $Na^+$, $Cl^-$, $Mg^2$, $O_2$, $HCO_3$, NO, and ATP.

As is evidenced throughout the text, some terms are used interchangeably to denote equivalent concepts. For example, the term "sensor" is used to denote a modulator entity that performs a specific function.

Common examples of ion channels include $K^+$-coupled receptor, $Ca^{2+}$,-channels, $K^+$-voltage-sensitive channels, $K^+$-channel $Ca^{2+}$ sensitive.

Other chemistry terms herein are used according to conventional usage in the art, as exemplified by The McGraw-Hill Dictionary of Chemical Terms (ed. Parker, S.,1985), McGraw-Hill, San Francisco).

The embodiments of the invention for which protection is sought are defined in the claims.

This invention provides a bioluminescence resonance energy transfer (BRET) system that comprises four parts: 1) a bioluminescent protein that has luciferase activity, 2) an acceptor fluorophore that can accept the energy from the bioluminescent protein when they are associated, in the presence of the appropriate substrate; 3) a modulator that influences the proximity or the orientation of the bioluminescent protein and the fluorophore, and 4) an appropriate substrate to activate the luciferase activity of the bioluminescent protein. The components of this system interact to influence the spatial relationship between the bioluminescent protein and the fluorophore, that is demonstrated by the light emission from the system. The modulator can be a single entity, covalently attached to both the bioluminescent protein and the fluorophore as demonstrated in Figure 1, or it can be two separate entities, each linked covalently to either the bioluminescent protein or the fluorophore as demonstrated in Figure 2, or an alternative configuration that falls within the scope of the invention.

This system can be used in *in vitro* assays to detect molecular changes in a wide variety of applications, and is amenable to automation. In particular, it is useful for assaying detecting changes in protein-protein interactions, and the concentration of analytes or signaling molecules in cells or in solution. It is useful for drug discovery, analyte screening, second messenger screening, drug screening, measuring the effect of a toxic compound on genome stability in genotoxicity, measuring the presence and concentration of toxic compounds in toxicology and determining the cellular function of a gene by determining its binding partner in functional genomics.

One preferred embodiment of this system is exemplified in Figure1. In this embodiment, there are generally two entities comprising the modulator, wherein the bioluminescent protein is covalently attached to one modulator entity and the fluorophore is covalently attached to the second modulator entity. The interaction between the two modulator entities determines the proximity and orientation between the bioluminescent protein and the fluorophore, and therefore the light emission from the system.

This embodiment can be used to detect the interaction (moving together, moving apart, or changing their orientation to one another) of the two modulator entities. In one example, wherein this embodiment can be used for detecting binding partners, the bio luminescent protein can be attached to one gene product and the fluorophore can be attached to an unknown gene product, such that interaction between the two gene products will be displayed by the light emission of the fluorophore. In another application, such as drug discovery, this embodiment is used to determine if a chemical or molecular substance will cause the molecular entities such as homo-dimers orhetero-dimers to move together or to move apart. By attaching the bioluminescent protein to one of the dimer-proteins and the fluorophore to the other dimer-protein, changes in the spatial relationship between the dimer-proteins in response to a chemical compound such as a drug candidate, will translate to the spatial relationship between the bioluminescent protein and the fluorophore, that will be signaled by a change the light emission from the system.

Another preferred embodiment is exemplified in Figure 2. In this embodiment, the bioluminescent protein and the fluorophore are attached to a common linker comprising the modulator component of the system. One typical example of this embodiment is the use of the modulator as a sensor that will detect changes in a component in the environment, causing the sensor to undergo a conformational change that affects the spatial relationship of the bioluminescent protein and the fluorophore, that is signalled by the light emission from the system.

This bioluminescent protein-modulator- fluorophore construct (BMFC) can be produced in a number of ways. It can be produced entirely using recombinant methods by preparing and expressing a recombinantDNA encoding the BMFC fusion protein. Alternatively, it can be produced by isolating the component proteins from a recombinant system and then covalently attaching the units using chemical means. Alternatively, the fusion protein construct can be produced in two parts, wherein the bioluminescent protein and the fluorophore are produced using recombinant methods, optionally fused to a peptide linker or a portion of the modulator component. In the latter situation, all or part of the modulator can be attached chemically to each peptide linker or directly to the bioluminescent protein or fluorophore, thereby generating a linear construct with the bioluminescent protein joined to the fluorophore through the peptide linker comprising the modulator. The modulator can be a single entity or two entities that interact. The modulator can further be either biologically derived or chemically synthesized or a modified biological molecule. Another option is to isolate the bioluminescent protein and/or the fluorophore from natural sources and chemically join them to the modulator.

There are a wide variety of examples demonstrating the versatility and usefulness of the BRET system. For example, a bioluminescent protein-modulator-fluorophore construct (BMFC) is presented in Example IV

*The Use of the BRET Assay of Protein-Protein Interactions for Cell-Based Assays*

The BRET system is useful for cell based assays. In general, the proteins will be introduced by transforming or transfecting a cell with one or more vectors comprising the recombinant DNA encoding some of the components of the system. The cell will produce the protein and BRET will occur when the luciferase, the fluorophore and the substrate are in the appropriate spatial relationship. The substrate can be either added or introduced in its nucleic acid form, such as via a vector with an inducible promoter.

*The Use of the BRET Assay of Protein-Protein Interactions for in vitro Assays*

The components of the BRET system can be produced using molecular biology techniques or isolated from natural sources. After purification, they can be used in non-cell based in vitro assays.

One application of this system to an in vitro system is demonstrated by the results presented in Figure 3, which presents spectra derived from mixing E. coli extracts from two different cultures, one expressing RLUC•KaiB and the other expressing EYFP•KaiB. In the luminescence spectrum acquired immediately after mixing, there is no evidence for a second peak. After room temperature incubation for 30 minutes, a significant shoulder has emerged on the spectrum of the mixed extracts. At the 30 min incubation time point, the intensity of the RLUC luminescence has also decreased to approximately 60% of the time 0 value. This decrease in luminescence intensity is likely due to proteolysis within the extract. After a two hours of room temperature incubation, the luminescence exhibited a clear bimodal spectrum, although the overall luminescence intensity was only about 6% of the initial value. Even though the assay needs further optimization, the development of a shoulder followed by an obvious bimodality indicates that BRET can be assayed in vitro.

The Use of the BRET Assay for High-Throughput Screening

As demonstrated in Example XI, the BRET system is particularly amenable to automation and high-throughput screening.

This example also demonstrates that the BRET system can be useful for cell based assays. For example, the ratio of luminescence intensities at 480 nm to 530 nm can be measured to determine the magnitude of BRET, and to correct for differences in overall levels of expression of RLUC and EYFP fusion proteins. To test the feasibility of BRET ratio imaging, we collected images with a cooled-CCD camera of E. coli cultures expressing our constructs. Figure 4 shows images of liquid E. coli cultures as seen through 10 nm bandpass interference filters centered at 480 nm and 530 nm. In the cultures containing a control strain (RLUC alone) or a non-interacting combination (RLUC•KaiB & EYFP•KaiA), there is much less light emitted at 530 nm than at 480 nm. In the fusion construct (RLUC•EYFP) or the interacting combination (RLUC•KaiB &EYFP•KaiB), the amount of light emitted at 480 nm and 530 nm are roughly equal, as would be predicted from the spectra depicted in Figures 1 and 2(b2). These images can be quantified, as shown in figure 5. The 530nm/480nm ratios of luminescence for the RLUC control and the non-interacting KaiB & KaiA couple are low (about 0.4), whereas the 530nm/480nm ratios for the fusion construct and for the interacting KaiB fusion proteins are above 0.9.

The Use of the BRET Assay of Protein-Protein Interactions for Chemical Detection

There are several new applications using the FRET ratio principle, e.g., for measuring gene expression (Zlokarnik et al., 1998) or for measuring intracellular levels of calcium ions (Romoser et al., 1997; Miyawaki et al., 1997). BRET could be used in these applications, and may in fact have advantages over using FRET. For example, two groups have reported a novel method for ratio imaging of $Ca^{++}$ based on using two GFP variants (e.g., BFP, CFP, GFP, YFP) linked by a Ca $^{++}$-binding calmodulin sequence (Romoser et al., 1997; Miyawaki et al., 1997). When the linker sequence binds $Ca^{++}$, it undergoes a conformational change that brings the GFP variants closer together, enabling FRET. In the absence of sufficient $Ca^{++}$, the GFP variants remain far apart and FRET does not occur. Therefore, the magnitude of FRET is an indicator of $Ca^{++}$ levels; because it is a ratio method, differences in the level of expression of the $Ca^{++}$ indicator do not introduce artifacts to the measurement.

This principle is adaptable to BRET by designing a construct where Rluc is linked to Eyfp via the same calmodulin linker. Here, the ratio of luminescence at 530 nm versus 480 nm should give an estimate of $Ca^{++}$. The potential advantages of the BRET approach are the same as those delineated above: a BRET indicator should be better for measuring $Ca^{++}$ in photoresponsive or auto-fluorescent tissue, and there will be no photobleaching of fluorophores. Furthermore, because there will be no direct excitation of the acceptor fluorophore, a better ratio measurement of $Ca^{++}$ can be obtained with BRET.

*Adaptabiliry of BRET to Automation and High-Throughput Screening*

The BRET system is adaptable to means of automation and high-throughput screening.

The experiment depicted in Figures 4 and 5 suggests a relatively simple scheme for designing a cell based library screening system for protein-protein interaction using BRET. By sensitively measuring the light emmision collected through interference filters, the 530nm/480nm luminescence ratio of E. coli (or yeast) colonies expressing a "bait" protein fused to RLUC and a library of "prey" molecules fused to EYFP (or vice versa) could be measured. Colonies that express an above-background ratio could be saved and the "prey" DNA sequence further characterized. Expression vectors would need the following features: multiple cloning sites for insertion of the bait and prey libraries to enable both N-terminal and C-terminal fusions of the bait molecule and prey library to RLUC and EYFP, and an assortment of antibiotic or other markers to allow various expression/terminal fusion combinations to be tested. With appropriate instrumentation, high-throughput screening using BRET is a possibility. Using an imaging instrument similar to the one we used here, it would be possible to screen colonies of bacteria or yeast on agar plates. On the other hand, a photomultiplier tube.

PREPARING THE BRET SYSTEM

*Selecting Suitable Bioluminescent Protein-Fluorophore Pairs*

To design a system, one first chooses a bioluminescent protein with luciferase activity and a fluorophore. In nature some bioluminescent protein-fluorophore pairings interact directly with one another. In other cases the pairing interaction is mediated through other proteins, in the absence of which interactive coupling occurs with a significantly lower affinity. If such a decreased affinity is present atexpression levels required to achieve BRET, when the biluminescent protein with luciferase activity and fluorophore are coupled respectively to other protein moieties, then such a pairing is suitable for BRET. Examples of such systems which occur naturally include the *Renilla* luciferase (RLUC) /*Renilla* GFP coupling (Cormier (1978) Methods Enzymol. 57:237-244) and the Aequorin/*Aequorea* GFP coupling (Wilson and Brand (1998) Annu. Rev. Cell Dev. Biol. 14:197-230). An example of an engineered system which is suitable for BRET is an RLUC and enhanced yellow mutant of GFP (EYFP) pairing which do not directly interact to a significant degree with one another alone in the absence of mediating proteins (Y. Xu, *et al*. (1999) PNAS 96:151-156).

Other fluorophores which are suitable pairings for RLUC include fluorophores from GFP classes 1,2,4 and some from 5. Specific examples of suitable fluorophores include but are not necessarily limited to: wild type GFP, Cycle 3, EGFP, Emerald, Topaz, 10C Q69K, and 10C.

Other criteria which should be considered in determining suitable pairings for BRET is the relative emission/fluorescence spectrum of the fluorophore compared to thatofbioluminescentprotein with luciferase activity. For example it has been demonstrated thatan RLUC/EGFP pairing is not as good as an RLUC/EYEF pairing based on observable emissionspectral peaks (Y. Xu, (1999), *supra* and Wang, *et al*. (1997) in *Bioluminescence and Chemiluminescence: Molecular Reporting with Photons,* eds. Hastings et al. (Wiley. New York), pp. 419-422) It should be apparent to one skilled in the art that other non-GFP fluorophores, or other bioluminescent proteins with luciferase activity may be suitable for the BRET system of the present invention if the key pairing criteria are met. Other sources for example may include pairings isolated or engineered from insects (U.S. Patent No. 5670356). Alternatively, it may be preferable even still, for the bioluminescent protein with luciferase activity and the fluorophore to be derived from completely different sources in order to minimize the potential for direct affinity between the pairings.

In a preferred embodiment a protein fusion of a blue-emitting luciferase (RLUC) and a GFP mutant acceptor fluorophore are expressed in E. coli. Initially, we tried EGFP as the acceptor, but as previously reported by Wang et al. (1997) with a similar fusion protein, only a barely detectable shoulder at 508 nm was added to the RLUC emission spectrum (data not shown). This shoulder was judged to be too small to use as a BRET assay for protein-protein interaction. We then tested the EYFP as an acceptor fluorophore (Orm et al., 1996), with the initial constructs used shown in figure 1(a). The RLUC•EYFP fusion construct included an intergenic sequence of 11 amino acid residues. Bioluminescence/fluorescence spectra of E. coli containing RLUC, EYFP, and RLUC•EYFP constructs under the control of the T7 promoter are as shown in Figure 1 (b). Both the excitation and fluorescence spectra of the EYFP-expressing E. coli are red-shifted from EGFP, with an excitation peak at 480 nm and fluorescence peak of 527 nm. The luminescence profile of the E. coli cells expressing the RLUC construct displayed a typical emission spectrum for Renilla luciferase: a rather broad emission spectrum with a single peak at 480 nm (Figure 1b). However, the luminescence spectrum from cells expressing the RLUC•EYFP fusion construct yielded a bimodal spectrum, with one peak centered at 480 nm (as for RLUC), and a new peak centered at 527 nm (as for EYFP fluorescence). These data suggest that a proportion of the RLUC luminescence was transferred to EYFP and re-emitted at its characteristic wavelength.

It should be confirmed that the donor and acceptor fluorophores do not spurious ly associate with each other. This can be accomplished by:

In a preferred embodiment, using RLUC and EYFP, it is confirmed that these two do not spuriously associate with eath other, and thus appear to be a good BRET pair for a protein-protein interaction assay. After co-expression of separate RLUC and EYFP (the top two constructs in Figure 1 (a)) in the same E. coli cells (without IPTG induction), the luminescence spectrum looks like an unalteredRLUC spectrum there is no evidence of a second peak. However, when EYFP and RLUC were overexpressed by IPTG induction, we sometimes did observe a small second peak, which suggests that there may be some weak BRET between the two proteins that can be forced by overexpression. Since we were able to achieve sufficient signal levels using the T7 promoter without IPTG addition, we could use a regime in which RLUC and EYFP do not associate by themselves. All further experiments reported in this paper were performed under conditions where we confirmed that no second peak developed in the RLUC & EYFP expressing cells.

Tests should be conducted to determine whether the donor and acceptor fluorophores still generate BRET when they are fused to interacting proteins. This can be performed by:

In a preferred embodiment, it is tested whether RLUC and EYFP would still generate BRET when they were fused to interacting proteins. As our test proteins, we chose the circadian clock proteins KaiA, KaiB, and KaiC from the cyanobacterium Synechococcus (Ishiura et al., 1998). Yeast two-hybrid assays indicate that KaiB proteins can form a homodimer (Iwasaki et al., 1998). Thus, we made fusion constructs of kaiB to the Eyfp and Rluc genes respectively (Figure 2(a)) and measured the luminescence spectra of E. coli expressing these constructs. As shown in Figure 2(b2), a second peak emerges from cells expressing both RLUC•KaiB and EYFP•KaiB. The similarity between the spectrum depicted in Figure 1 (b) for the fusion construct and Figure 2(b2) strongly suggests that interaction among KaiB molecules has brought the RLUC and EYFP into close proximity such that energy transfer occurs for ~50% of the RLUC luminescence. To demonstrate that this bimodal spectrum is specific for the KaiB interaction, we fused EYFP to KaiA and co-expressed it with RLUC•KaiB. In this case, there is no evidence of a second luminescence peak, and thus no sign of interactions between KaiA and KaiB (Figure 2(b3)). We also found no BRET-assayable interaction between KaiB and the third cyanobacterial clock protein, KaiC. This latter result is somewhat surprising because the yeast two-hybrid assay found a KaiB-KaiC interaction (Iwasaki et al., 1998). This negative result was not due to altered levels of expression or optical activity of the RLUC and EYFP fusion proteins since the RLUC signals and EYFP fluorescence levels were similar in all the E. coli strains. However, it may be due to the fact that we examined only N-terminal fusions of RLUC and EYFP to the Kai proteins, and that this orientation prevented the occurrence of BRET. Whatever the explanation for the lack of KaiB KaiC interaction in our measurements, the data suggest that false positives may be rare in the BRET assay.

*Methods for Chemically or Genetically Attaching the Bioluminescent and the Fluorescent Proteins to the Proteins or Polypeptides of Interest*

There are a number of methods well known in the art for chemically or genetically attaching the bioluminescent donor protein and the fluorescent acceptor protein to the target proteins or polypeptides of interest.

ADVANTAGES AND LIMITATIONS OF THE BRET SYSTEM

Currently, the yeast two-hybrid system is the most popular library-based technique for identifying protein interactions. Since it is a selection technique, potential interactors can be identified rapidly and further characterized without extensive screening. Other than this single (but admittedly important) advantage, optical energy transfer techniques (i.e., FRET & BRET) are potentially superior to the yeast two-hybrid system for assaying protein interactions. For example, in the yeast two-hybrid system, interaction must occur in the nucleus, but FRET or BRET interactions can be assayed in different cellular compartments by appropriate targeting of the labelled constructs. This feature is particularly important in the case of interacting membrane proteins that are unlikely to yield a positive result with the two-hybrid assay. Moreover, interactions dependentupon cell-type specific post-translational modifications that do not occur in yeastwill obviously not be detected in the two-hybrid assay. Theoretically, FRET or BRET should be able to uncover these interactions. After a possible interacting pair is found with the yeast two-hybrid assay, further characterization by analytical techniques is necessary. On the other hand, FRET and BRET can be both screening and analytical techniques. Potential interactors can be identified by screening libraries in E. coli, yeast, or the original cell type. Subsequent FRET/BRET analyses of these candidates may then be performed in the original cell type such that the cell-type specific post-translational modification and/or compartmentalization of the proteins is preserved. Finally, it may be possible in some cases to use FRET/BRET to assay the kinetics of changes in protein interactions in vivo. All of these advantages are particularly useful for investigations of protein interactions in mammalian cells, with applications to membrane protein interactions and high-throughput screening.

Comparison of BRET with FRET

Because BRET does not require the use of excitation illumination, it has potential advantages over FRET. BRET should be superior for cells that are either photo-responsive (e.g., retina or any photoreceptive tissue) or damaged by the wavelength of light used to excite FRET (typically near-UV; Periasamy and Day, 1998). Cells that have significant auto-fluorescence would also be better assayed by BRET than by FRET. Autofluorescence is not an uncommon problem; in addition to the cell types that have unusual autofluorescent pigments or crystals, almost all cells have broad spectrum autofluorescenceof NAD (P)H elicited by near-UV light. Inaddition, while photobleaching of the fluorophores can be a serious limitation of FRET, it is irrelevant to BRET. A more subtle, but significant, advantage of BRET over FRET is that FRET may be prone to complications due to simultaneous excitation of both donor and acceptor fluorophores. Specifically, even with monochromatic laser excitation, it is practically impossible to excite only the donor without also exciting the acceptor fluorophore to some degree. Therefore, specific acceptor-only control experiments must be performed for FRET. In contrast, because BRET does not involve optical excitation, all the light emitted by the fluorophore must result from resonance transfer. In this respect, BRET is theoretically superior to FRET for quantifying resonance transfer., but it remains to be determined how significant this advantage will be in practical applications.

Some limitations of the BRET Assay should be kept in mind when designing the system. For example, Förster resonance energy transfer is dependent on proper orientation of the donor and acceptor dipoles. In some embodiments of the present invention, conformational states of the fusion proteins may fix the dipoles in a geometry that is unfavorable for energy transfer. Further, because the fluorophore/luciferase tags are fused to ends of the potentially interacting molecules, it is possible that some parts of the candidate molecules are interacting without allowing the fluorophore/luciferase tags to be close enough for energy transfer to occur. Consequently, two proteins might interact in a way that is blind to the FRET/BRET technique. In other words, a negative result with a resonance transfer technique does not prove non-interaction.

Another limitation concomitant with the use of GFP variants as fluorophore tags is that GFP does not turn over rapidly. First, ittakes at least an hour for the GFP molecule to fold properly (Chalfie et al., 1994; Heim et al., 1995: Heim et al., 1995). Second, once folded, GFP is remarkably resistant to proteolysis in foreign cells. Therefore, in some cases, the slow kinetics of GFP turnover may hamper measuring the kinetics of interaction. On the other hand, Renilla luciferase does not suffer these same disadvantages in turnover rate. Therefore, if one of the interacting proteins turns over more rapidly than the other, it might be useful for kinetic estimates to fuse the more labile protein to RLUC.

BRET is not without its own limitations, however. RLUC requires a substrate, coelenterazine. Coelenterazine is hydrophobic and appears to permeate many cell types (E. coli, yeast, plant seedlings, mammalian cell cultures), but it is possible that there are some cells that are not permeable to coelenterazine. Coelenterazine can also exhibit significant auto-luminescence in certain media. We find little if any coelenterazine auto-luminescence in simple salt media, but this may be a more serious problem in complex media. Moreover, lack of sensitivity may hinder using BRET in some cases. Depending upon the expression level of RLUC, the luminescence can be dim, and for ratio imaging only a small proportion of the total light is collected. Therefore, a very sensitive light-measuring device is necessary.

EXAMPLES

## EXAMPLE I: CONSTRUCTION OF RLUC•EYFP FUSION EXPRESSION CASSETTE

The vectors used for creating expression cassettes of RLUC, EGFP, EYFP, and their fusions were as follows: RLUC used pRL-null (Promega, Madison, WI), whileEGFP and EYFP used pEGFP-N1 and pEYFP respectively (both from CLONTECH, Palo Alto, CA). pRSET was also used as a shuttle vector (Invitrogen, Carlsbad, CA). Table 1 lists the constructs used.

Table 1.

| Expression strains used in this study | | |
|---|---|---|
| Strain Producing: | Plasmid | Antibiotic Resistance* |
| RLUC | pT7/Rluc | Km |
| EYFP | pT7/Eyfp | Amp |
| RLUC::EYFP | pT7/Rluc::Eyfp | Km |
| RLUC & EYFP | pT7/Rluc & pT7/Eyfp | Amp & Km |
| EYFP::KaiB | pT7/Eyfp::*kaiB* | Amp |
| RLUC::KaiB | pT7/Rluc::*kaiB* | Km |
| EYFP::KaiA | pT7/Eyfp::*kaiA* | Amp |
| RLUC::KaiB & EYFP::KaiB | pT7/Rluc::*kaiB* & pT7/Eyfp::*kaiB* | Amp & Km |
| RLUC::KaiB & EYFP::KaiA | pT7/Rluc::*kaiB* & pT7/Eyfp::*kaiA* | Amp & Km |

*Km: Kanamycin; Amp: ampincillin

ARLUC•EGFP fusion cassette was constructed by removing the stop codon TAA of Renilla luciferase cDNA (Rluc) and adding linker sequences such that the downstream EGFP coding region was in frame with that of the upstream RLUC. Based on pRL-null DNA sequences, two primers were designed for cloning of the RLUC coding region with a T7 promoter using Pwo DNA polymerase (BOEHRINGER MANNHEIM, Indianapolis, IN): 5'-(NdeI)CATATGCTTAAGGCTAGAGTACT-3'(SEQ ID NO:1) and 5'-(ApaI) GGGCCCGTTGTTCATTTTTGAGAAC-3'(SEQ ID NO:2), where the linker sequences are underlined. These primers were used to amplify a 992 bp fragment from the pRL-null template, which was then inserted into the NdeI/ApaI site of the vector pEGFP-N1 containing an enhanced green fluorescent protein gene (Egfp) to give the plasmid pRluc•Egfp under the control of a T7 promoter, yielding a $P_{T7}$/Rluc•Egfp fusion expression cassette. This insertion also removed the human cytomegalovirus (CMV) promoter of pEGFP-N1. The intergenic sequence created between Rluc and Egfp was 27bp: 5'-CGGGCCCGGGATCCACCGGTCGCCACC-3' (SEQ ID NO:3). This gene fusion construct was confirmed by DNA sequencing, RLUC luminescence, and GFP fluorescence.

To make the Rluc•Eyfp gene fusion, the EGFP coding region in the pRluc•Egfp was replaced with the BamHI/NotI fragment of enhanced yellow fluorescence protein coding sequence (Eyfp) from the vector pEYFP (from CLONTECH) to produce the plasmid pT7/Rluc•Eyfp, in which the EYFP open reading frame is in frame with that of RLUC. There are 11 codons between Rluc and Eyfp in this gene fusion, as shown in Figure 3(a). To create the pT7/Rluc plasmid, the EYFP coding region was removed from pRluc•Eyfp by digestion with BamHI and NotI, end-blunting and self-ligation.

## EXAMPLE II: CLONING OF CYANOBACTERIAL CLOCK PROTEIN CODING REGIONS

The recovered plasmid p48N from genetic complementation experiments that carries the cyanobacterial circadian clock gene cluster kaiA, kaiB, and kaiC (Ishiura *et al*., (1998) *Science,* 281,1519-1523), was used as a template for cloning of the kai coding regions. To fuse the coding region sequences for kaiA or kaiB in frame to His-Tag (a ProBond binding domain), the following two pairs of primers were designed and include the underlined linker sequences: 5'-(BglII) AGATCTATGCTCTTGGAGTATGC-3' (SEQ ID NO:4) and 5'-(HindIII) AAGCTTCGAGCTTAAGACCTCCT-3' (SEQ ID NO:5) (for cloning of kaiA); 5'-(BglII) AGATCTATGAGCCCTCGTAAAACCTAC-3' (SEQ ID NO:6) and 5'-(HindIII) AAGCTTGCAGTTAACGACAGTTAGAAG-3' (SEQ ID NO:7) (for cloning of kaiB), where the restriction sites are underlined and the start codons are emboldened. The 766 bp kaiA fragment and the 323 bp kaiB fragment that was amplified by Pwo DNA polymerase were each cloned into the BglII/HindIII site of the expression vector pRSET-B to give rise to plasmids pHis•kaiA and pHis•kaiB, respectively. Both the sequences of these constructs and the correct molecular weights of the fusion proteins His•KaiA and His•KaiB were confirmed by techniques known to those skilled in the art and/or as described herein.

**Fusion of the coding sequences for cyanobacterial clock proteins to Rluc or Eyfp**

After the plasmid pHis•kaiB was digested with HindIII, the recessed 3' termini were filled by the Klenow fragment of *E. coli* DNA polymerase. The linearized plasmids were then completely digested with BamHI. The purified HindIII (blunt end)/BamHI fragments of the full length kaiB coding region were used to replace the EYFP coding region in the pRluc•Eyfp plasmid that had been digested with NotI (blunt end created by Klenow fragment digestion) and BamHI to produce pRluc•kaiB, in which the kaiB coding region is in frame with that of RLUC. The construct was confirmed by restriction enzyme analysis and *in vivo* RLUC luminescence assay known to those skilled in the art and/or as described herein.

To create expression cassettes with an in-frame fusion of clock proteins to EYFP, a EYFP coding sequence without stop codon was synthesized by designing the following two primers and include the underlined linker sequences: 5'-(NdeI) CATATGGTGAGCAAGGGCGA-3' (SEQ ID NO:8) and 5'- (BglII) AGATCTCTTGTACAGCTCGTCCA-3' (SEQ ID NO:9), where the restriction sequences are underlined, and the first and last codons for EYFP are emboldened. This fragment was inserted into the NdeI/BglII sites of pHis•kaiA and pHis•kaiB respectively. This insertion removed the His-Tag and enterokinase site sequences of pRSET and produced the two plasmids pEyfp•kaiA and pEyfp•kaiB. These constructs were confirmed by DNA sequencing and *in vivo* EYFP fluorescence assay known to those skilled in the art and/or as described herein. The kaiB coding region in pEyfp•kaiB was removed by digestion with BglII and HpaI and ligated to make the plasmid pT7/Eyfp.

**Bacterial strains and transformation**

The host strain used for cloning was E. coli DH5α. For expression of Rluc, Eyfp, and all gene fusions that were controlled by the T7 promoter, E. coli BL21 (DE3) was used as a host strain (Novagen, Madison, WI). The expression host strain was transformed with single or double plasmids using competent cells prepared by calcium chloride. All transformants were confirmed by luminescence and/or fluorescence assays. Various strains containing possible combinations of fusion proteins were made and analyzed for this study. Table 1 lists those transformed strains.

**EXAMPLE III: BIOLUMINESCENCE AND FLUORESCENCE ASSAY *IN VIVO***

*E. coli* strains were grown overnight in LB medium containing 50 μg/ml of kanamycin and/or 100 μg/ml of carbenicillin (for ampicillin resistant strains) at 37°C. Because LB medium has background fluorescence, the *E. coli* cultures were washed and resuspended in M9 minimal salts medium before assay. To assay EYFP fluorescence, 1.5 ml of *E. coli* cells in M9 medium ($OD_{600}$ = 0.8-1.0) were transferred to a 2 ml cuvette. Fluorescence excitation and emission measurements were performed on a SPEX (Edison, NJ) Fluorolog spectrofluorometer with a 250w xenon arc lamp. EYFP fluorescence was excited at 470 nm and its emission scanned from 505 to 580 nm. For the luminescence assay, the lamp was shuttered off, and the cuvette was gently bubbled with air to supply sufficient oxygen for the Renilla luciferase reaction following the addition of coelenterazine (BioSynth) to a final concentration of 1 μM. Bioluminescence emission spectra were collected between the wavelengths 440 -580 nm.

Figure 3a shows the different constructs and the linker region made for that example. EYFP was tested as an acceptor fluorophore, with the constructs shown in Fig.3a. Bioluminescence and fluorescence spectra of *E.coli* containing Rluc, EYFP and Rluc::EYFP constructs under the control of the T7 promoter are shown in Figure 3b. The luminescence profile of the *E.coli* cells expressing the Rluc construct displayed a typical emission spectrum for Rluc (Renilla luciferase) with a single peak at 480nm. (Fig.3b). The luminescence spectrum from cells expressing the Rluc::EYFP fusion construct upon coelenterazine addition, yielded a bimodal spectrum, with one peak centered at 480nm (Rluc) and a new peak centered at 527nm (as for EYFP fluorescence). These data suggest that a significant proportion of the R luc energy was transferred and emitted at the characteristic wavelength of EYFP. Because Rluc and EYFP appear to be good BRET pair for a protein-protein interaction assay, we wanted to confirm that they do not spuriously associate with each other. After co-expression of separate Rluc and EYFP (the top two constructs in Fig. 3a) in the same *E.coli* cells (without IPTG induction), the luminescence spectrum looks like an unaltered Rluc spectrum-there is no evidence of a second peak (Fig 4b, spectrum b1).

Based on the example of interacting clock protein described in Xu et al, (1999) *Proc. Natl. Acad. Sci.* 96, p151-156), we decide to test whether the BRET assay could be used to discover interaction among the kai proteins. Thus, we made fusion constructs of kaiB to the EYFP and Rluc genes, respectively (Figure 4a), and measured the luminescence spectra of *E.coli* expressing these constructs. As shown in Figure 4b, (spectrum b2), a second peak emerges from cells expressing both Rluc::kaiB and EYFP::kaiB. The similarity between the spectrum depicted in Figure 3b for the fusion construct (Rluc::EYFP) and the Rluc::kaiB and EYFP: :kaiB combination in Figure 4B (b2) strongly suggests that interaction among kaiB molecules has brought the Rluc and EYFP into close proximity such that energy transfer between Rluc and EYFP, occurs. To demonstrate that this bimodal spectrum does notoccur nonspecifically, we fused EYFP to a slightly truncated kaiA (see Figure 4a) and coexpressed it with Rluc::kaiB construct. As expected since kaiA and kaiB should not interact together, there is no second luminescence peak (Figure 4b graph b3) and thus no indication of interaction between kaiA and kaiB.

**EXAMPLE IV: *IN VITRO* BRET ASSAY FOR KAIB-KAIB INTERACTION**

In this Example, the BRET method is demonstrated by assaying interactions between proteins encoded by circadian clock genes. In cyanobacteria, Drosophila (Gekakis *et al*., (1995) *Science* **270**, 811-81) and mammals (King *et al*., (1997) *Cell*, **89**, 641-653; Tei *et al*., (1997) *Nature (London)* **389**, 512-516; and Albrecht *et al*., (1997), interactions between proteins encoded by clock genes appear to play a crucial role in the function of the timekeeping loop. The circadian clock genes kai A and B from cyanobacteria (Ishiuraetal., (1998) *Science,* **281**, 1519-1523) was therefore used to demonstrate the BRET principle.

Two expression strains, RLUC•KaiB and EYFP•KaiB, were used to examine KaiB to KaiB interaction *in vitro*, and the RLUC•EYFP fusion strain (pRluc•Eyfp) was used as a positive control. A single of colony of each strain was inoculated into LB medium with 50 μg/ml of kanamycin or 100 μg/ml of carbenicillin and grown overnight at 37°C. The cells were washed once with fresh LB medium and resuspended in the same medium containing 1 mM IPTG. After a 3 hour incubation at 37°C, the cells were collected and washed once in assay buffer (50 mM KCl, 50 mM NaCl, 2.5 mM $MgCl_2$, 2 mM EDTA, 5 mM DTT, 0.2% NP40, 1 00 μg/ml PMSF, 2 μg/ml Leupeptin, 2 μg/ml Aprotinin, and 20 mM HEPES, pH 8.0). The cells were then resuspended in fresh assay buffer at 4 °C containing 10 μg/ml of lysozyme and kept on ice for 30 min. Microcentrifuge tubes containing the cells wereput into a -70°C EtOH bath to quickly freeze the cells and then were placed on ice. An equal volume of fresh assay buffer was added into the cell lysates. After the mixtures were spun for 5 min in a microcentrifuge at 8,000 rpm (4°C), the supernatants were tested for fluorescence and luminescence as above except that no air w as bubbled into the extracts. For monitoring KaiB-KaiB interaction *in vitro* by BRET, RLUC•KaiB extracts were mixed 1:1 with EYFP•KaiB extracts and thereafter incubated at room temperature; luminescence spectra were taken at 0 min, 30 min, and 120 min after mixing.

Figure 5 represents spectra derived from mixing *E.coli* extracts from two different cultures one expressed Rluc::kaiB and the other expressing EYFP::kaiB. Immediately after mixing, there is no evidence of the second peak. After room temperature incubation for 30 minutes (second graph), a significant shoulder has emerged on the spectrum. At the 30-minites incubation point, the intensity of the Rluc luminescence has decreased probably because of proteolysis within the extract. After 2 hours of room temperature, the luminescence exhibited a clear bimodal spectrum.

**EXAMPLE V: BRET IMAGING OF E. COLI CULTURES**

About 1.5 ml *E. coli* cultures grown overnight at 37°C in LB medium with appropriate antibiotics was washed twice with M9 medium. The cell pellet was resuspended in 300 ml of M9 medium containing 3 μM coelenterazine. Five microliters of the suspension was immediately added to the wells of a Nunc-Microwell plate. The small volume of the sample allowed good gas exchange and so bubbling was not required (fig. 6). Eight duplicates were made for each strain, which were then divided into two groups. One group was visualized through a 480 nm (± 5 nm) interference filter, and the other through a 530 nm (± 5 nm) filter (Ealing Electro-Optics. Holliston, MA). Images were captured with a cooled-CCD camera (TE/CCD512BKS, Princeton Instruments, Trenton, N.J.) under the control of and analyzed by custom software created by Dr. Takao Kondo (Kondo *et al*., (1994) *Science* 266:1233-1236. This example also showed that BRET could be detected and measured using CCD-camera based technology.

Figure 7 represents the quantification analysis of luminescence intensity (in RLU) of Figure 6 whenusing the two interference filters 480nm and 530nm (upper graph). Lower graph shows the luminescence ratio of intensity obtained with the 530nm filter on the luminescence intensity obtained with the filter 480nm, a, b, c, and d found on both figure 6 and 7, represent Rluc, Rluc::EYFP, Rluc::kaiB and EYFP::kaiA, andRluc::kaiB andEYFP::kaiB, respectively. The 530/480nm ratios of luminescence for the Rluc control (a) and the noninteracting kaiA and kaiB (c) couple are low (approximately 0.4), whereas the 530/480nm ratios for the Rluc: :EYFP fusion construct and for the interacting kaiB fusion proteins are above 0.9.

**EXAMPLE VI: DEMONSTRATION OF USE OF METHOD FOR IN-VITRO FUSION ASSAY**

Fusion assays are achieved when the Rluc moiety (donor) is attached either genetically or chemically to the fluophore acceptor moiety, the EYFP. A linker region is found in between the two moieties. Specific sites that have specific functions, could be introduced in this linker region. In this example, a specific protease site for the enterokinase was introduced between the Rluc and EYFP. Upon interaction of the enterokinase with its site in the sensor (Rluc-enterokinase-EYFP), the enzyme will cut the linker region which in turn will separate the two moieties. Therefore, we should observe a decrease of BRET (i.e. ratio 550/470nm should decrease).

The DNA constructs generated to perform this experiment are as follows: Rluc origin of the gene (pRL-CMV-Promega, Madison, WI), Rluc•EYFP origin of the gene (Xu, et al., 1999 *Proc. Natl. Acad Sci. USA,* 96:151-156), and Rluc:enterokinase:EYFP origin of the gene (this construct is built by introducing DNA sequences coding for the enterokinase recognition site into the linker region on the Rluc•EYFP construct).

The enterokinase site is introduced into the linker region of Rluc•EYFP (Gly-Asp-Asp-Asp-Asp-Lys-Leu) when the enterokinase enzyme recognizes the four Asp and the lys, and cuts the peptide linker located after the amino acid Lys.

The two oligonucleotides (sense and antisense), which correspond to the enterokinase site (BRL/Gibco-Life Technologies, Gaethersburg, MD), were complementary to each other and were annealed together using molecular biology techniques well known to those skilled in the art. The double strand of DNA was engineered to have cohesive ends (BamHI (5' end) and KpnI (3' end)) after annealing and was subcloned into the Rluc•EYFP construct at the BamHI-KpnI sites.

The following are the DNA sequences corresponding to the enterokinase site (sense and antisense) having cohesive ends:

**5'GATCCGGGCGACGATGACGATAAGTTGGCGGTA3' (SEQ ID NO:10) sense oligonucleotide**

**5'CGCCAACTTATCGTCATCGTCGCCCG3' (SEQ ID NO:11) antisense oligonucleotide**

The three above-mentioned DNA constructs were used to produce their corresponding proteins in bacteria, which in turn were used as substrates for the enterokinase enzyme in the BRET assay (see below).

All three DNA constructs are subcloned in frame with the His tag sequences into the pQE plasmid (Qiagen, Mississauga, ON). This is done by introducing a His tag at the N-terminus of the constructs. The His tag serves to purify the different gene products from the bacterial lysats using the Nickel (Ni) beads based technology (QIAexpress kits, Qiagen, Mississauga, ON). All the constructs (Rluc gene alone, Rluc•EYFP and Rluc:enterokinase:EYFP) are subcloned into pQE-32 vector at the following restriction sites:

Table II

| Construct | Construct restriction site used | Vector restriction site used (pQE-32) |
|---|---|---|
| Rluc | NheI*-XmaI | BamHI*-XmaI |
| Rluc::EYFP | NheI*-NotI* | SmaI |
| Rluc:enteroldnase:EYFP | NheI*-NotI* | SmaI |

*all these sites were blunted before being subcloned into the pQE-32 plasmid using the Klenow enzyme under standard molecular biology techniques known to those skilled in the relevant art (Ausubel, F.M. et al., Current protocols in molecular biology, Vol. 1 (1995) John Wiley & Sons, Inc., and Sambrook, J. et al., Molecular Cloning: A laboratory manual, 2$^{nd}$ ed. (1989) Cold Spring Harbor Laboratory Press).

The DNA sequences for the two fusion constructs needed in this example are presented in Figures 57 and 58. Rluc DNA sequence could be found at GeneBank under the accession number : M63501

The DNA constructs in the pQE-32 vectors were then transfected into a suitable bacteria (M15) for protein expression. Bacteria was inoculated in 100ml of LB media and grown for 4 hours at 37 °C until they reached a density of 0.6 $OD_{600}$. Protein expression was induced by adding IPTG (1mM) into the bacteria cultures for 16 hours at 37°C.

The different proteins were purified from the bacterial lysats using 0.5ml Ni-NTA resin as described by a standard manufacturer's protocol (Qiagen, Mississauga, ON). The purified proteins were then desalted and concentrated (to a volume between 400-700ul) using a Centriprep-10 or -30 and Centricon-10 or 30 (Amicon, Beverly, MA) with aPBS + NaCl 250mM buffer. Protein concentrations were determined by the Bradford assay (Bradford, M.M., 1976, *Anal. BioChem*. 72: 248-254) using bovine serum albumin as a standard. Once purified, the proteins were used in the enterokinase BRET assay. The purity of the extracted protein, which is between 60-80%, was determined by SDS-PAGE.

The BRET assay was performed by mixing 60-100 μg of purified proteins for each well with the BRET assay buffer (PBS+Ca2+ 1mM Mg2+0.5mM +glucose (1g/l) + 10mM DTT) to a total volume of 150 μl. Each assay was conducted in triplicate (i.e. three wells) in 96-well Optiplate plate (Packard, U.S.A.). Half of the assays were done in the presence of two units of the enterokinase enzyme (Invitrogen, Carlsbad, CA). Coelenterazine h (Molecular Probes, Eugene, OR) at a concentration of 20 μM/50 μl was added to each well to start the bioluminescent reaction of Rluc. The final concentration of coelenterazine h was 5 μM. The total volume of the assay was 200 μl. After the addition of the coelenterazine h, the microplates were loaded into the BRETCount™. Detection was performed using the BRETCount at 30°C for four hours. For each time point and assay, ratios were calculated by dividing the emission light output at 550nm by the emission light output at 470nm.

An example of the results are as follows. Only the ratio for the construct Rluc:enterokinase:EYFP in the presence of the enterokinase enzyme (E) decreased over time (Figure 8) demonstrating that over time more and more constructs were cut. The ratios from the negative controls (Rluc and Rluc •EYFP) did not change over time when in the presence (E) or absence of the enterokinase enzyme.

In general, this assay demonstrates that BRET can be done with partially purified proteins. Furthermore, it demonstrates the utility of the fusion type of BRET by the insertion of specific amino acid sequences (protease site, phosphorylation site or a site that recognizes analytes e.g. Ca2+) in between the two moieties.

The TopCount is a microplate scintillation and luminescence counter sold by Packard Instrument (Meriden, CT). Unlike a classical counter which utilized two photomultiplier tubes (PMTs) in coincidence to reduce noise, TopCount employs a single-PMT technology and a time-resolved pulse counting for noise reduction to allow counting in opaque standard format microplate. The use of opaque microplates can reduce optical crosstalk to negligible levels. The TopCount comes in various formats (eg. 1, 2, 6, 12 detectors (PMTs)), which could read 1,2,6 or 12 samples simultaneously depending on the format.

A modified 2-detectorTopCount (BRETCount) was used to measure BRET. Modifications included positioning the bandpass interference filters under each TopCount PMT (between the PMT and the detector mounting plate), and adjusting theTopCount software to allow each PMT to read each well of a microplate. These changes allow the sequential detection of light at specific wavelengths emitting from the samples. Accordingly, for each well (or sample) we obtained two light output values corresponding to each filter. Beside its throughput (microplate reader), the TopCount is a temperature controlled instrument that is useful when performing cell-based assays or timecourse experiments.

The bandpass interference filters were designed based on emission spectra found in Figure 3b and 22. The two filters used were 1) for the acceptor emission (fluorophore): 550DF80 (Omega Optical Inc, Brattleboro VT); and 2) for the donor emission (bioluminescent protein): 470DF60 (Omega Optical Inc, Brattleboro VT).

BRET is measured by either dividing the light output for the 550 nm filters by the 470nm (acceptor/donor) or the opposite. The instrument parameters used for the BRETCount were luminescence SPC mode and read length of 1 sec for each PMT.

**EXAMPLE VII: CONSTITUTIVE PROTEIN-PROTEIN INTERACTION**

The interaction of the kaiB domain, which is involved in the circadian cycle of bacteria, with itself has been well documented (Xu, et al., 1999, Proc. Natl. Sci. USA, 96:151-156). This system is composed of three proteins identified as kai A, B, C, and is used to assay the BRET technology.

The kai based constructs (Xu, et al., 1999, Proc. Natl. Sci. USA, 96:151-156) were subcloned into mammalian expression vectors as follows: The two constructs (pT7/EYFP-kaiB; pT7/EYFP-kaiA) were cut with the restriction enzyme XbaI-HindIII to retrieve the resulting fusion gene (EYFP-kaiB; EYFP-kaiA). The remaining construct pT7/Rluc-kaiB was cut with the restriction enzyme NheI-HpaI to retrieve the Rluc-kaiB fusion gene. The fusion genes were then inserted into the multiple cloning site of pCDNA3.1 (-) zeo (Invitrogen, Carlsbad, CA) and digested with the same enzyme used to create the respective fusion gene.

The B RET ass ay was performed as follows. First, CHO-K 1 cells were transiently transfected using the kit LipofectAMINE™ (BRL/Gibco-Life technology, Geathersburg, MD) in 100mm dishes with the following combinations of the DNA constructs: pCDNA3.1/Rluc-kaiB alone; pCDNA3.1/Rluc-kaiB + pCDNA3.1/EYFP-kaiB, and pCDNA3.1/Rluc-kaiB + pCDNA3.1/EYFP-kaiA.

When combinations of vectors were used, a total amount of 8 μg of DNA was used. Each cell sample was transfected with 4 or 0.5 μg of each construct with enough carrier DNA (pCDNA3.1 (-) wild type ) to bring the amount up to a total of 8 µg.

Following transfection, the cells were washed 48 hours later with a mixture of PBS Ca2+ 1mM/Mg2+ 0.5mM + glucose 1g/l + aprotinin 2 µg/ml +DTT 10mM and counted. The transfected cells are distributed into a 96-well white Optiplate (Packard, U.S.A.) at a sufficient density to ensure a good bioluminescence reaction (in this example, 50 000 cells per well in 150 µl were used). The cells were plated into the same buffer that was used for the wash. To start the bioluminescence reaction. 50 µl of coelenterazine h (Molecular Probes, Eugene, OR) at a concentration of 20 µM was added to each well.

The final concentration of coelenterazine was 5µM. The microplates were then loaded into the BRETCount for the light emission measurements at 25°C using a read length of 1 sec per filter.

**EXAMPLE VIII: REGULATED PROTEIN-PROTEIN INTERACTION**

The cAMP response element binding (CREB) protein is a well known transcription factor involved in the cAMP pathway. When cellular cAMP levels increase by activation of the cAMP pathway, it triggers the activation of the protein kinase A (PKA) which in turns phosphorylates CREB. Once phosphorylated, CREB is recognized by other cellular factors like the CREB binding protein (CBP). The CBP then binds to CREB using a specific protein domain. Phosphorylated CREB alone or bound to CBP stimulates transcription activities of promoters that have a cAMP response element (CRE).

In the following example, we used two methods to increase the cellular cAMP levels:

First, a chemical method using forskolin that activates directly the enzyme that produces cAMP, the adenylate cyclase is used. Second, cAMP levels may be increased biologically using a G-protein coupled receptor (GPCR) and an agonist for the receptor. Examples of GPCRs that may be used include the calcitonin receptor and its agonist calcitonin, and the b2 receptor (adrenergic receptor) with its agonist isoproterenol. Both receptors are know to be coupled to a Gs G-protein which stimulates cAMP production. We also used a receptor (CCR5) that is coupled to a Gi protein which decreases cAMP levels.

The CREB domain needed for the experiment was found between amino acid 1-283. This region is well known to be recognized and phosphorylated by PKA on the ser133. This region has been amplified by PCR using the vector pFA-CREB (Stratagene, La Jolla, CA) as a template, which encodes the full length human CREB gene. The primers used for the PCR reaction are as follows:

oligonucleotide sense: 5'GGAAGATCTATGACCATGGACTCTGGAG (SEQ ID NO:12);

and

oligonucleotide antisense: 5' CCAAGCTTTGCTGCTTCTTCAGCAGG (SEQ ID NO:13).

To facilitate the subclonlng, the oligonucleotides were engineered with two different restriction sites (one per oligonucleotide) at their 5' end. The sense oligonucleotide has a Bgl II restriction site and the antisense oligonucleotide has a Hind III restriction site. Hence, after the amplification, the amplified product was purified and digested with these enzymes. The pT7/EYFP-kaiB (Xu, et al., 1999 *Proc. Natl. Acad. Sci*. USA, 96:151-156) was digested using the same restriction enzyme to remove the kai B gene. The amplified CREB domain was then subcloned at the C-terminal of EYFP in the pT7/EYFP construct. A linker of two amino acid was created between the EYFP and the CREB domains.

The novel fusion protein (EYFP-CREB) from the bacterial pT7 expression vector, was then subcloned into the mammalian expression vector pCDNA3.1 (+) zeo (Invitrogen, Carlsbad, CA). For this purpose, the pT7 EYFP-CREB was digested with XbaI and HindIII and the pCDNA3.1 was digested by NheI (which is XbaI compatible) and HindIII.

A SV40 nuclear localization signal (NLS) was introduced at the C-terminus of the fusion EYFP-CREB gene in order to ensure that the construct once expressed, was localized in the nucleus. We anneal two new complementary oligonucleotides encoding the following SV40 NLS : Pro-Lys-Lys-Lys-Arg-Lys-Val-stop codon:

Oligonucleotide sense: 5' GATCCCCCAAGAAGAAGAGGAAGGTGTGACCGGTC (SEQ ID NO:14)

Oligonucleotide antisense: 5' TCGAGACCGGTCACACCTTCCTCTTCTTCTTGGGG (SEQ ID NO:15)

Once the oligonucleotides were annealed, they form a double strand DNA having two cohesive restriction sites at both ends (BamHI at the 5' and Age I at the 3'). The construct pCDNA3.1-EYFP-CREB was digested using these enzymes and the annealed double oligonucleotide was subcloned. A linker region of eight amino acids was created between the CREB and the NLS. The final construct is referred to as pCDNA3.1/EYFP-CREB-NLS.

The CBP domain needed for the experiment was found between amino acid (462-661). This region is well known to bind phosphorylated CREB (Chrivia, J.C., et al., 1993, Nature, 365:855-859; Shih, H-M, et al., 1996, Proc. Natl. Acad. Sci. USA, 93:13896-13901; Kwok, R.P.S., et al., 1994, Nature, 370: 223-226; Arias, J., et al., 1994, Nature, 370,:226-229). This region has been amplified by RT-PCR using a cDNA library of human placenta. This library was made using mRNA from human placenta (from Clontech, Palo Alto, CA) and the Marathon ready kit (Clontech, Palo Alto, CA). The oligonucleotides used are as follows:

oligonucleotide sense 5' CGGGTACCGACAGGGCAACAGAATGCC (SEQ ID NO:16)

oligonucleotide antisense 5' ATGCGGCCGCTATCTTGTAGATTTTCTCTGC (SEQ ID NO:17)

To facilitate the subcloning, the oligonucleotides were engineered with two different restriction sites (one per oligonucleotide) at their 5' end. The sense oligonucleotide has a Kpn I restriction site and the antisense oligonucleotide has a Not I restriction site. After the amplification, the amplified product was purified and digested with these enzymes. The pCDNA3.1/ Rluc-EYFP (made at BioSignal from the pT7/ Rluc-EYFP vector) was digested using the same restriction enzyme to remove the EYFP gene. The amplified CBP domain was then subcloned at the C-terminal of Rluc into the pT7/Rluc-EYFP construct lacking the EYFP gene. A linker of eight amino acid was created between the EYFP and the CREB domain.

A SV40 nuclear localization signal (NLS) was introduced at the C-terminus of the fusion Rluc-CBP gene in order to ensure that the construct once expressed, was localized in the nucleus. Two new complementary oligonucleotides were annealed that encoded the following SV40 NLS: Pro-Lys-Lys-Lys-Arg-Lys-Val-stop codon:

oligonucleotide sense corresponding to the NLS: 5' TCGAGGATTCCCCAAGAAGAAGAGGAAGGTGTAGGTTT (SEQ ID NO:19)

oligonucleotide antisense corresponding the NLS.: 5' AAACCTACACCTTCCTCTTCTTCTTGGGGAATTC (SEQ ID NO:20)

Once annealed, the oligonucleotides form a double strand DNA having one cohesive restriction site at the 5' end XhoI and a blunt end at the 3'. The pCDNA3.1-Rluc-CBP was digested using XhoI enzyme and PmeI (generating a blunt end). The annealed double oligonucleotide was subcloned into these sites. A linker region of six amino acids was created between the CBP and the NLS. The final construct is referred to as pCDNA3.1/Rluc-CBP-NLS.

DNA sequences for all the constructs needed in this example are presented in Figures 60 and 61.

**BRET assay:**

The pCDNA3./Rluc-CBP-NLS and pCDNA3.1 /EYFP-CREB-NLS DNA constructs were transiently transfected into CHO-K1, CHO-CCRS or CHW-β2 cell lines using LipofectAMINE™ (BRL/Gibco-Life technology, Gaethersburg, MD) using equal amount (4 μg) of DNA for each construct in 100mm dishes.

**BRET assay using the transfected cells in suspension.** At 48 hours post-transfection, the transfected cells were starved for 2 hours in a mixture of PBS + Ca2 + 1 mM/Mg2 + 0.5mM + glucose 1 g/l +aprotinin 2 μg/ml at 37°C before being harvested, washed with a mixture of PBS Ca2+/Mg2+ + glucose + aprotinin 2 μg/ml and counted. For the BRET assay, to ensure a sufficient signal, 50 000 cells per well was used in 96-well white Optiplates (Packard, Meriden, CT).

**BRET assay using transfected cells attached onto the bottom of the microplate.** In some experiments, cells were harvested at 24h post-transfection and seeded onto a 96-well white CulturePlate™ microplates (Packard, Meriden, CT) at a density of 25 000 cells per well. At 48 hours post-transfection, the transfected cells were starved as previously mentioned, washed and then analyzed without being harvested.

The assay conditions for both methods (cell in suspension or attached), cells were in PBS Ca2+ Mg2+ + glucose + aprotinin 2 μg/ml in volume of 100ml. Ligands or forskolin in PBS Ca2+Mg2++glucose (at various concentrations see graphs) was added to the cells to make an intermediate volume of 150 μl. To this, 50 μl of coelenterazine h (20mM in PBS Ca2+ Mg2++ glucose + aprotinin 2 μg/ml) was added in each well to start the bioluminescent reaction (final concentration of coelenterazine h 5 μM). Total volume of the reaction 200 μl. After the addition of coelenterazine h in each well, the microplate was loaded into the BRETCount for detection at 25°C. Each well was read using both filters for 1 second each.

**Effect of adenylate cyclase activation by forskolin or calcitonin receptor on cAMP sensor**

The CHO-K1 cells were transfected with both constructs. At 48 hours post-transfection, the cells were starved for 2 hours, washed, harvested and counted. A total of 50 000 cells was added to a 96-well microplate. Forskolin was added to each well to a final concentration of 10 μM. Coelenterazine h was then added in each well to start the reaction. The microplate was loaded into the BRETCount and light outputs at 470nm and 550nm were determined at several points to determine a timecourse (fig. 10). As for forskolin, the agonist calcitonin was also used to increase cAMP levels. The CHO-K1 cells express the calcitonin receptor endogenously, and once activated, couples to the Gs type G-protein. The Gs type G-proteins are known to activate adenylate cyclase and hence increase cellular cAMP levels. In this example, 2 nM and 10nM of calcitonin were used. The results demonstrate the sensor is sensitive to changes of cAMP level. Ratios increased over time in presence of calcitonin or forskolin compared to wells that have not been induced (buffer alone). Each time point represents mean +/-SD of four wells (assays are done in quadruplicate).

The timecourse experiment of Figure 10 was repeated using attached cells this time and various concentration of calcitonin (ranging from 0 to 3.1 X $10^{-8}$ M). Dose response curve (Figure 11) was built using the data point at 45 minutes after addition of coelenterazine. The dose response determined an EC50 of 0.027 nM which was close to those found in literature (0.03-0.06 nM) for a functional assay. Each time point represents mean +/- SD of four wells (assays are done in quadruplicate).

**Effect of the activation of an overexpressed GPCR on the cAMP sensor**

Instead of using an endogenous GPCR or forskolin to increase cellular cAMP levels, the constructs were transfected into a stable cell line expressing an heterologeous GPCR. The CHW- β2 cell line was used for this purpose. It expresses the human adrenergic β2 GPCR (level of expression: 0.77 pmole/mg of membrane protein) and like calcitonin receptor it is coupled to Gs G-protein. It had been shown that this receptor is functionally coupled to the cAMP pathway: upon binding of its agonist the isoproterenol, it triggers theproductionofcellular cAMP. At 48 hours post-transfection of the sensor constructs, the cells were starved, washed, harvested and counted as mentioned above. A total of 50,000 transfected cells per well was added into a 96-well white Optiplate (Packard, Meriden, CT) along with two concentrations of isoproterenol. In several wells, the cells were incubated with forskolinas control (as above). Figure 12 represents the induction of β2 receptors by isoproterenol over time. Again, the ratios increased in the presence of isoproterenol or forskolin, indicating that the two moieties (Rluc and EYFP) are brought in close proximity when cAMP levels increase.

Figure 13 represent the dose response of isoproterenol in the transfected CHW-β2 cells from another experiment. In this case, the transfected cells were analysed at 24 hours post-transfection. EC50 of 0.013 nM was calculated from the curve. This value is similar to values found in literature for b2 receptor using a functional assay known to those skilled in the art.

**Gi coupled receptor**

The cAMP sensor could be used to monitor receptor activation (upon ligand binding) for the Gs-coupled receptor since the Gs-coupled receptors are known to increase cellular cAMP levels. This experiment examined if the cAMP sensor be used for Gi-coupled receptor. These receptors couple to different G-proteins that decrease cellular cAMP levels once the ligand binds to its receptors. A stable cell line that constitutively expressed the chemokine CCR5 receptor was used (BioSignal, Montreal, PQ). The CCR5 is coupled to a Gi type G-protein once it is activated. Two different approaches were used to determine if the cAMP sensor could monitor the decreased cAMP levels.

The first method used transfected CHO-CCR5 cells plated in 100mm dishes. These cells were starved and seeded in 96-well plates as previously described. They were then induced with forskolin at a final concentration of 1uM to increase their cAMP content and with MIP-1β, which is a selective agonist for the CCR5 receptor. Coelenterazine was added in each well to start the reaction.

Alternatively, the cells were not starved and only MIP-1β was added. Since cAMP levels are naturally high in cells we did not starve the cells to "artificially" increase cAMP level with forskolin in order to detect the inhibitory effect of the CCR5 on the adenylate cyclase.

Figures 14 and 15 represent the above two approaches, respectively. In both cases, the activation of the CCR5 by its agonist was monitored. Ratios decrease over time in the presence of the agonist MIP-1β.

**EXAMPLE IX: PROTEIN-PROTEIN INTERACTION USING TYROSINE KINASE RECEPTOR (TKR)**

This assay /sensor is similar to the CBP:CREB assay. It detects TKR activities (Heldin, C.H., et al., 1998, Biochim. Biophys. Acta, 1378: F79-F113). The TKRs are a family of receptors that share one transmembrane region (instead of 7 like the GPCRs) and have tyrosine kinase (TK) activities. Examples of this family include EGF, PDGF, insulin, etc. The TK domain is localized at the C-terminus of the protein inside the cell. This domain is regulated by the ligand binding domain localized at the N-terminus (outside the cell). Upon ligand binding, the receptor homo-dimerizes and the TK domain is activated. Once activated, one receptor will phosphorylate (on a tyr) its partner (autophosphorylation). This phosphorylation is then recognized by cellular adapterproteins which in turn activates downstream effectors. Two of these adaptor proteins were engineered: SHC and Grb2. Both of these proteins could recognize and bind to a tyr phosphorylated receptor (at different sequences). Most often (at least for the EGF and PDGF receptors), these proteins act sequentially (Yajnik, V., et al., 1996, J. Biol. Chem., 271:1813-1816; Sasaoka, T., et al., 1996, J. Biol. Chem., 271:20082-20087; Gram, H., et al., 1997, Eur. J. Biochem., 246:633-637; Rozakis-Adcock, M., et al., 1992, Nature, 360: 689-692; Sakaguchi, K., et al., 1998, Mole. Endocrin.,12: 536-543). The adaptorprotein SHC first recognized and bound to the tyr phosphorylated receptor (directly at the specific phosphorylation site). Theprotein domain responsible for the binding of SHC to the receptor is found at the N-terminus of SHC and it is called phosphotyrosine binding (hereafter PTB). This domain has a different sequence signature compared to the well known prototype SH2 which shares the same phosphorylated tyr binding activity (Yajnik, V., et al.,1996, J. Biol. Chem., 271: 1813-1816; Schaffhausen, B., 1995, Biochem. Biophys. Acta, 1242: 61-75; Songyang, Z., et al., 1993, Cell, 72: 767-778). Once bound to the receptor, SHC is then phosphorylated (at a specific tyr) by the receptor. The Grb2 then recognizes and binds the phosphorylated SHC using its SH2 domain (SH2 of Grb2:SHC has also a SH2 domain but it is used for other signal pathway) (SH2: Src homology 2). In our assay, we measured this link between SHC and Crb2 in order to monitor the RTK activity.

In order to build the assay, it was necessary to fuse the protein domains involved in the RTK activity, with the Rluc and the EYFP. The following is a description of the procedure.

**EYFP-SH2**

SH2 was fused at the C-terminus of BYFP. SH2 domain of Grb2 (amino acid 49-164) was amplified by PCR (standard technique) using as template an human EST (expressed sequence tag) and specific oligonucleotides:

**Oligonucleotide sense : 5= GGAAGATCTCCCAAGAACTACATAGAAATG (SEQ ID NO:21)**

**Oligonucleotide antisense: 5= CCAAGCTTTCAGAGGGCCTGGACGTATG (SEQ ID NO:22)**

EST No: human Image clone no. 26670

To facilitate the subcloning, the PCR oligonucleotides were engineer with two different restriction sites (one per oligonucleotide) at their 5=end (underlined). Sense oligonucleotide has a Bgl II and antisense oligonucleotide has a Hind III site. Hence, after the amplification, the amplified product was purified and digested with those enzymes. The pT7/EYFP-kaiB (Xu,) was digested using the same restriction enzyme to remove kai B gene. The amplified CREB domain was then subcloned at the C-terminal of EYFP in the pT7/EYFP. A linker of 2 amino acid was created between the EYFP and the CREB domain.

The EYFP-SH2 fusion gene was then subcloned into the pCDNA3.1 (-) hygromycin mammalian expression vector using the restriction sites XbaI and HindIII.

**Rluc-PTB**

PTB domain of SHC was fused to the C-terminus of Rluc. PTB domain of SHC (amino acid 111-487 of the $p66^{SHC}$ variant) was amplified by RT-PCR using as template a HeLa cDNA library. This library was made using total RNA from HeLa cell and the SuperScript II Rnase $H^-$ Reverse Transcriptase (BRL/Gibco-Life Technologies, Gaethersburg, MD) with the oligo dT primer as described in manufacturer protocol.

PCR was perform using standard technique known to those skilled in the art and the following primers:

**Oligonucleotide sense 5' CGGGTACCGATGAACAAGCTGAGTGGA (SEQ ID NO:18)**

**Oligonucleotide antisense: 5' TAGCGGCCGCTCAGGGCTCCCCTCGGAGC (SEQ ID NO:24)**

To facilitate the subcloning, the PCR oligonucleotides were engineered with two different restriction sites (one per oligonucleotide) at their 5' end. Sense oligonucleotide has a KpnI and antisense oligonucleotide has a NotI site. Hence, after the amplification, the amplified product was purified and digested with those enzymes. The pCDNA3.1/Rluc-EYFP (this construct was made at BioSignal (Montreal, Canada) using the pT7/Rluc-EYFP from Vanderbilt University using the restriction sites NheI-NotI of pCDNA3.1 zeo (+)) was digested using the same restriction enzyme to remove EYFP gene. The amplified PTB domain was then subcloned at the C-terminal of Rluc in the pT7/Rluc. A linker of 8 amino acid was created between the Rluc and the PTB domain.

DNA sequences for the two constructs needed in this example are presented in Figures 62 and 63.

**BRET assay**

The two constructs pCDNA3.1/Rluc-PTB and pCDNA3.1/EYFP-SH2 were co-transfected into GH4-C1 cell line with LipofectAMINE (BRL/Gibco-LifeTechologies, Gaethersburg, MD) using manufacturer protocol in 100mm dishes. The GH4-C1 cell line was used because it is known that these cells express endogenously the EGF receptors needed for the assay. At 48 hours post transfection, transfected cells were starved in MEM without serum for 2 hours, washed with PBS Ca2+/Mg2+ + glucose + aprotinin, harvested and seeded in 96-well microplate at a cell density of 50,000 cell perwell in the PBS Ca2+/Mg2++glucose+aprotinin assay buffer. The agonist EGF was then added at various concentration to make at this point a total volume of 150ml. 50ml of coelenterazine h (at a concentration of 20mM) was added in each well to start the bioluminescent reaction. Microplates were loaded in the BRETCount for the measurement of Rluc and EYFP emissions. Read time for each filter: 1sec. Temperature of detection was at 25 °C.

Figure 16 represents a dose response of EGF on the transfected cells at 45 minutes post induction (with EGF). From this curve, a EC50 of 0.92nM was calculated which is similar of the value found in literature. This sensor is a valuable tool to monitorTKR activity and hence would be useful for drug discovery.

## EXAMPLE X: PROTEIN-PROTEIN INTERACTION USING TOPAZINSTEAD OF EYSP

Evidence that GPCR associate to form dimer or multimeric species remain challenging and rely solely on biochemical experiments performed *in vitro.* In this example, we set to investigate if GPCR dimerization occurs in live cell. For that purpose, the moieties were fused in frame at the C-terminus of b2 receptor. Hence two fusion constructs were generated: 1- b2-Rluc and b2-Topaz. Topaz is another GFP variant having similar spectral proprieties when compared to EYFP.

The following assay demonstrates the results showing β2 dimerization in live cells using BRET.

The DNA of both Rluc (from pRL-CMV, Promega) and Topaz (pGFPtpz-N1, Packard) genes were inserted at the C-terminus of the human β2 gene without its stop codon, having spacers of four and five amino acids, respectively. Topaz is used instead of EYFP because it has a higher extinction coefficient and quantum yield. Both fusion proteins were then subcloned into a pRSV vector to permit their expression in mammalian cells.

HEK-293 and CHO-K1 cells were transiently transfected using Ca2+-phosphateprecipitation and LipofectAMINE™ (BRL/Gibco, Gaethersburg, MD) respectively in 100mm dishes. Transfection efficiencies between 40-60% were generally obtained using this technique (when analyzed using a GFP reporter vector and fluorescence microscope) for CHO-K1 cells and about 20-30% for HEK-293 cells. The cells were always transfected with the same amount of total DNA.

The assays were performed 48 hours post transfection in quadruplicate. The cells were harvested using versene (PBS+EDTA), counted and diluted in PBS (containing Ca2+/Mg2+) to about 50,000 cells per well in a white Optiplate (Packard, Meriden, CT). The assays may be performed using either suspension or adherent cells. When the assays were performed with adherent cells, the cells were harvested from 100mm dishes 24h post-transfection, counted and seeded into a 96-well plate before the detection step performed the following day. Light emission was triggered by the addition of coelenterazine 5 uM. Emissions were detected at various times after addition of the coelenterazine using the BRETCount. Parameters used for the BRETCount were luminescence SPC mode, a read length of 1 sec for each wavelength at 25°C, and 470DF60 550DF80 filters.

### 1- Pharmacological characterization of the fusion b2-Rluc and -Topaz proteins.

For the pharmacological characterization. 293 cells were transfected with either a β2-wt expressing vector, β2-Rluc expressing vector or β2-Topaz expressing vector. Saturation binding experiments using isoproterenol were performed on the transfected 293 cells. Table III shows the $K_D$ obtained from the saturation curves. Values obtained for the fusion proteins were significantly different from β2 wt receptor but fusion proteins still show a high affinity for the agonist.

Table III:

| $K_D$ generated from the transient transfected 293 cells (pM). | | | |
|---|---|---|---|
| | *β2-wt* | *β2-Rluc* | *β2-Topaz* |
| $K_D$ | 37 | 59 | 113 |

### 2- Detection of β2 receptor dimerization using BRET.

For the detection of β2 receptor dimerization, 293 cells were transfected with β2-Rluc vectoraloneor in combination with β2-Topaz or CCR5-Topaz. The results of this assay (Figure 17), also included the results of tri-transfections where β2-Rluc and β2-Topaz transfected cells were further transfected with β2 wt or V2 receptor DNA. At 48h post-transfection, the cells were harvested, washed, counted and distributed into a 96-well plate (50 000 cells per well) inPBS. After the addition of coelenterazine, microplates were read at different times (Figure 17). In Figure 18, the cells were harvested at 24h post-transfection, counted and distributed into 96-well plates before the analysis at 48h. By using this procedure, the cells adhere to the bottom of the well before the analysis. Although this process does not alter the cells, the ratios obtained tend to be lower compared to analysis performed with harvested cells.

Figures 17 and 18 demonstrate that the BRET ratio is increased when β2-Rluc is co-expressed with β2-Topaz compared to β2-Rluc alone, indicating that these fusion receptors were in close proximity (dimerized). Hence, even in absence of agonist, part of the receptor pool is found in a dimerized form. This association was stable for at least 20 minutes (Figure 17) even as the Rluc/coelenterazine reaction continues. This association could be perturbed by the addition of β2 wt DNA construct (at 5 times the amount of the fusions) during transfection (diamond, Figure 17). In contrast, V2 receptor expression had no effect on the dimerization of β2 receptors. CCR5 receptor was fused to Topaz as for β2, and used as negative control (Figure 18). This fusion protein was characterized pharmacologically (data not shown). When co-transfected with β2-Rluc, a significant increase of the BRET ratio was observed.

### 3- Kinetics of β2 receptors dimerization

The effect of the agonist isoproterenol on the dimerization state of β2 receptors was determined. After harvesting the cells, cells were pre-incubated for 10 minutes with 1 mM of isoproterenol at 37°C. After this period of time, the cells were distributed into the 96-well plate and coelenterazine was added to start the reaction. BRET analysis was then proceeded at25 °C in the BRETCount. Figure 19 shows that BRET ratio increased for 15 minutes and then started to decline.

### 4- Dose-response experiments.

Doseresponseexperiments on the transiently transfected cells was also performed. At 48 hours post transfection, the cells were harvested, seeded into 96-well plate and incubated with various concentration of isoproterenol. After 10 minutes at 37 °C, coelenterazine was added to start the Rluc reaction. The addition of the agonist generated a dose-response curve with a EC50 of 2nM (Figure 20). This EC50 value obtained by BRET analysis is similar to the one obtained in ligand binding analysis. Similar results were obtained when assays were repeated using adherent cells instead of suspended cells.

The use of the BRET technology in the above examples demonstrates for the first time β2 receptor dimerization in live cells. Previously, β2 dimerization had only been shown by immunoprecipitation experiments. The use of the BRET technology also showed that the addition of agonist affects dimerization by the modification of the BRET ratio. BRET assays using other agonists, antagonists and even inverse agonists (data not shown) were also performed and have generated pharmacological data. The above examples reflective of the application of the BRET technology.

From the foregoing description, one skilled in the art can easily ascertain the essential characteristics of this invention, and without departing from the spirit and scope thereof, can make various changes and modifications of the invention to adapt it to various usages and conditions. Consequently, such changes and modifications are properly, equitably, and "intended" to be, within the full range of equivalence of the claims to follow.

### EXAMPLE XI: DEMONSTRATION THAT THE METHOD IS AMENABLE TO AUTOMATION.

To test the feasibility of BRET ratio imaging, images were collected with a cooled-CCD cameraof E. coli cultures expressing certain constructs. For these applications, the ratio of luminescence intensities at 480 nm to 530 nm can be measured to determine the magnitude of BRET, and to correct for differences in overall levels of expression of RLUC and EYFP fusion proteins.. Figure 4 shows images of liquid E. coli cultures as seen through 10 nm bandpass interference filters centered at 480 nm and 530 nm. In the cultures containing a control strain (RLUC alone) or a non-interacting combination (RLUC•KaiB & EYFP•KaiA), there is much less light emitted at 530 nm than at 480 nm. In the fusion construct (RLUC•EYFP) or the interacting combination (RLUC•KaiB & EYFP•KaiB), the amount of light emitted at 480 nm and 530 nm are roughly equal, as would be predicted from the spectra depicted in Figures 1 and 2(b2). These images can be quantified, as shown in figure 5. The 530nm/480nm ratios of luminescence for the RLUC control and the non-interacting KaiB & KaiA couple are low (about 0.4), whereas the 530nm/480nm ratios for the fusion construct and for the interacting KaiB fusion proteins are above 0.9.

Because of the better throughput of the BRETCount compared to the LumiCount, it is possible to develop and test various assay parameters to improve the detection of the BRET signal and to adapt BRET detection for high throughput screening (HTS). The assay parameters were characterized. Parameters tested were: cell numberper well: type of media: the use of protease inhibitor: effect of the pH: effect of HEPES: effect of BSA: effect of DMSO: other.

CHO-K1 cells were transiently transfected using LipofectAMINE (BRL/Gibco Life Sciences, Gaethersburg, MD) in 100mm dishes according to the manufacturer's protocol (0.2mg DNA per construct + X mg of carrier DNA for a total of 8 mg). Transfection efficiencies between 40-60% using this technique were obtained (when analyzed using a GFP reporter vector and fluorescence microscope). 48 hours post transfection, cells are harvested using a low concentration of trypsin, counted and diluted in D-PBS (containing $Ca^{2+}/Mg^{2+}$). For most of the examples given below, 50,000 cells per well were used in white Optiplate (Packard, Meriden, CT). The assays were performed in quadruplicate in a total of 200µl. Light emissions were triggered by the addition of coelenterazine h (5µM; addition of 50µl of a stock 20µM).

The assays were conducted using the BRETCount at various times after the addition of the coelenterazine. Parameters used for the BRETCount were: luminescence SPC mode, a read length of 1 sec for each wavelength at 25°C, 470DF60 and 550DF80 filters.

Spectral analysis of BRET reaction were performed using a Spex instrument (Instrument S.A.) with the excitation lamp turned off, an integration time of 0.4sec, Scan 400-600nm, emission slit 10-12nm, step 2nm, at 25°C (fig. 22).

To follow are examples of assays demonstrating the effect of changing different assay parameters:

**1- Cell number per well.**

A standard BRET assay was performed (Figure 21) using the BRETCount with the assay parameters used for the LumiCount (modified LucLitebuffer, coelenterazine h 5 µM, white Optiplate, total volume of 200µl, etc).

BRET ratios were very stableovertime. The positive control slightly increased and at the same time both negative controls slightly decreased. Rluc emission and energy transfer peaks change their shape during the light emission (see Figure 22). The ratio increased over time because the Rluc/coelenterazine reaction decays producing less light but the energy transfer is still at its maximum efficiency. The effect of cell number per well is shown in Figure 23. Different total cell numbers were distributed into wells of the microplate. After the addition of the coelenterazine h, the microplate was read at different times. Figure 23 shows that number of cells per well has little effect on the ratios. As little as 2000 cells per well (in a 96-well plate) were used without significant changes in the ratios. Note that at this low number of cells, observed large variations in the BRET ratios late (over 75min.) in the assays were observed, which may be attributed to the numerous plate movements and cell adhesion to the vessel.

**2.- Type of media.**

The effect of the type of media on the luminescence of the Rluc was assayed in absence or in the presence of various amount of DTT. Transfected CHO cells were collected and redistributed in 96 well plate using different media conditions (Figures 24-26). Luminescence timecourses at 470nm (TopCount) were performed at 25°C upon coelenterazine addition. The effects of three different media were assayed: PBS $Ca^{2+}/Mg^{2+}$ (Figure 24), PBS $Ca^{2+}/Mg^{2+}$ + glucose (Figure 25) and DMEM without phenol red (Figure 26).

In absence of DTT, PBS alone gave higher Rluc long term emission and hence the best long term Rluc emission stability, followed by PBS+glucoseandDMEM. Similar results were found in presence of 100mM DTT: PBS alone gave higher long term counts. As expected, increasing the concentration of DTT improved Rluc stability in all three media. Unfortunately, high amounts of DTT (50 and 100mM) which gave the best stability, damaged the cells: viability was assayed using trypan blue dye and decreased more than 20% over a period of 2 hours. A good compromise between viability (less than 15%) and Rluc stability was achieved at 10mM. This concentration was chosen for the BRET assays. The addition of DTT modifies the BRET ratio of the positive and negative controls but does not modify the dynamic range (DR). However, DTT is not necessary for the BRET reaction to occur efficiently: as it acts as a boost for the Rluc long term emission.

**3- The use of protease inhibitor.**

In order to avoid cell damage during the assay procedure and timecourse experiments, an analysis was conducted to determine whether the use of aprotinin perturbs ratiometric measurements. Aprotinin is a protease inhibitor which is often used during cell culture to maintain cell integrity. Aprotinin at a concentration of 2µg/ml, did help maintaining cell viability over a time period of 2 hours. In the presence of aprotinin, viability decreased by less than 10% compared to more than 15% in absence of the protease inhibitor. During the same period of time, the BRET ratios were not affected significantly by the presence of aprotinin (data not shown).

**4- Effect of the pH.**

The effect of cell media pH on BRET ratios was analyzed. For that purpose, we made stable transfections of Rluc alone and Rluc•EYSP cell lines. Stable Rluc and Rluc•EYFP CHO cell lines were seeded using (PBS) media having different pH. Upon substrate addition, a BRET timecourse was started and the ratios werecalculated. Figure 27 shows the effect of media pH on BRET ratios. The ratios tended to increase as pH decreased. This was true for the Rluc alone but not necessarily for the Rluc•EYFP fusion. The best signal to noise was found at pH 7.1 which is close to the PBS pH (7.2). These results suggest that a pH stabilizer like HEPES could be used during drug screening and that the addition of certain compounds (eg. very acidic or basic peptides) could change the pH sufficiently to modify BRET detection.

**5- Effect of HEPES**

Because pH stabililty is important in order to get meaningful ratio (see above), an analysis was conducted to determine whether Hepes could be used in a BRET assay without disrupting the energy transfer. Cells stably expressing Rluc or Rluc•EYFP. were incubated during BRET detection in presence or absence of Hepes 10mM pH 7.2. The effect of Hepes in presence of DTT was also assayed. Figures 28 and 29 show the effect of Hepes on Rluc and Rluc•EYFP fusion constructs, respectively. Hepes modified the ratio without disrupting it. The ratios were increased in presence of HEPES for both the Rluc and Rluc•EYFP constructs, but DR did not change. Furthermore, there was an additional effect on the ratio when the assay was performed in the presence of DTT and HEPES, but again without changing the DR.

**6- Effect of DMSO**

DMSO is often used as a solvent to dissolve the compounds and as a result could find its way into a given assay mixture. Various quantities of pure DMSO were added directly into wells of the microplate. Total volume was kept at 200 µl for each condition. Figure 30 shows theeffecton the energy transfer in presence of HEPES. It was observed that only a small effect resulted with Rluc emissions in the presence of DMSO (data not shown).

**EXAMPLE XII: IN VIVO APOPTOSIS SENSOR**

ApoptosisorPCD (programmed cell death) is a normal cellular process found in most cell types. It is involved in many aspects of cell homeostasis and organism development (e.g. PCD is responsibleof the modeling (shape) of an organ during morphogenesis) (Nicholson, D.W. and Thornberry, N.A., 1997, TIBS, 22: 299-306; Kinloch, R.A., et al., 1999, TIPS, 20: 35-42) . Very often, inappropriate PCD is found in many diseases like cancer, neurodegenerative diseases, etc. PCD is a regulated way for cells to die at a specific time without stimulating the defense mechanism of the organism (e.g. inflammation) and hence distinct from the other type of cell death, necrosis, generally resulting from cellular accident.

Many proteins and lipids are involves in the PCD. Among them, the caspases which are part of a large family of cysteine proteases (Nicholson, D.W. and Thornberry, N.A., 1997, TIBS, 22: 299-306). Caspases recognize and cleave specific amino acid sequences (usually a sequence of 4-5 amino acids) and are part of a protein cascade that ends by activation of effectors involved in the degradation of protein (by activating general proteases) and DNA. Specific receptors at the plasma membrane e.g. fas-R, TNF-R) and their adaptor proteins trigger the apoptotic signal by the activation of a first line of caspases (caspase -2, 8, -10) depending of the cell type). These activated caspases then activate by cleaving a specific amino acid sequence, other caspases (-3, -7, -9, -4). These, in turn could activate other caspases and act also on cellular targets to generate the cell disruption. Apoptosis can be triggered also by chemical agents in some cell types (e.g. thapsigargin on HL-60 cell line or staurosporine on HeLa cell line).

A BRET apoptosis sensor was engineered by introducing a caspase-3 recognition site in the linker regionof the Rluc-EYFP fusionconstruct. Upon inductionof apoptosis, caspase-3 should recognize and cleave the linker region which should separate Rluc of EYFP and thereby decrease the BRET ratio over time.

The caspase-3 site was introduced using annealed complementary oligonucleotides. The two complementary oligonucleotides encoding the caspase-3 site: Gly-Asp-Glu-Val-Asp-Gly. Only the Asp-Glu-Val-Asp used were: Oligonucleotide sense: 5' GATCCGGCCGACGAGGTGGACGGCGAATCCGCGGTAC (SEQ ID NO:23) and Oligonucleotide antisense: 5' CGCGGATTCGCCGTCCACCTCGTCGGCCG (SEQ ID NO:25)

Once annealed, the oligonucleotides form a double strand DNA having two cohesive restriction sites at both ends: 5' BamHI and at 3' KpnI. pT7/ Rluc-EYFP was digested using BamHI and KpnI and the annealed double oligonucleotide was subcloned into the pT7/ Rluc-EYFP vector. The Rluc-EYFP and the Rluc-caspase-EYFP fusion genes from pT7 vector were then subcloned into the mammalian expression vector, pCEP4 (Invitrogen) using NheI and NotI restriction enzymes.

DNA sequences for the two constructs needed in this example are presented in Figures 57 and 59.

pCEP4/Rluc-EYFP and pCEP4/Rluc-caspase-EYFP DNAs were transfected into HeLa cell using LipofectAMINE (BRL/Gibco-Life technologies) in 100mm dishes as described in the manufacturer's protocol. 24 hours post-transfection, transfected cells were harvested and distributed into 96-well microplates (white Optiplate from Packard) at a density of 30,000 cells per well. The following day, cells are induced to undergo apoptosis using staurosporine 1mM dissolved in ISCOVE media (BRL/Gibco-Life technologies) without serum and phenol red at 37°C (total volume here is 100ml) for 5 hours. 50 ml of BRET buffer (PBS $Ca^{2+}/Mg^{2+}$ + glucose without aprotinin) and 50ml of coelenterazine h (20mM) is added to start the bioluminescent reaction. Light emissions are detected using the BRETCount (read length sec for each filter, temperature 25°C). Data was collected 10 minutes after addition of coelenterazine h. The assay was done in quadruplicate.

Figure 31 gives representative BRET ratio changes in HeLa cells after 5 hours of apoptosis induction by staurosporine. A significant ratio change (0.1 unit) occurs when cells transfected with theapoptosis sensor were induced by staurosporine. Ratios decreased.

## EXAMPLE XIII: MINI-SCREEN ASSAY

A major application of BRET is drug screening in live cells. A target (in this example, β2 receptor) was stably transfected into mammalian cells (in this example, CHW) and co-expressed in combination with a transiently transfected sensor or a reporter gene (in this example, the cAMP sensor CBP:CREB). Cells were then seeded in 96-well microplate in a suitab le buffer. Mini-screens were performed either with attached cells (on the bottom of the well) orusing cell suspensions. If attached cells were used, a waiting period of at least several hours at 37°C was allowed for so that cells could adhere to the bottom of the wells. Once adhered, test compounds were added to the cells and incubated at a suitable temperature for the assay (generally between 15-42°C). Generally but not necessarily, compounds were dissolved in an organic solvent (e.g. DMSO). Compounds can be pooled before addition to the wells. Coelenterazine was then added to start the bioluminescent reaction, followed by emissions detection.

While in this example, the cells were transiently transfected with the sensor in a stable cell line expressing the receptor, alternative embodiments may use cells transiently transfected with target in a s table cell line expressing sensor, or a cell line stably expressing both the receptor (target) and the sensor.

48 hours post-transfection, transfected CHW-b2 were seeded at a density of 50 000 cells per well in a white Optiplate (Packard). 10 ml of different compounds dissolved in DMSO at various concentrations, were added in each well to a total volume of 150ml (cell+buffer+compound). The first row on the microplate was dedicated for the positive control (isoproterenol) and the negative control (DMSO). The assay can be conducted as a "blind test", such that the assayerdoes know the identity of the compounds being tested and a second person prepares a "mother plate" containing each of the compounds at two different concentrations. The "blind" assayer takes 10ml from each well from the mother plate and transfers the samples to the assay plate containing the cells. 50ml of coelenterazine h (20mM) was then added to each well to start the bioluminescent reaction. Microplate was then loaded in the BRETCount for light measurement, as described above, at 25 °C. Data was collected 68 minutes after additionof coelenterazine. False positives may be identified by repeating the assay with the mother cell line CHW, transfected with the sensor but which do not express b2.

It should be apparent to one skilled in the art, that alternative embodiments falling within the scope of the invention and employing other drug screening protocols may be adapted to employ the BRET assay.

Figure 32 represents the results from a mini-screen assay ratio 550/470 nm vs compound position in the well. The first four wells of row 1 are the negative control (DMSO alone) (N). The four following wells are the positive control (P). Median line was calculated by the following formula: Ratio positive (obtained with control positive) minus ratio negative (obtained with the negative control) divided by 60 multiplied by 100. For this particular assay the median line was settled at 2.029. All ratio values from the other wells, higher than the median line, are considered as hits (1-5). The identify of all the reactive compounds that interacted with b2 receptor are: 1: forskolin 2 mM: 2: epinepiarine 20nM: 4 forskolin 2mM).

## EXAMPLE XIV: AN ALGORITHM FOR THE BRET ASSAY

An algorithm was developed to decrease mathematically basal level during BRET assay. Although there other algorithms that could also be used, one simple approach, is to remove the portion ofRluc emission in the EYFP peak. For example, this could be calculated using the simple formula:

$$\frac{(\text{Em550 - Em470 X correcting factor})}{\text{Em 470}} = \text{corrected ratio}$$

where Em550 is the emission value (CPS) obtained with the filter 550nm (filter for EYFP emission)

Ex470 is the emission value (CPS) obtained with the filter 470nm (filter for Rluc emission)

The correcting factor was experimentally calculated using Rluc construct transfected alone (without the EYFP construct). The correcting factor is the ratio (550/470nm) of the Rluc construct at a given time after addition of coelenterazine. In the following example, Figures 33 and 34 represent the non corrected and corrected data, respectively. The same raw data (light emissions using both filters)) were used in both figures. The only difference is the that in Figure 34 the ratio have been corrected using the above formula. In this example, the above algorithm do not change the range dynamic of the assay which still approximately 0.1 (the difference between maximal and minimal ratio) in both figures. But the SNR (signal to noise ratio) does change. SNR is the ratio between maximal and minimal BRET ratio values. In Figure 33, the SNR is 1.07 compared to 4.77 for Figure 34. Introduction of algorithm in calculating BRET ratios, couldbevery useful during HTS process since itdecreases background and increase SNR therefore ease result interpretation.

## EXAMPLE XV: VARIOUS PARAMETERS THAT AFFECT THE ASSAY (LUMICOUNT)

This example summarizes various experiment performed to analyze BRET in mammalian cells. The experiments were divided in two parts: 1) determination coelenterazine permeability; 2) BRET feasibility using the LumiCount.

Determination of Coelenterazine permeability

i) **Coelenterazine derivatives.** UsingtheplasmidpRluc-CMV (Promega) and the LumiCount, it was determined which of the coelenterazine derivatives gives the highest signal. Cells (HeLa) were transiently transfected inT-75 flask with pRluc-CMV orcontrol vector using LipofectAMINE. At 48 hours post-transfection, cells were harvested and seeded in 96-well white ViewPlate (in duplicate). Coelenterazine derivatives (1 mM) were then added in each well. Luminescence from transfected cells was measured using the LumiCount 4 minutes after addition of coelenterazine derivatives. LumiCount was set at 1100V PMT and 1 sec read length.

Table IV:

| Assay of coelenterazine derivatives | |
|---|---|
| Coelenterazine derivatives | Normalized RLU |
| native | 2030 |
| n | 35936 |
| f | 180126 |
| h | 173547 |
| hcp | 48144 |
| control vector (without Rluc gene) | 855* |

*This value is elevated because of cross-talk between wells. The electronic background is about 20-40 RLU. An evaluation of the cross-talk was determined in the LumiCount at 1.3% in the well next to the emitter and at 0.2% the second well next to the emitter. The cross-talk increased to 3% when filters (see below) were used.

ii **Cell linearity experiment.** Cells (HeLa and CHO) were transiently transfected in T-75 flasks with pRluc-CMV using LipofectAMINE. At 48 hours post-transfection, cells were harvested and seeded (in quadruplicate) in 96-well white ViewPlate atdifferent cell densities (ranging from 0 to 150,000 cells per well) . Coelenterazine h in PBS (1 mM) was then added in each well. Luminescence from transfected cells was measured using the LumiCount, 10 minutes after addition of coelenterazine. LumiCount was set at 1100V PMT and 0.1 sec read length.

Figure 35 presents the results observed. As is evidenced, Rluc luminescence is linear over the range of cell number analyzed. Very high signal to noise (SNR) ratios (over 200) could be obtained using moderate amount of cells (50,000 cells per well). Even higher RLU and SNR could be achieved if the analysis was performed during the Rluc flash of luminescence. The slopes for HeLa and CHO cells are different which presumably reflects the differences in transfection efficiencies between the two cell lines and/or differences in CMV promoter activity between the two cell lines.

iii) **Time-course experiment.** Cells (HeLa and CHO) were transiently transfected inT-75 flasks with pRluc-CMV using LipofectAMINE. At 48 hours post-transfection, cells were harvested and seeded in 96-well white ViewPlate at a density of 50 000 cells per wells (in quadruplicate). Coelenterazine f (1 mM) was then added in each well. Luminescence from transfected cells was measured using the LumiCount at different times after addition of coelenterazine. LumiCount was set at 1100V PMT and 0.1 sec read length.

Figure 36 shows the results of these Time-course studies of Rluc luminescence. In both cell lines, decay ofRluc luminescence is rapid after addition of coelenterazine f. This experiment was repeated with native coelenterazine and similar results were obtained. The half-life of the reaction is 11 minutes for CHO cells and 13 minutes for HeLa cells. These values are similar to what was found in literature for *in vitro* (cell extracts) experiments.

iv) **Dose-response of coelenterazine derivatives.** HeLa cells were transiently transfected in T-75 flasks with pRluc-CMV using LipofectAMINE. At 48 hours post-transfection, cells were harvested and seeded in 96-well white ViewPlate at a density of 25 000 cells per well (in quadruplicate). Coelenterazine f or h was then added in each well at different concentrations. Luminescence from transfected cells was measured using the LumiCount, 3 minutes after addition of coelenterazine. LumiCount was set at 1100V PMT and 0.1 sec read length.

Figure 37 shows the results of the experiments for a dose response of coelenterazine derivatives. Saturations were obtained for both coelenterazine derivatives. From these curves, the Km ofRluc for both coelenterazines were calculated. These are 1.2 and 0.8 mM for coelenterazine f and h respectively. Again, these values are similar to what was found in literature for *in vitro* (cell extracts) experiments.

v) **Dose response using lysed and whole cells.** HeLa cells were transiently transfected in T-75 flasks with pRluc-CMV using LipofectAMINE. At 48 hours post-transfection, cells were harvested, washed and divided in two. One fraction of the cells were directly seeded in 96-well white ViewPlate at a dens ity of 25 000 cells per wells (in quadruplicate). Cells in the other fraction were lysedusing a Dounce potter and lysing buffer (hypotonic solution). Afterpotting, unlysed cells and nuclei were removed by centrifugation. The lysed extract was distributed in the 96 well white plate. Coelenterazine h was then added in each well at differentconcentrations. Luminescence from transfected cells was measured using the LumiCount, 2-3 minutes after addition of coelenterazine. LumiCount was set at 900V PMT and 1 sec read length.

Figure 38 shows the results of a comparison of lysed and non-lysed cells. Both assays gave approximately the same Km (around 1mM) and similar absolute RLU meaning that coelenterazine cross rapidly and massively the cell membrane.

The following studies pertain to the BRET assay.

**i) Fusion type assay**

Using the above parameters for Rluc luminescence and knowing that cell membrane is permeable to coelenterazine, it was determined that one should start to assay BRET in mammalian cells. The Vanderbilt University constructs were subcloned into mammalian expressing vectors (pCDNA3.1). BRET was assayed using initially the fusion-type vector where the Rluc protein was fused to EYFP (third construct in annex 1). For that purpose, the LumiCount was modified by increas ing the length between the PMT and the microplate. This was done in order to put a filter on the microplate. To measure BRET, the luminescence is detected at two wavelengths: 480nm and 530nm (using two 50 X 50mm filters from Ealing). Hence, after addition of coelenterazine on cells, the 530nm filter was fixed on the microplate using Fun-Tak (from Lepage) and the luminescence from the microplate was read. Then the 530nm filter was replaced with the 480 filters and luminescence was again read. Ratios of emission at 530nm and 480nm were calculated in order to quantify BRET.

The cells (CHO) were transiently transfected in T-75 flask with pcDNA3.1-Rluc +pcDNA3.1-EYSP or pcDNA3.1-Rluc+pcDNA3.1-Rluc•EYSP (fusion) using LipofectAMINE (fig. 41). At48 hours post-transfection, cells were harvested and seeded in 96-well white ViewPlate at a density of 50.000 cells per well (in quadruplicate) in 50 ml of PBS. The modified LucLite buffer (100 ml) was then added before adding 50 ml of coelenterazine f (final concentration: 1 or 5 mM). Luminescence from transfected cells was measured using the LumiCount at different times after addition of coelenterazine. LumiCount was set at 1100V PMT and 1 sec read length. Table V shows a summary of thehesults from different transfections.

Table V:

| BRET assay using the LumiCount (* N>10 different transfections) | |
|---|---|
| Constructs | Ratio 530/480 |
| Control Rluc* | 0.6-07 |
| Control Rluc+EYFP (cotransfected)* | 0.7-1.0 |
| Fusion Rluc:EYFP* | 1.2-1.6 |
| Rluc-kaiB:EYFP-kaiB (positive) | 1.2-1.45 |
| Rluc-kaiB (negative control) | 0.8 |
| Rluc-kaiB+EYFP-kaiA (negative control) | 0.86-0.91 |

BRET canbe detected using the LumiCount. The ratios from the fusion Rluc::EYFP are always higher compare to both negative controls. Note that the ratios do not seem to be stable over time (see Figure 39 and 40) and the ratios from negative controls were higher than expected especially when using higher coelenterazine concentrations.

1) Ratio stability. BRET or ratios were stable if Rluc emission was stable. But results were variable if Rluc envssion was not stable. When using coelenterazine h, ratios from negative control increase with time and ratios from the fusion decreased to a point where both curves crossed each other (Figure 39). This point occurred latter in time when lower concentration of coelenterazine was used. When native coelenterazine was used, ratios were more stable (see figure 40).

2) High ratios for negative control. According to fluorescence scan in bacteria (Vanderbilt's results) the negativecontrol should have a ratio of 0.5-0.6. This is less than obtained in mammalian cells.

Figure 39 shows the results of studies of ratio stability when using coelenterazine h in LucLite. Both problems are related. They are dependent of coelenterazine (type and concentration) and probably of number of sensor molecules per cells. To verify new assays were performed using less DNA during the transfections (Figure 41 and 42) or by lysing the cells to decrease the entropy of the reaction: sensor molecules dilution in higher volume (Figure 43). The volumeof the cells is very small (high entropy). When too much of Rluc is emitting (because of its high expression or the use of the strong coelenterazine derivatives), nearby EYFP in the negative control is activated resulting in a increase of the ratio. Hence, light output at 530nm increase so the ratio. If there is high levels of Rluc or EYFP molecules and/or coelenterazine, activation of EYFP (nearby) from Rluc, emission is stronger. In fact, by comparing the negative control Rluc and the negative control Rluc+EYFP (see table 2), ratios from the latter were usually higher indicating that some non-specific BRET (radiative energy transferred) occurred. Figure 40 shows results of ratio stability when using native coelenterazine in LucLite.

Figure 41 shows BRET detection in cells transfected with 10 times less DNA. Figure 42 shows BRET detection in cells transfected with 20 times less DNA. By using less DNA during the transfections, the Rluc+EYFP negative control ratio was diminished to the level of Rluc alone, indicating that there is less non-specific BRET occurring in the Rluc+EYFP negative control. Furthermore the ratios were more stable over time when expressing lower amount of molecules. The difference between fusion and negative control were higherwhenusing less DNA hence increasing the dynamic range of the assay. Finally from a biological point of view, expressing lower amount of sensormolecules within the cells is better: it will cause less disturbance to the "normal" metabolism of the cell.

The best conditions in terms of the stability of ratio and non-specific BRET produced, where found when the cells are lysed (Figure 43). In these conditions, all the ratios are stable and all the negative controls were equivalent. Figure 43 shows the results of BRET detection when cells are lysed. Transfections were done using the "normal" amount of DNA.

**ii) Protein-protein type assay.**

Protein-protein assay using kai domains was also performed (see table 2). Only Rluc-kai B/EYFP-kaiB interaction resulted in higher ratio than Rluc-kai B/EYFP-kai A control.

**iii) Formatting of the assay.**

**Assay media.** It was determined whether the assay media had a effect on Rluc emission (Figure 44). The above experiments were repeated using PBS or DMEM/F12 (without phenol red) media and LucLite. For both the fusion and the negative control, PBS gave the highest Rluc emission. Figure 44 shows the results of the comparison between PBS and DMEM/F12 (without phenol red) for assaying BRET.

**Other conditions tested.** Different types of microplates and assay volumes were tested as to whether they had an effect on BRET. The volume used for the assay did not influence the ratio. The type of microplate (black or white) did influence the ratio: black microplate decreased the ratios compared to white plate. But this happened for both the negative control and the fusion.

Table VI:

| Microplate type and volume effect on BRET | |
|---|---|
| Construct/Conditions | Ratio 530/480 |
| Rluc+EYFP White microplate vol. 200ul | 0.87 |
| Rluc•EYPP | 1.31 |
| Rluc+EYFP Black microplate vol.200ul | 0.76 |
| Rluc•EYFP | 1.04 |
| Rluc+EYFP White microplate vol. 100ul | 0.90 |

Table VI: (continued)

| Microplate type and volume effect on BRET | |
|---|---|
| Construct/Conditions | Ratio 530/480 |
| Rluc•EYFP | 1.32 |
| 5 uM coelenterazine 50,000 cell per well. | |

The dynamic range (number of folds over negative control) is somewhat small being about two, which was not that different from literature. Usually for FRET assay, the dynamic range was between 2 and 3 times.

**EXAMPLE XVI: VARIOUS PARAMETERS THAT AFFECT THE ASSAY (BRETCOUNT)**

The following experiments pertain to factors affecting the stability of the assay. During the course of the BRET study, it was determined that Rluc emission in live cells was not stable over time having a half-life of about 5-8 min. Rluc emission stability important for BRET assay in order to achieve higher reproducibility (tighter CV, SD) and easier adaptation to HTS. Initially, the modified LucLite buffer was used to stabilize the Rluc emission with little success. This buffer which does not contain the Triton N-101 detergent, but still contains DTT, had some stabilization effect by doubling the Rluc emission half-life (12-16 min). But the use of this modified LucLite, is not recommended in live cell assay because long term exposure damaged the cells: solutions are not isotonic. Besideor in combination with the modified LucLite buffer, different assay conditions were tried in order to obtain the better stability. Among them, $Ca^{2+}/Mg^{2+}$ dependency, amount of DNA trans fected, cell concentration, coelenterazine concentration, type of media (PBS vs DMEM), time of incubation of the modified LucLite with the cells. Beside DTT, two observations came out from this previous study for the stabilization of Rluc: 1-coelenterazine concentration was the only conditions that had a affect (small though) on the half-life on the reaction. 2- true glow (half-life over 1 hour) was achieved only if the cells were lysed. The stability (half-life) of the Rluc even in lysed cells, is differentcompared to FFluc. FFluc half-life is more than 3 hours compared to 1 hour for the Rluc when they were assayed using the LumiCount and the DuoLite (DuoLite was later renamed FireLite). The main objective here is to find assay conditions that will allow stable Rluc emission and that thoseconditions will not interfere with the energy transfer of BRET. The followings are the conditions that were tested:

**3- Time-resolved emission analysis.** Rluc emission is divided into two phases. Within the first 20-30 minutes the emission decays rapidly without hitting the zero. After 30 minutes Rluc emission is more stable. It needed to be determined whether this "stable" state could be use for HTS: it is caused by the autoluminescenceof the coelenterazine and if not, is the heightof the curves are linear with the Rluc content of the cell.

**4- Temperature effects**. The effect of temperature on R luc emission and on BRET ratio is to be assayed.

**5- Analogue effect.** If available, the use of inactive coelenterazine analogues and/or coelenteramide (coelenteramide is a by-product of the reaction) would be tried for the stabilization of Rluc.

**6- Radical scavenger effect.** Finally, the effect of the cell permeable radical scavenger: DTT.

Two compounds found in the LucLite solutions are important for the stability of FFluc emission: DTT and AMP. DTT athigh concentration seems to act has an radical scavenger and functions by limiting the availability of $O_2$. At very high concentration, DTT even increases emission of photons through an unknown mechanism. $O_2$ is essential for the FFluc reaction as are ATP, Mg2+ and the FFluc substrate, luciferin. Figure 45 shows DTT effects on the Rluc emission stability.

FFluc oxidation reaction is divided in two steps. First, there is the formation of a "activated intermediate" when luciferin and ATP bind the enzyme and when the ATP is degraded into AMP which in turn forms a reactive anhydride. Adding AMP to the reaction slows the formation of the activated enzyme and so the turnover of the reaction making it more slower. In the second step, the activated intermediate reacts with $O_2$ and breaks down into $CO_2$, oxyluciferin and green-yellow light at 560nm. Others components of the LucLite kit are also important (to a lesser degree though) for the emission stability of FFluc: oxalic acid and phenylacetic acid as protease inhibitors, EDTA for the control of $Mg^{2+}$ concentration and Hepes buffer for a tight control of the pH (independent of the $CO_2$ content of the media). Promega (Madison, WI) also adds in the FFluc kit D-analogue substrate and CoA (see below).

The enzymatic mechanism of Rluc is different compared to FFluc. First, Rluc uses the coelenterazine as substrate and produces blue light at 470-480nm. It is not a two step process: there is no requirement for ATP and Mg2+. The turnover of the reaction is faster than for FFluc. Because it is not a two step process Rluc reaction suffers from autoluminescenceof its substrate. Therefore, Rluc reaction is less sensitive than FFluc reaction. The levels of the autoluminescence is greatly influenced by the availability of hydrophobic microenvironments to protect the luminescent excited state from the water. Hence, the presence of hydrophobic proteins, cellular debris (membrane), detergent micelles can diminish sensitivity. On the other hand, the coelenterazine is membrane permeable making Rluc attractive for cell based assays. Finally, it seems that DTT is also effective to stabilize Rluc acting probably as radical scavenger. Note that DTT is also membrane permeable at concentration higher than 1 mM.

From a structural point of view, both enzymes are very different. Rluc comes from a coelenterate and has a MW of 36 kDa. FFluc comes from the insect and has a MW of 61 kDa. There is no obvious amino acid sequence identity between them. The amino acid sequence of the FFluc is more related to a diverse family of acyl-CoA synthetase. That emphasized the importance of CoA presents in Promega's kit, to stabilize the FFluc. Hence, both enzymes are totally different both structurally and enzymaticaly (substrate, requirements for the reaction, etc.)

**Methods:**

Transfections: CHO-K1 cells were transiently transfected using LipofectAMINE (BRL/Gibco) in 100mm dishes according to manufacturer's protocol (0.2ug for each construct + X carrier DNA for a total of 8ug). Transfection efficiencies between 40-60% were obtained using this technique (when analyzed using a GFP reporter vector and fluorescence microscope). 48 hours post transfection, cells were harvested using low concentrated trypsin, counts and diluted in D-PBS (containing Ca/Mg). For most experiment, 50,000 cells per well were used in white Optiplate. Assay were performed in quadruplicate. The modified LucLite buffer was used initially to stabilize Rluc emission. Light emission was triggered by the addition of coelenterazine h 5 uM (50ul of a 20 uM stock).

The experiments were conducted using the BRETCount at various time after addition of the coelenterazine. Parameters used for the BRETCount were: luminescence SPC mode, read length 1 sec for each wavelengths at 25°C, filters: 470DF60 550DF80. In some experiments, results were obtained with the LumiCount. Parameters for the LumiCount were: read length of 0.5sec PMT 1100V.

**1- Time resolved Rluc emission.**

Cells were transfected with the Rluc constructonly. Two days after transfection, cells were harvested and seeded at different concentration in the modified LucLite buffer. Coelenterazine h was added before the detection in the BRETCount. Figure 46 shows Rluc emission stability over time.

Figure 46 shows that starting at 10 minutes after addition of coelenterazine, Rluc emission tend to stabilizeover time. Furthermore, even after 10 minutes, it was noted that RFU levels still dependent of cell number. In order to be sure, was repotted the graph by using RFU vs cell number at different fixed times (see Figure 47).

Figure 47 shows Rluc emission levels versus cell number at fixed time. Figure 47 shows that Rluc emissions were linear with cell number even at 58 minutes post induction (t=58). This is a surprise since at this time, the enzyme is not anymore in the optimal conditions to work properly (i.e. substrate concentration is limited, high amount of by-product present in the reaction). Furthermore, the slopes at t=30 and t=58 were similar meaning that Rluc emission half-life does not change substantially during this time period. The experiment was repeated us ing different relative amount of transfected cells but with the same total amount of cells. The transfected cells were mixed with untransfected cells to achieve a total cell number of 100,000 cells per well. The rational is that coelenterazine autoluminescence is cell dependent (in fact lipid dependent). Hence increased cell numbers per well will contribute to the linearity seen in Figure 47. To verify this assumption, the experiment was repeated using the same total number of cells per well. Figure 48 shows the Rluc lightemissions using different amount of transfected cells mixed with untransfected cells to make a total cell number of 100,000 cells. These mixed populations were then seeded into the microplate just before starting the reaction with the coelenterazine.

Figure 48 shows R luc emission stability in presence of a fixed number of cells. Figure 49 shows Rluc emission levels vs cell number at fixed time.

Figures 47 and 49 show that Rluc emission levels are linear with cell number even at 40 minutes post-coelenterazine addition. Therefore, the Rluc light outputs observed at 30 minutes and beyond, do not result from autoluminescence of the coelenterazine but from the true Rluc enzymatic activities and these latter are linearly dependent of Rluc content of the cells.

**2- Temperature effects.** Using the LumiCount, it was determined that at lower temperature, Rluc emission was reduced and stabilized over time. These results are summarized in Figure 50 and 51. Figure 50 shows that at 15°C the Rluc emission is stabilized over the period of time analyzed. The BRET ratio (Figure 51) appears to change with assay temperature: it decreases when temperature is decreased. At 10°C, the Rluc emission is too much reduced to be workable using the LumiCount (data not shown).

Figure 50 shows the effect of the temperature on the Rluc emission. The Lumi Count and the reagents needed for the assay were stored in the walk-in refrigerator 2-3 hours before starting the experiment and the internal LumiCount temperature was settled either at 15 or 10 °C. Figure 51 shows the effect of the temperature on the BRET ratio (LumiCount).

Further characterization of the effect of temperature using the BRETCount in which the internal temperature is more stable and controllable compared to the LumiCount (Figure 52). The experiments were repeated using the BRETCount at different temperature. The highest SNRs were obtained at the assay temperature of 19°C. The ratio tends to increase when temperature increases which confirms the results obtained with the LumiCount. Above 19°C, even the ratios of the negative controls (Rluc+EYFP) increase with temperature. Figure 52 shows the Effect of the temperature on the BRET ratio (BRETCount).

**3- Radical scavenger effect**. The BRETCount was used (which has a higher through-put compared to the LumiCount) to analyze the effect of radical scavenger, more particularly the DTT, on both the Rluc emission and the BRET ratio. It has been previously shown that the DTT helps to stabilize the Rluc emission (see Figure 53).

Figure 53 shows the effect of the DTT on the Rluc emission. Harvested cells were diluted into PBS (+$Ca2^+$/$Mg2^+$) containing different concentration of DTT. Figure 53 shows the effect of the DTT (in PBS $Ca2^+$/$Mg2^+$) on the Rluc emission in livecell. Increasedconcentration of DTT stabilizes the Rluc emission by lowering the initial emission flash of the enzyme. The highest stability was obtained at 100mM of DTT. At this concentration, the DTT decreases the viability of the cells. Viability analysis was performed using the trypan blue dye at different time after the cells were diluted in the buffer containing the DTT. 10mM DTT was chosen which gi ves some (not that high though) Rluc stability and good long term viability (below 15% loss over 2 hours).

Figure 54 shows the effect of the DTT on the Rluc emission and the BRET ratio (BRETCount). These results were obtained with the stable cell lines expressing Rluc only (negative control). PBS=PBS+$Ca^{2+}$/$Mg^{2+}$+glucose. DTT 10mM. Figure 55 shows the effectof the DTTon the Rluc emission and the BRET ratio (BRETCount). These results were obtained with the stable cell lines expressing the fusion Rluc::EYFP (positive control). PBS=PBS+$Ca^{2+}$/$Mg^{2+}$+glucose DTT 10mM.

Figures 54 and 55 show that DTT effect the BRET ratio. In PBS ($Ca^{2+}$/$Mg^{2+}$+glucose) alone, BRET ratios are lower than in presence of DTT. When DTT is added, ratios increase by almost 0.1 BRET unit. The ratio of the negative control also increased. Hence, the DTT do not affect (or has a small effect) the energy transfer.

It was determined that the assay temperature has a effect on the BRET ratio. This observation is important in the context that most biological processes which will be assayed using BRET, are performed at 37°C.

To a certain point, DTT helped to stabilize the Rluc emission. Good stabilization occurs at the emission at 100mM DTT which at this concentration cells were damaged making this concentration are not suitable forcell-based assay. At 10mM, it is noted that DTT has also a small effect on the BRET ratio and not only on the yield and duration of Rluc emission. In fact it appears that DTT changes the spectral characteristic of the Rluc emission to a small degree. The emission would be right shifted on the emission scan.

Time resolved emission analysis revealed that 30 minutes after addition of coelentetazine, the Rluc emission entered a relatively stable phase. This phase lasted for more than 60 min (data not shown).

## Claims

**1.** A bioluminescence resonance energy transfer (BRET) system comprising: 1) a bioluminescent protein that has luciferase activity; 2) an acceptor fluorophore that can accept the energy from the bioluminescent protein when they are associated, in the presence of the appropriate substrate; 3) a modulator that influences the proximity or the orientation of the bioluminescent protein and the fluorophore in response to interaction with another molecule and 4) an appropriate substrate to activate the luciferase activity of the bioluminescent protein.

**2.** A system as in claim 1, wherein the luciferase and the fluorophore are attached to the same modulator.

**3.** A system as in claim 1, wherein the luciferase and the fluorophore are attached to separate modulator entities.

**4.** The system as in claim 1, wherein conformational change in the modulator causes a decrease in the proximity of a luciferase and a fluorophore.

**5.** The system as in claim 1, wherein conformational change in the modulator causes an increase in the proximity of a luciferase and a fluorophore.

6. The system as in claim 1, wherein conformational change in the modulator causes a change in the orientation of a luciferase and a fluorophore.

7. A system as in claim 2, in which the system is a fusion protein construct comprising a luciferase attached to modulator which is attached to a fluorophore.

8. A system as in claim 3, which comprises:

(a) a fusion protein construct comprising a luciferase attached to modulator entity that is a protein or protein domain; and
(b) a fusion protein construct comprising a fluorophore attached to a modulator entity that is a protein or protein domain; and wherein
(c) the protein or protein domain in (a) is capable of interacting with the protein or protein domain in (b).

9. A system as in claim 3, wherein the luciferase is attached to a known protein, a fluorophore is attached to the products of a gene library, and an association between a known protein and a product of the gene library is detected by measuring resonance energy transfer between the luciferase and the fluorophore.

10. A fusion protein construct as in claim 7, wherein the fluorophore is a green fluorescent protein that is red-shifted in its excitation and/or emission spectra compared to wild type green fluorescent protein.

11. A fusion protein construct as in claim 7, wherein a target sequence is inserted into the amino acid linker sequence.

12. A fusion protein construct as in claim 11, wherein the target sequence is selected from the list comprising: a protease site, a phosphorylation sit, a $Ca^{+2}$ binding site, a cAMP binding site, an IP3 binding site, and a cGMP binding site.

13. A fusion protein construct as in claim 12, wherein the fluorophore is a green fluorescent protein that is red-shifted in its excitation and/or emission spectra compared to wild type green fluorescent protein.

14. A fusion protein construct as in claim 8 or 13, wherein the fluorophore is a green fluorescent protein selected from the list comprising EYFP variants, EGFP, GFP wild type, GFPS65T, YFP, Topaz.

15. A recombinant DNA encoding the fusion protein construct of claim 7 or claim 11.

16. A fusion gene that comprises the recombinant DNA of claim 15.

17. A DNA cassette comprising a promoter operably linked to one or more fusion protein genes of claim 16.

18. A vector comprising a fusion gene as in claim 17.

19. A host cell transformed or transfected by the vector of claim 18.

20. A host cell as in claim 19, wherein the cell is human, mammalian, bacterial or yeast.

21. A vector as in claim 18, wherein the gene construct is under the control of a constitutive promoter.

22. A vector as in claim 21, wherein the constitutive promoter is selected from the list comprising the following promoters and their variants and derivatives: CMV, SV40, RSV, EF-1α, Tk, AdML, when the cell to be transformed or transfected is mammalian.

23. A vector as in claim 21, wherein the constitutive promoter is selected from the list comprising the following promoters and their variants and derivatives: T7, AraBAD, trc, pL, tac and lac, when the cell to be transformed or transfected is a bacterial cell.

24. A vector as in claim 22, wherein the fluorophore is a green fluorescent protein that is red-shifted in its excitation and/or emission spectra compared to wild type green fluorescent protein.

25. A vector as in claim 21,whrein the constitutive promoter is selected from the list comprising the following promoters and their variants and derivatives: nmt1, gal1, gal10, TEF1, AOX1, GAP and ADH1, when the cell to be transformed or transfected is a yeast cell.

**26.** A vector as in claim 24, wherein the fluorophore is a green fluorescent protein selected from the list comprising EYFP variants, EGFP, GFP wild type. GFPS65T, YFP, Topaz.

**27.** A virus comprising a fusion gene as in claim 17.

**28.** A host cell infected by the viral vector as in claim 27.

**29.** A virus as in claim 27 selected from the list comprising: retrovirus, adenovirus, Semliki and Sinbis and their variants and derivatives.

**30.** A host cell as in claim 28, wherein said host cell is an insect cell.

**31.** A vector as in claim 21, wherein the constitutive promoter is bacwovirus, when the cell to be transformed or transfected is an insect cell.

**32.** A vector as in claim 18, wherein the vector is under the control of an inducible promoter.

**33.** A vector as in claim 21, wherein the inducible promoter is selected from the list comprising GRE, Tet-off, Tet-on, HSP, MMTV based promoter, and metallothionein.

**34.** A recombinant DNA encoding one of the fusion protein constructs of claim 24.

**35.** A fusion gene encoding the recombinant DNA of claim 34.

**36.** A vector comprising a fusion gene as in claim 35.

**37.** A host cell transformed or transfected by a vector of claim 36.

**38.** A host cell as in claim 37, wherein the cell is human, mammalian, bacterial, or yeast.

**39.** The use of the system of claim 1 for second messenger screening.

**40.** The use of the system as in claim 39, wherein the second messenger is selected from the list comprising: cAMP, cGMP, $Ca^{2+}$, IP3, NO, DAG, ceramide and derivatives thereof, and arachidonic acid and derivatives thereof.

**41.** The use of the system as in claim 1 for analyte screening.

**42.** The use of the system as in claim 41, wherein the analyte is selected from the list comprising $K^+$, $H^+$, $Ca^{2+}$, $CO_2$, $Na^+$, $Cl^-$, $Mg^{2+}$, $O_2$, $HCO_3$, NO and ATP.

**43.** The use of the system of claim 1 for drug discovery.

**44.** The use of the system of claim 1 for drug screening.

**45.** The use of the system as in claim 1 to detect changes in protein-protein interaction.

**46.** The use of the system as in claim 1 in functional genomics to determine the cellular function of a gene by determining its binding partner.

**47.** The use of the system as in claim 1 in toxicology to measure the presence and concentration of toxic compounds.

**48.** The use of the system in claim 1 in genotoxicity to measure the effect of a toxic compound on genome stability.

**49.** The use of the system in claim 1 for *in vitro* assays.

## Patentansprüche

1. Biolumineszentes Resonanz-Energie-Transfersystem (BRET), das aufweist: 1) ein biolumineszentes Protein, das Luciferaseaktivität aufweist; 2) ein Akzeptor-Fluorophor, das die Energie von dem biolumineszenten Protein annehmen kann, wenn sie in Anwesenheit des geeigneten Substrats zusammengebracht werden; 3) einen Modulator, der die Nähe oder die Ausrichtung des biolumineszenten Proteins und des Fluorophors in Reaktion auf Interaktion mit einem anderen Molekül beeinflusst; und 4) ein geeignetes Substrat zum Aktivieren der Luciferaseaktivität des biolumineszenten Proteins.

2. System nach Anspruch 1, wobei die Luciferase und das Fluorophor an denselben Modulator angeheftet sind.

3. System nach Anspruch 1, wobei die Luciferase und das Fluorophor an separate Modulatoreinheiten angeheftet sind.

4. System nach Anspruch 1, wobei Konformationsänderung am Modulator eine Abnahme der Nähe einer Luciferase und eines Fluorophors verursacht.

5. System nach Anspruch 1, wobei Konformationsänderung am Modulator eine Zunahme der Nähe einer Luciferase und eines Fluorophors verursacht.

6. System nach Anspruch 1, wobei Konformationsänderung am Modulator eine Änderung bei der Ausrichtung einer Luciferase und eines Fluorophors verursacht.

7. System nach Anspruch 2, wobei das System ein Fusionsproteinkonstrukt ist, das eine Luciferase aufweist, die an den Modulator angeheftet ist, der an ein Fluorophor angeheftet ist.

8. System nach Anspruch 3, das aufweist:

   (a) ein Fusionsproteinkonstrukt, das eine Luciferase enthält, die an eine Modulatoreinheit, die ein Protein oder eine Proteindomäne ist, angeheftet ist; und

   (b) ein Fusionsproteinkonstrukt, das ein Fluorophor enthält, das an eine Modulatoreinheit, die ein Protein oder eine Proteindomäne ist, angeheftet ist; und wobei

   (c) das Protein oder die Proteindomäne in (a) zur Interaktion mit dem Protein oder der Proteindomäne in (b) fähig ist.

9. System nach Anspruch 3, wobei die Luciferase an ein bekanntes Protein angeheftet ist, ein Fluorophor an die Produkte einer Genbibliothek angeheftet ist und eine Assoziation zwischen einem bekannten Protein und einem Produkt der Genbibliothek durch Messen des Resonanzenergietransfers zwischen der Luciferase und dem Fluorophor ermittelt wird.

10. Fusionsproteinkonstrukt nach Anspruch 7, wobei das Fluorophor ein grün fluoreszierendes Protein ist, das in seiner Erregung und/oder seinen Emissionsspektren im Vergleich zu grün fluoreszierendem Protein des Wildtyps rotverschoben ist.

11. Fusionsproteinkonstrukt nach Anspruch 7, wobei eine Zielsequenz in die Aminosäure-Linkersequenz eingefügt ist.

12. Fusionsproteinkonstrukt nach Anspruch 11, wobei die Zielsequenz aus der Liste gewählt ist, die eine Proteasestelle, eine Phosphorylierungsstelle, eine $Ca^{+2}$-Bindungsstelle, eine cAMP-Bindungsstelle, einen IP3-Bindungsstelle und eine cGMP-Bindungsstelle aufweist.

13. Fusionsproteinkonstrukt nach Anspruch 12, wobei das Fluorophor ein grün fluoreszierendes Protein ist, das in seiner Anregung und/oder seinen Emissionsspektren im Vergleich zu grün fluoreszierendem Protein des Wildtyps rotverschoben ist.

14. Fusionsproteinkonstrukt nach Anspruch 8 oder 13, wobei das Fluorophor ein grün fluoreszierendes Protein ist, das aus der Liste gewählt wird, die EYFP-Varianten, EGFP, Wildtyp-GFP, GFPS65T, YFP, Topaz enthält.

**15.** Rekombinante DNA, die das Fusionsproteinkonstrukt nach Anspruch 7 oder Anspruch 11 codiert.

**16.** Fusionsgen, das die rekombinante DNA nach Anspruch 15 aufweist.

**17.** DNA-Kassette, die einen Promotor aufweist, der mit einem oder mehr Fusionsproteingenen nach Anspruch 16 wirkungsmäßig gekoppelt ist.

**18.** Vektor, der ein Fusionsgen nach Anspruch 17 aufweist.

**19.** Wirtszelle, die durch den Vektor nach Anspruch 18 transformiert oder transfiziert ist.

**20.** Wirtszelle nach Anspruch 19, wobei die Zelle eine Human-, Säugetier-, Bakterien- oder Hefezelle ist.

**21.** Vektor nach Anspruch 18, wobei sich das Genkonstrukt unter der Kontrolle eines konstitutiven Promotors befindet.

**22.** Vektor nach Anspruch 21, wobei der konstitutive Promotor aus der Liste gewählt ist, die die folgenden Promotoren und deren Varianten und Derivate aufweist: CMV, SV40, RSV, EF-1a, Tk, AdML, wenn die zu transformierende oder zu transfizierende Zelle eine Säugetierzelle ist.

**23.** Vektor nach Anspruch 21, wobei der konstitutive Promotor aus der Liste gewählt ist, die die folgenden Promotoren und deren Varianten und Derivate aufweist: T7, AraBAD, trc, pL, tac und lac, wenn die zu transformierende oder zu transfizierende Zelle eine Bakterienzelle ist.

**24.** Vektor nach Anspruch 22, wobei das Fluorophor ein grün fluoreszierendes Protein ist, das in seiner Erregung und/oder seinen Emissionsspektren im Vergleich zu grün fluoreszierendem Protein des Wildtyps rotverschoben ist.

**25.** Vektor nach Anspruch 21, wobei der konstitutive Promotor aus der Liste gewählt ist, die die folgenden Promotoren und deren Varianten und Derivate aufweist: nmt1, gal1, gal10, TEF1, AOX1, GAP und ADH1, wenn die zu transformierende oder zu transfizierende Zelle eine Hefezelle ist.

**26.** Vektor nach Anspruch 24, wobei das Fluorophor ein grün fluoreszierendes Protein ist, das aus der Liste gewählt wird, die EYFP-Varianten, EGFP, Wildtyp-GFP, GFPS65T, YFP, Topaz enthält.

**27.** Virus, der ein Fusionsgen nach Anspruch 17 aufweist.

**28.** Wirtszelle, die durch den viralen Vektor nach Anspruch 27 infiziert ist.

**29.** Virus nach Anspruch 27, der aus der Liste gewählt ist, die Retrovirus, Adenovirus, Semliki und Sindbis und deren Varianten und Derivate enthält.

**30.** Wirtszelle nach Anspruch 28, wobei die Wirtszelle eine Insektenzelle ist.

**31.** Vektor nach Anspruch 21, wobei der konstitutive Promotor Baculovirus ist, wenn die zu transformierende oder zu transfizierende Zelle eine Insektenzelle ist.

**32.** Vektor nach Anspruch 18, wobei sich der Vektor unter der Kontrolle eines induzierbaren Promotors befindet.

**33.** Vektor nach Anspruch 21, wobei der induzierbare Promotor aus der Liste gewählt wird, die GRE, Tet-off, Tet-on, HSF, MMTV-basierten Promotor und Metallothionein enthält.

**34.** Rekombinante DNA, die eines der Fusionsproteinkonstrukte nach Anspruch 24 codiert.

**35.** Fusionsgen, das die rekombinante DNA nach Anspruch 34 codiert.

**36.** Vektor, der ein Fusionsgen nach Anspruch 35 aufweist.

**37.** Wirtszelle, die durch einen Vektor nach Anspruch 35 transformiert oder transfiziert ist.

**38.** Wirtszelle nach Anspruch 37, wobei die Zelle eine Human-, Säugetier-, Bakterien- oder Hefezelle ist.

**39.** Verwendung des Systems nach Anspruch 1 zum Second-Messenger-Screening.

**40.** Verwendung des Systems nach Anspruch 1, wobei der Second Messenger aus der Liste gewählt ist, die cAMP, cGMP, $Ca^{2+}$, IP3, NO, DAG, Ceramid und Derivate davon und Arachidonsäure und Derivate davon enthält.

**41.** Verwendung des Systems nach Anspruch 1 zum Analyt-Screening.

**42.** Verwendung des Systems nach Anspruch 41, wobei der Analyt aus der Liste gewählt ist, die $K^+$, $H^+$, $Ca^{2+}$, $CO_2$, $Na^+$, $Cl^-$, $Mg^{2+}$, $O_2$, $HCO_2$, NO und ATP umfasst.

**43.** Verwendung des Systems nach Anspruch 41 zur Wirkstoffentdeckung.

**44.** Verwendung des Systems nach Anspruch 41 zum Wirkstoffscreening.

**45.** Verwendung des Systems nach Anspruch 1 zum Nachweisen von Änderungen bei Protein-Protein-Interaktion.

**46.** Verwendung des Systems nach Anspruch 1 in der funktionellen Genomforschung zum Bestimmen der zellulären Funktion eines Gens durch Bestimmen seines Bindungspartners.

**47.** Verwendung des Systems nach Anspruch 1 in der Toxikologie zum Messen der Anwesenheit und Konzentration toxischer Verbindungen.

**48.** Verwendung des Systems nach Anspruch 1 bei Gentoxizität zum Messen der Wirkung einer toxischen Verbindung auf die Genomstabilität.

**49.** Verwendung des Systems nach Anspruch 1 für In-vitro-Assays.

## Revendications

**1.** Système de transfert d'énergie de résonance par bioluminescence (BRET) comprenant : 1) une protéine bioluminescente qui présente une activité de luciférase; 2) un fluorophore accepteur qui peut accepter l'énergie de la protéine bioluminescente lorsqu'ils sont associés, en présence du substrat approprié; 3) un modulateur qui influence la proximité ou l'orientation de la protéine bioluminescente et du fluorophore en réponse à une interaction avec une autre molécule et 4) un substrat approprié pour activer l'activité de luciférase de la protéine bioluminescente.

**2.** Système suivant la revendication 1, dans lequel la luciférase et le fluorophore sont attachés au même modulateur.

**3.** Système suivant la revendication 1, dans lequel la luciférase et le fluorophore sont attachés à des entités distinctes de modulateur.

**4.** Système suivant la revendication 1, dans lequel le changement de conformation du modulateur provoque une diminution de la proximité d'une luciférase et d'un fluorophore.

**5.** Système suivant la revendication 1, dans lequel le changement de conformation du modulateur provoque une augmentation de la proximité d'une luciférase et d'un fluorophore.

**6.** Système suivant la revendication 1, dans lequel le changement de conformation du modulateur provoque une modification de l'orientation d'une luciférase et d'un fluorophore.

**7.** Système suivant la revendication 2, dans lequel le système est une construction protéine de fusion comprenant une luciférase attachée à un modulateur qui est attaché à un fluorophore.

**8.** Système suivant la revendication 3, qui comprend :

(a) une construction protéine de fusion comprenant une luciférase attachée à une entité de modulateur qui est une protéine ou un domaine protéique; et
(b) une construction protéine de fusion comprenant un fluorophore attaché à une entité de modulateur qui est une protéine ou un domaine protéique; et où
(c) la protéine ou le domaine protéique de (a) peut interagir avec la protéine ou le domaine protéique de (b).

**9.** Système suivant la revendication 3, dans lequel la luciférase est attachée à une protéine connue, un fluorophore est attaché aux produits d'une banque génétique, et une association entre une protéine connue et un produit de la banque génétique est détectée en mesurant le transfert d'énergie de résonance entre la luciférase et le fluorophore.

**10.** Construction protéine de fusion suivant la revendication 7, dans laquelle le fluorophore est une protéine vert fluorescent qui est déplacée vers le rouge dans son spectre d'excitation et/ou d'émission en comparaison d'une protéine vert fluorescent de type sauvage.

**11.** Construction protéine de fusion suivant la revendication 7, dans laquelle une séquence cible est insérée dans la séquence d'acides aminés du lieur.

**12.** Construction protéine de fusion suivant la revendication 11, dans laquelle la séquence cible est sélectionnée à partir de la liste comprenant : un site de protéase, un site de phosphorylation, un site de liaison de $Ca^{2+}$, un site de liaison d'AMPc, un site de liaison d'IP3, et un site de liaison de GMPc.

**13.** Construction protéine de fusion suivant la revendication 12, dans laquelle le fluorophore est une protéine vert fluorescent qui est déplacée vers le rouge dans son spectre d'excitation et/ou d'émission en comparaison d'une protéine vert fluorescent de type sauvage.

**14.** Construction protéine de fusion suivant la revendication 8 ou 13, dans laquelle le fluorophore est une protéine vert fluorescent sélectionnée à partir de la liste comprenant des variantes de l'EYFP, l'EGFP, la GFP de type sauvage, la GFPS65T, l'YFP, la Topaz.

**15.** ADN recombinant codant pour la construction protéine de fusion suivant la revendication 7 ou la revendication 11.

**16.** Gène de fusion qui comprend l'ADN recombinant suivant la revendication 15.

**17.** Cassette d'ADN comprenant un promoteur lié de manière fonctionnelle à un ou plusieurs gènes de protéine de fusion suivant la revendication 16.

**18.** Vecteur comprenant un gène de fusion suivant la revendication 17.

**19.** Cellule hôte transformée ou transfectée par le vecteur suivant la revendication 18.

**20.** Cellule hôte suivant la revendication 19, dans laquelle la cellule est humaine, mammalienne, bactérienne ou de levure.

**21.** Vecteur suivant la revendication 18, dans lequel la construction génétique est sous le contrôle d'un promoteur constitutif.

**22.** Vecteur suivant la revendication 21, dans lequel le promoteur constitutif est sélectionné à partir de la liste comprenant les promoteurs suivants et leurs variantes et dérivés : CMV, SV40, RSV, EF-1$\alpha$, Tk, AdML, lorsque la cellule à transformer ou à transfecter est mammalienne.

**23.** Vecteur suivant la revendication 21, dans lequel le promoteur constitutif est sélectionné à partir de la liste comprenant les promoteurs suivants et leurs variantes et dérivés : T7, AraBAD, trc, pL, tac et lac, lorsque la cellule à transformer ou à transfecter est une cellule bactérienne.

**24.** Vecteur suivant la revendication 22, dans lequel le fluorophore est une protéine vert fluorescent qui est déplacée vers le rouge dans son spectre d'excitation et/ou d'émission en comparaison d'une protéine vert fluorescent de type sauvage.

**25.** Vecteur suivant la revendication 21, dans lequel le promoteur constitutif est sélectionné à partir de la liste comprenant les promoteurs suivants et leurs variantes et dérivés : nmt1, gal1, gal10, TEF1, AOX1, GAP et ADH1, lorsque la cellule à transformer ou à transfecter est une cellule de levure.

**26.** Vecteur suivant la revendication 24, dans lequel le fluorophore est une protéine vert fluorescent sélectionnée à partir de la liste comprenant des variantes de l'EYFP, l'EGFP, la GFP de type sauvage, la GFPS65T, l'YFP, la Topaz.

**27.** Virus comprenant un gène de fusion suivant la revendication 17.

**28.** Cellule hôte infectée par le vecteur viral suivant la revendication 27.

**29.** Virus suivant la revendication 27, sélectionné à partir de la liste comprenant : un rétrovirus, un adénovirus, un Semliki, un Sinbis et leurs variantes et dérivés.

**30.** Cellule hôte suivant la revendication 28, dans laquelle ladite cellule hôte est une cellule d'insecte.

**31.** Vecteur suivant la revendication 21, dans lequel le promoteur constitutif est d'un baculovirus, lorsque la cellule à transformer ou à transfecter est une cellule d'insecte.

**32.** Vecteur suivant la revendication 18, dans lequel le vecteur est sous le contrôle d'un promoteur inductible.

**33.** Vecteur suivant la revendication 21, dans lequel le promoteur inductible est sélectionné à partir de la liste comprenant GRE, Tet-off, Tet-on, HSP, le promoteur basé sur le MMTV et la métallothionéine.

**34.** ADN recombinant codant pour une des constructions protéines de fusion suivant la revendication 24.

**35.** Gène de fusion codant pour l'ADN recombinant suivant la revendication 34.

**36.** Vecteur comprenant un gène de fusion suivant la revendication 35.

**37.** Cellule hôte transformée ou transfectée par un vecteur suivant la revendication 36.

**38.** Cellule hôte suivant la revendication 37, dans laquelle la cellule est humaine, mammalienne, bactérienne ou de levure.

**39.** Utilisation du système suivant la revendication 1, pour un criblage de second messager.

**40.** Utilisation du système suivant la revendication 39, dans lequel le second messager est sélectionné à partir de la liste comprenant : l'AMPc, la GMPc, le $Ca^{2+}$, l'IP3, le NO, le DAG, le céramide et des dérivés de ceux-ci et l'acide arachidonique et des dérivés de ceux-ci.

**41.** Utilisation du système suivant la revendication 1, pour un criblage d'analyte.

**42.** Utilisation du système suivant la revendication 41, dans lequel l'analyte est sélectionné à partir de la liste comprenant $K^+$, $H^+$, $Ca^{2+}$, $CO_2$, $Na^+$, $Cl^-$, $Mg^{2+}$, $O_2$, $HCO_3$, NO et l'ATP.

**43.** Utilisation du système suivant la revendication 1, pour une découverte de médicament.

**44.** Utilisation du système suivant la revendication 1 pour un criblage de médicament.

**45.** Utilisation du système suivant la revendication 1, pour détecter des modifications d'une interaction protéine-protéine.

**46.** Utilisation du système suivant la revendication 1, en génomique fonctionnelle pour déterminer la fonction cellulaire d'un gène en déterminant son partenaire de liaison.

**47.** Utilisation du système suivant la revendication 1, en toxicologie pour mesurer la présence et la concentration de composés toxiques.

**48.** Utilisation du système suivant la revendication 1, en génotoxicité pour mesurer l'effet d'un composé toxique sur la stabilité du génome.

**49.** Utilisation du système suivant la revendication 1 pour des analyses *in vitro*.

FIGURE 1

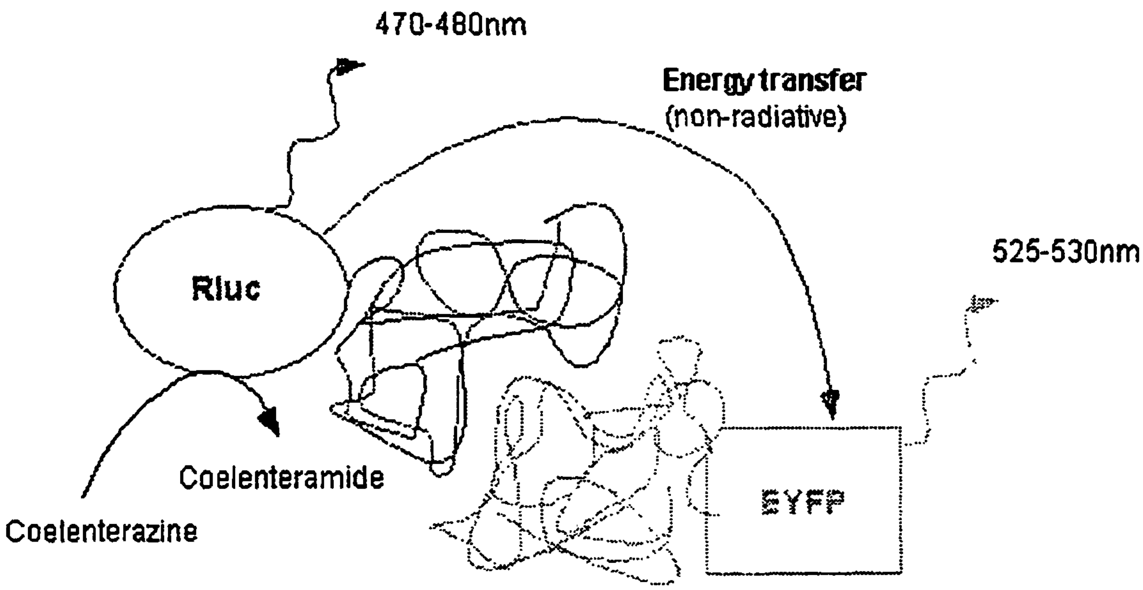
470-480nm
Energy transfer
(non-radiative)
525-530nm
Rluc
Coelenteramide
Coelenterazine
EYFP

FIGURE 2

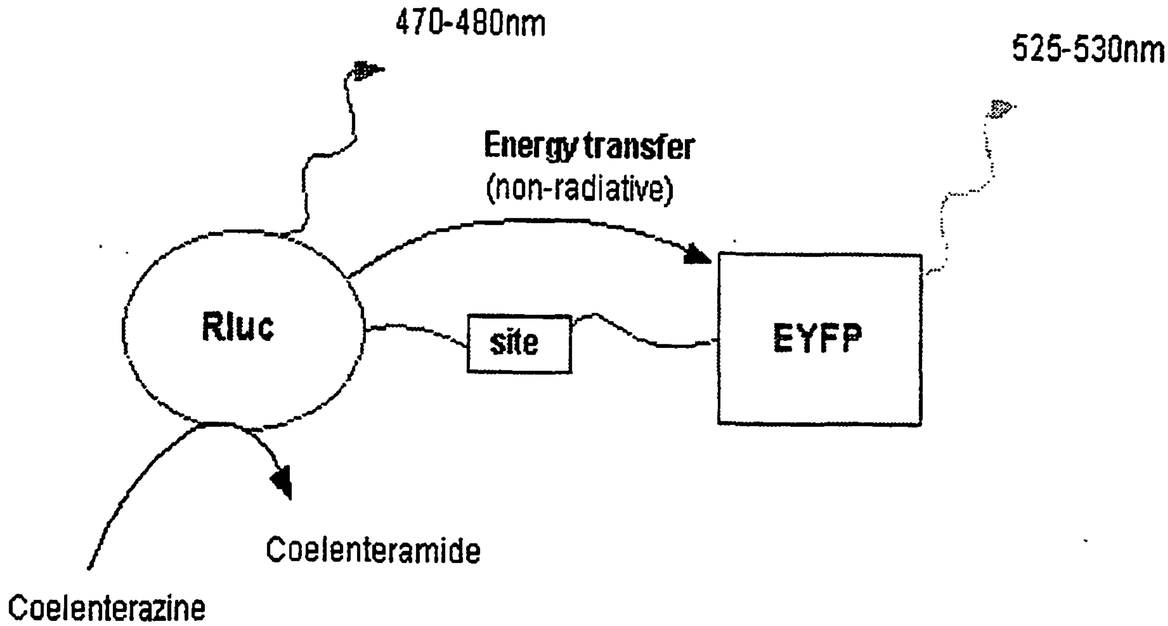
470-480nm
525-530nm
Energy transfer
(non-radiative)
Rluc
site
EYFP
Coelenteramide
Coelenterazine

FIGURE 3

(a)

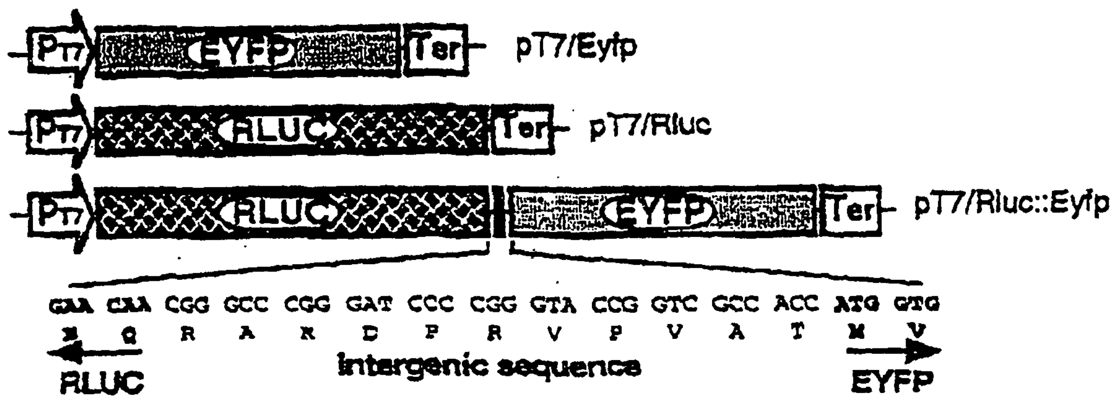
PT7
EYFP
Ter
pT7/Eyfp
PT7
RLUC
Ter
pT7/Rluc
PT7
RLUC
EYFP
Ter
pT7/Rluc::Eyfp
GAA CAA CGG GCC CGG GAT CCC CGG GTA CCG GTC GCC ACC ATG GTG
E Q R A R D P R V P V A T M V
RLUC
intergenic sequence
EYFP

(b)

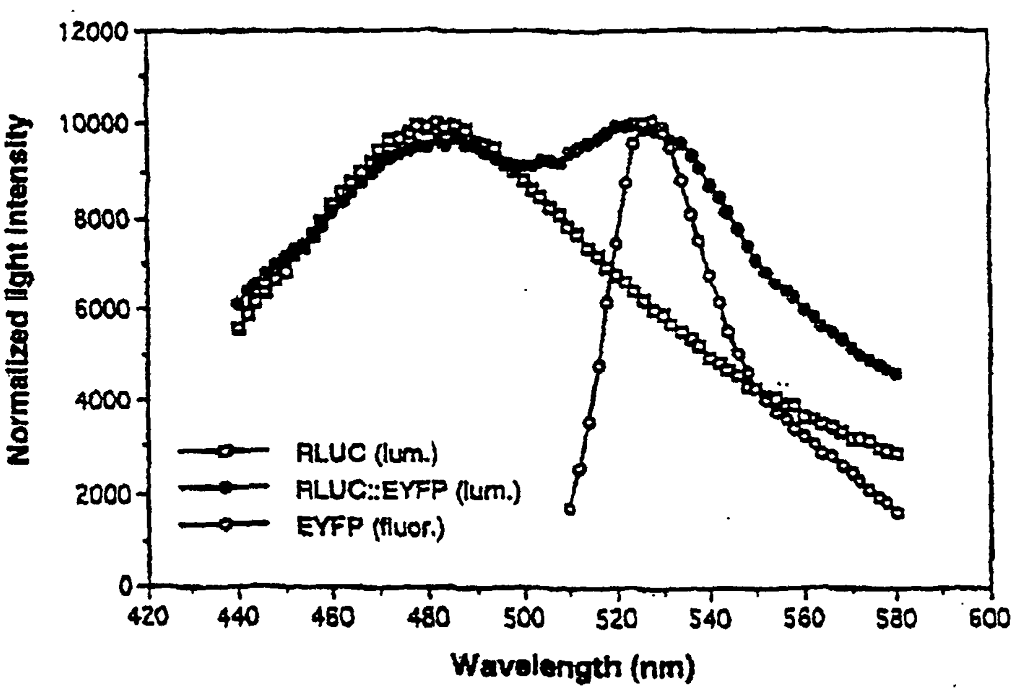
Normalized light intensity
12000
10000
8000
6000
4000
2000
0
420
440
460
480
500
520
540
560
580
600
Wavelength (nm)
RLUC (lum.)
RLUC::EYFP (lum.)
EYFP (fluor.)

FIGURE 4

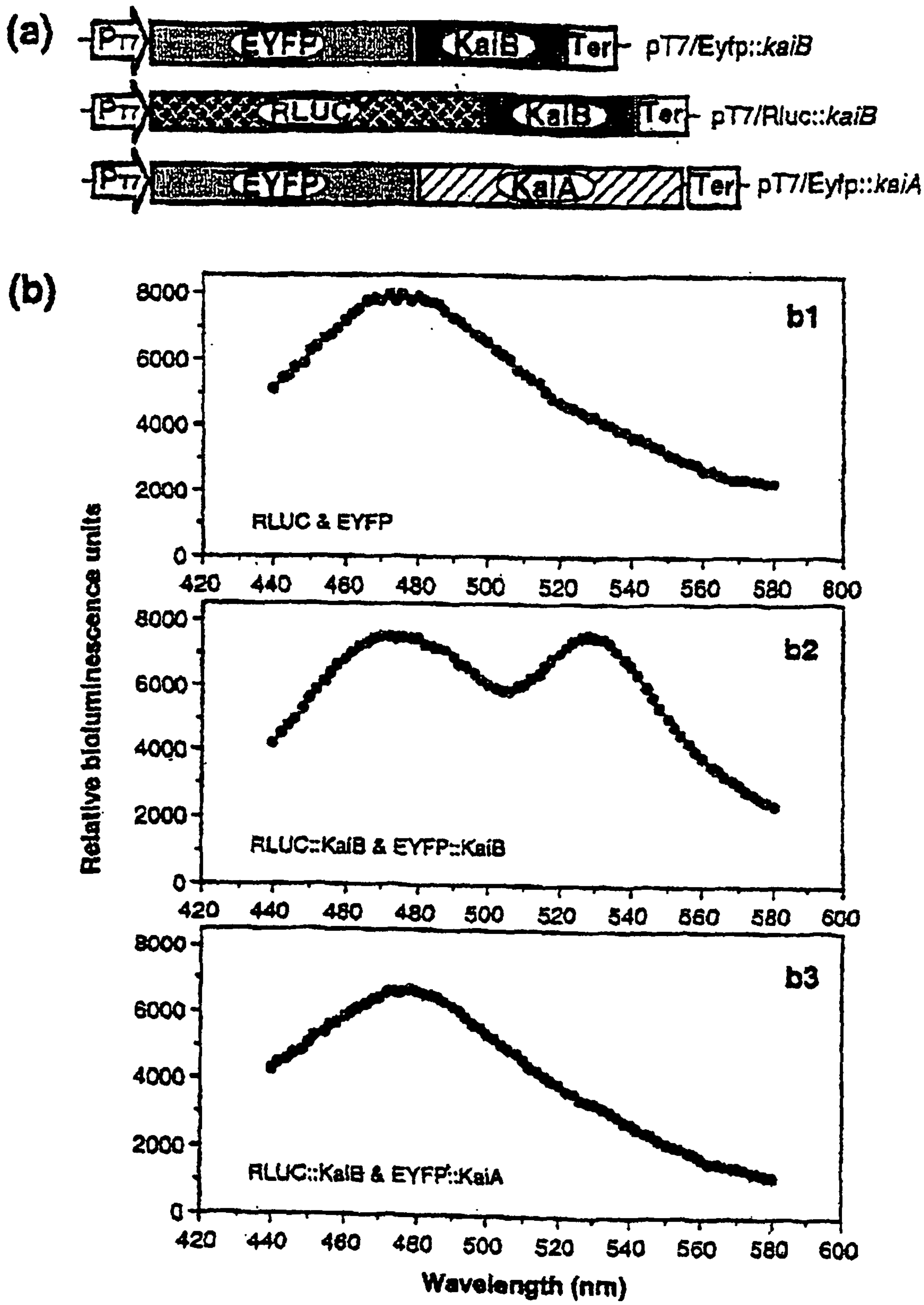
(a)
PT7
EYFP
KaiB
Ter
pT7/Eyfp::kaiB
PT7
RLUC
KaiB
Ter
pT7/Rluc::kaiB
PT7
EYFP
KaiA
Ter
pT7/Eyfp::kaiA
(b)
Relative bioluminescence units
b1
RLUC & EYFP
b2
RLUC::KaiB & EYFP::KaiB
b3
RLUC::KaiB & EYFP::KaiA
0
2000
4000
6000
8000
420
440
460
480
500
520
540
560
580
600
Wavelength (nm)

FIGURE 5

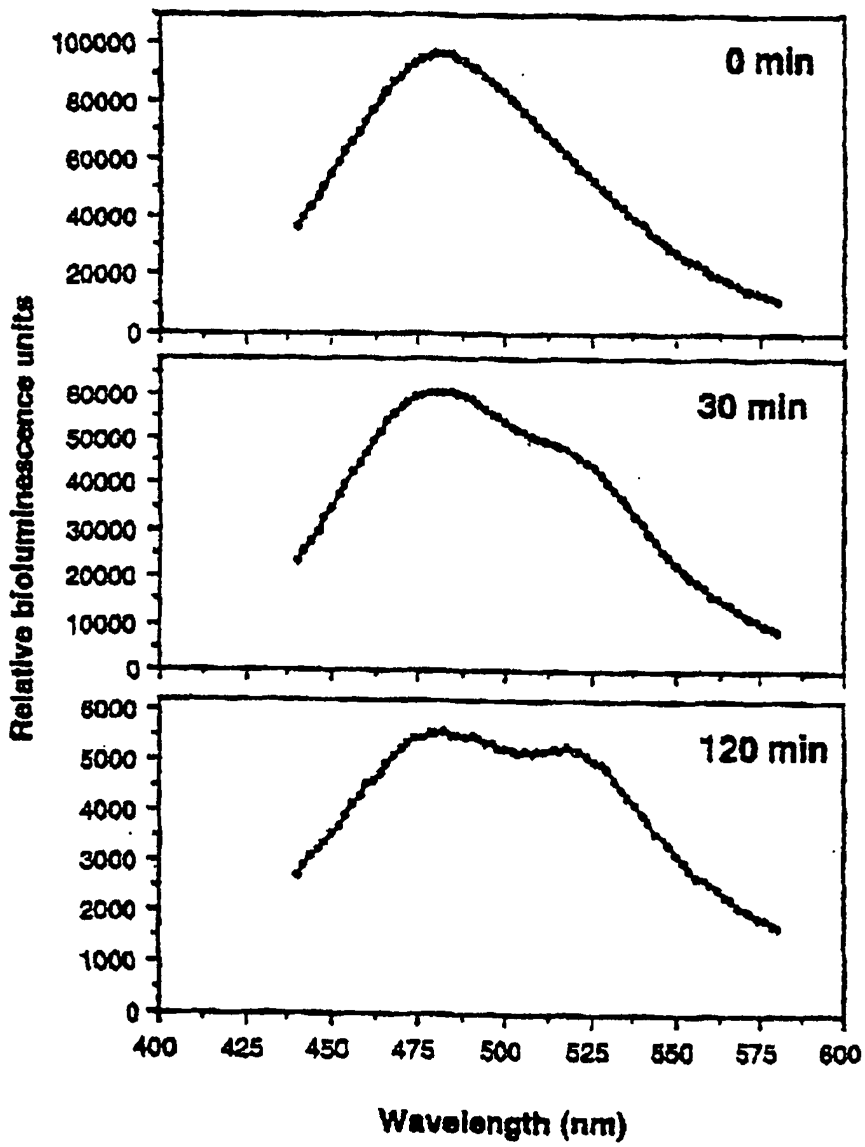

Relative bioluminescence units
100000
80000
60000
40000
20000
0
0 min
60000
50000
40000
30000
20000
10000
0
30 min
6000
5000
4000
3000
2000
1000
0
120 min
400
425
450
475
500
525
550
575
600
Wavelength (nm)

FIGURE 6

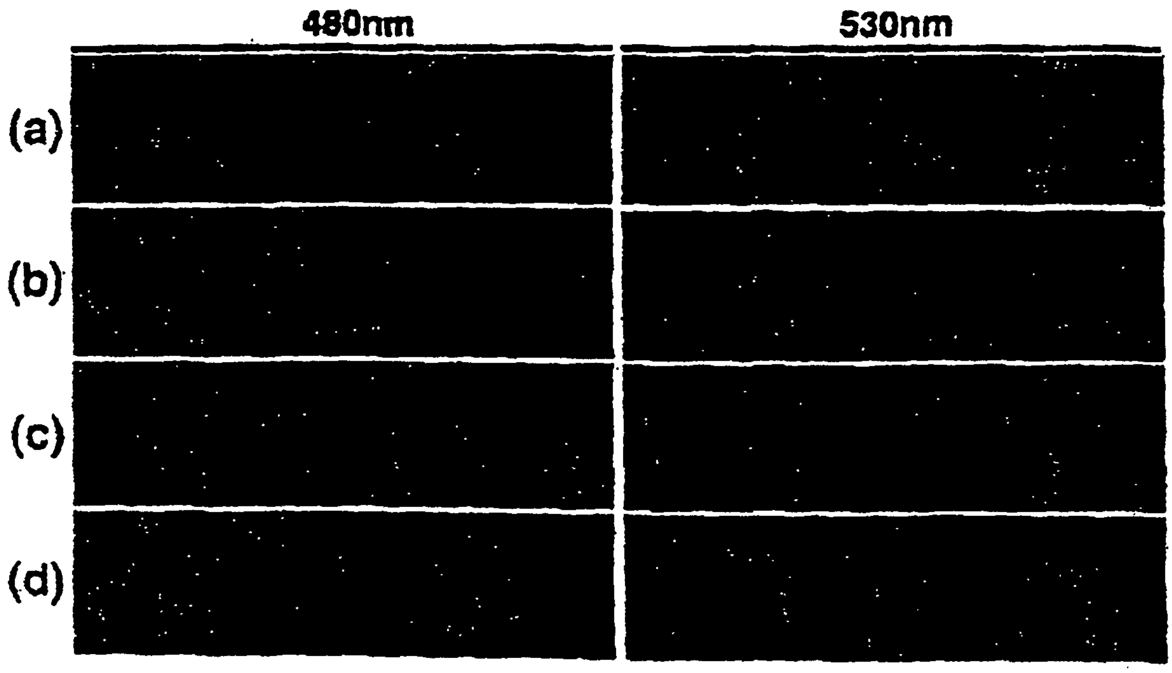

FIGURE 7

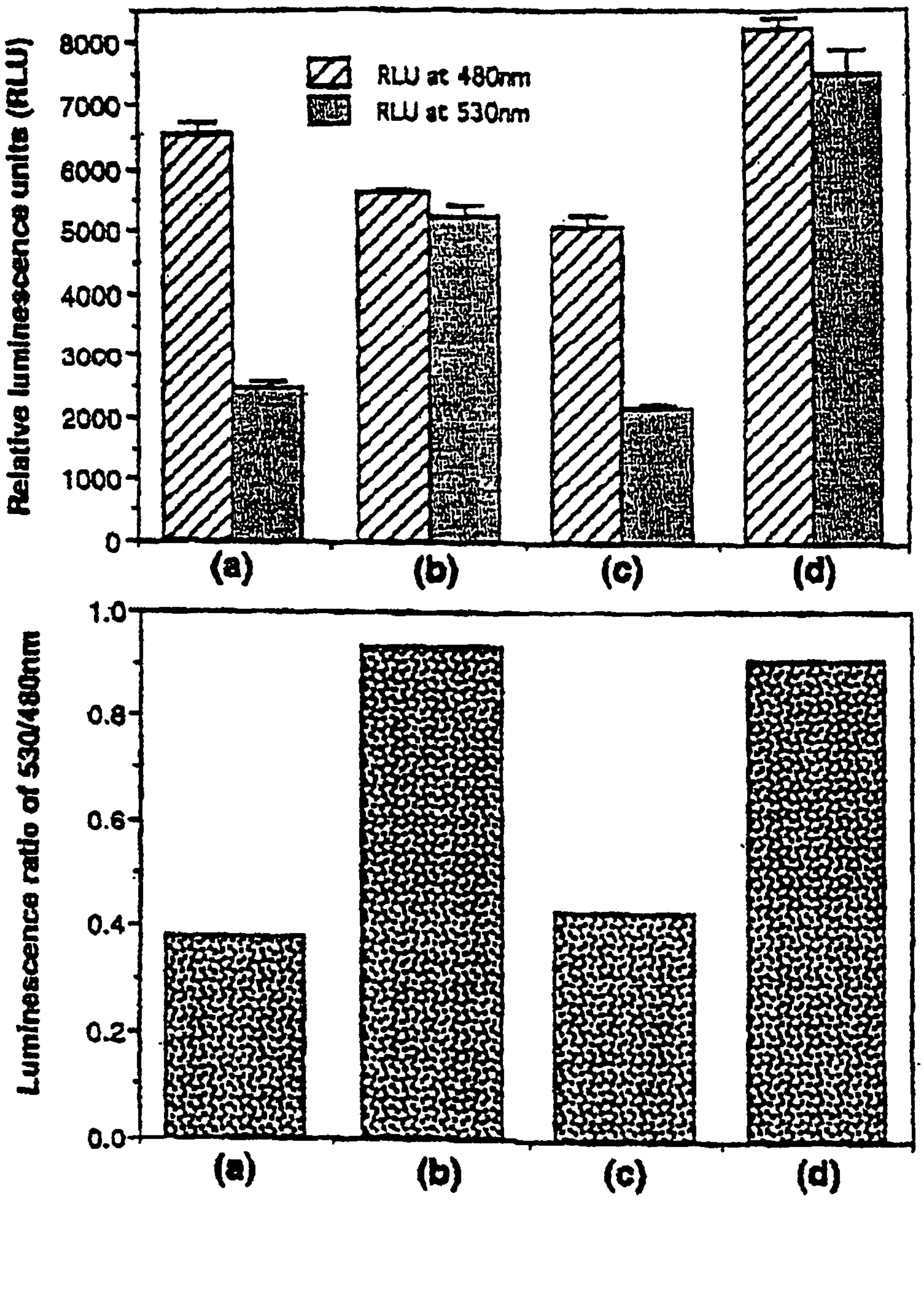
Relative luminescence units (RLU)
0
1000
2000
3000
4000
5000
6000
7000
8000
RLU at 480nm
RLU at 530nm
(a)
(b)
(c)
(d)
Luminescence ratio of 530/480nm
0.0
0.2
0.4
0.6
0.8
1.0
(a)
(b)
(c)
(d)

FIGURE 8

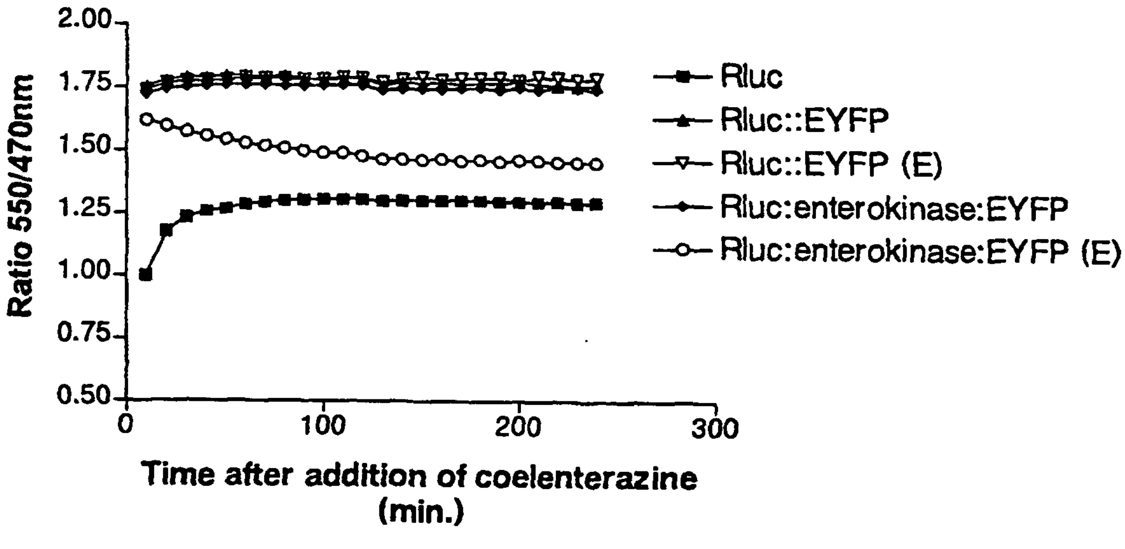
2.00
1.75
1.50
1.25
1.00
0.75
0.50
Ratio 550/470nm
0
100
200
300
Time after addition of coelenterazine (min.)
Rluc
Rluc::EYFP
Rluc::EYFP (E)
Rluc:enterokinase:EYFP
Rluc:enterokinase:EYFP (E)

FIGURE 9

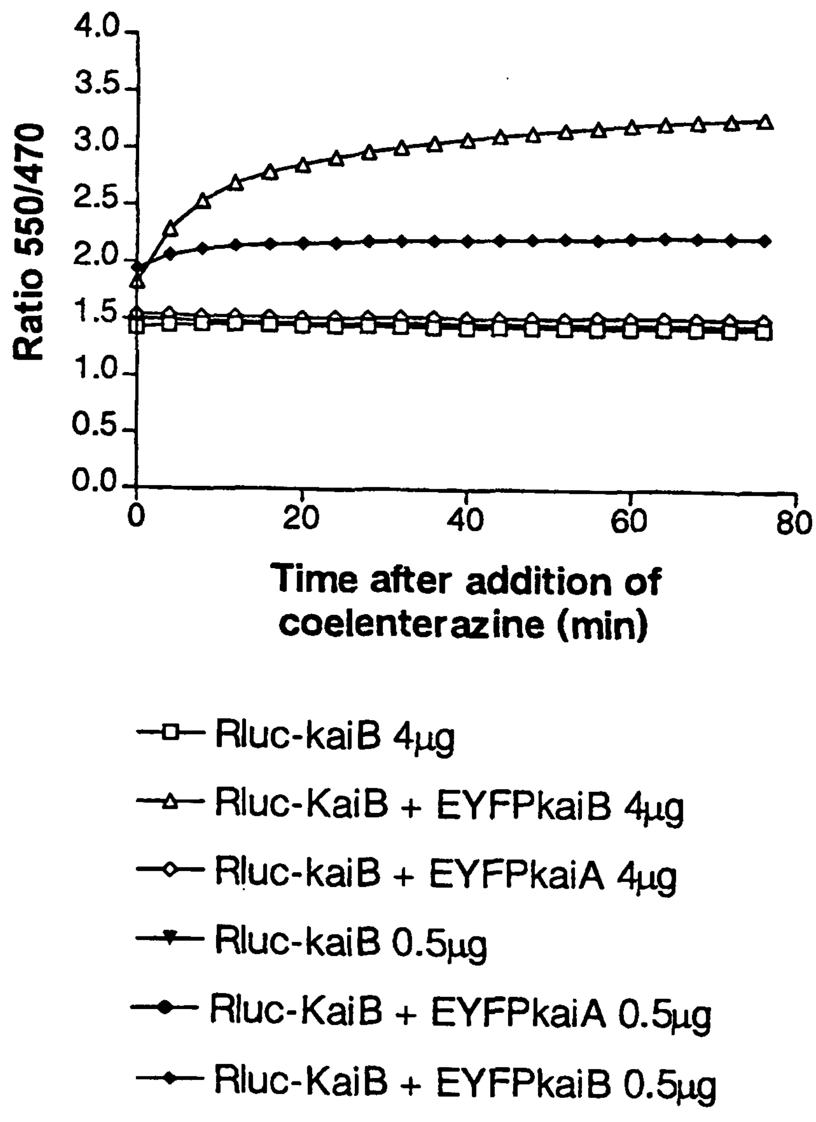

Ratio 550/470
4.0
3.5
3.0
2.5
2.0
1.5
1.0
0.5
0.0
0
20
40
60
80
Time after addition of coelenterazine (min)
Rluc-kaiB 4μg
Rluc-KaiB + EYFPkaiB 4μg
Rluc-kaiB + EYFPkaiA 4μg
Rluc-kaiB 0.5μg
Rluc-KaiB + EYFPkaiA 0.5μg
Rluc-KaiB + EYFPkaiB 0.5μg

FIGURE 10

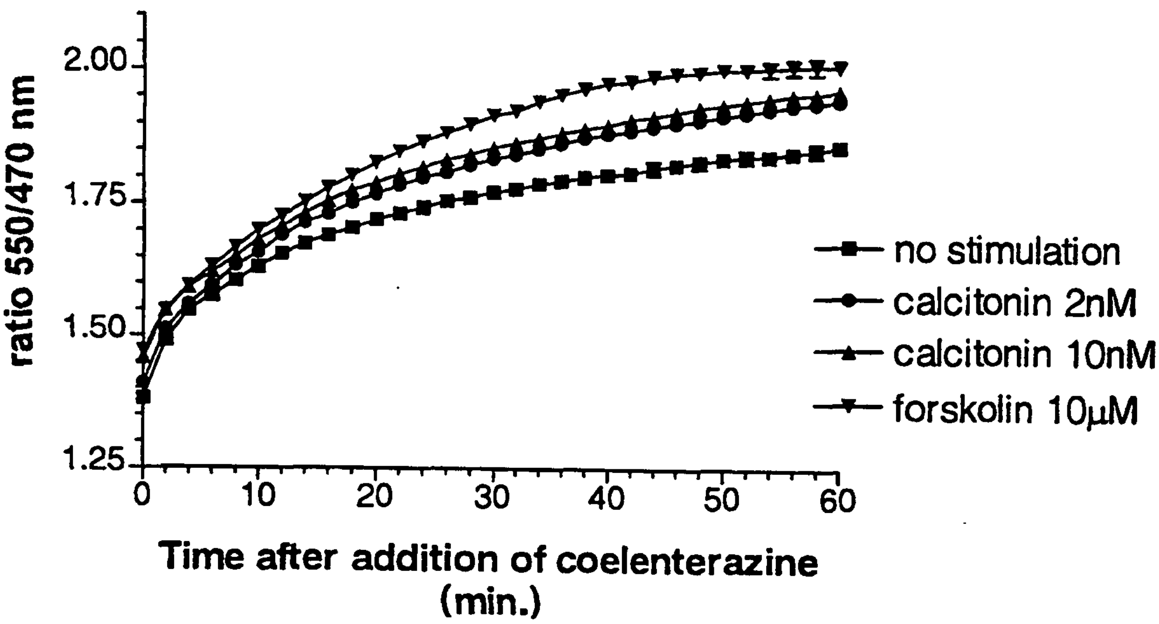
ratio 550/470 nm
2.00
1.75
1.50
1.25
0
10
20
30
40
50
60
Time after addition of coelenterazine (min.)
no stimulation
calcitonin 2nM
calcitonin 10nM
forskolin 10μM

FIGURE 11

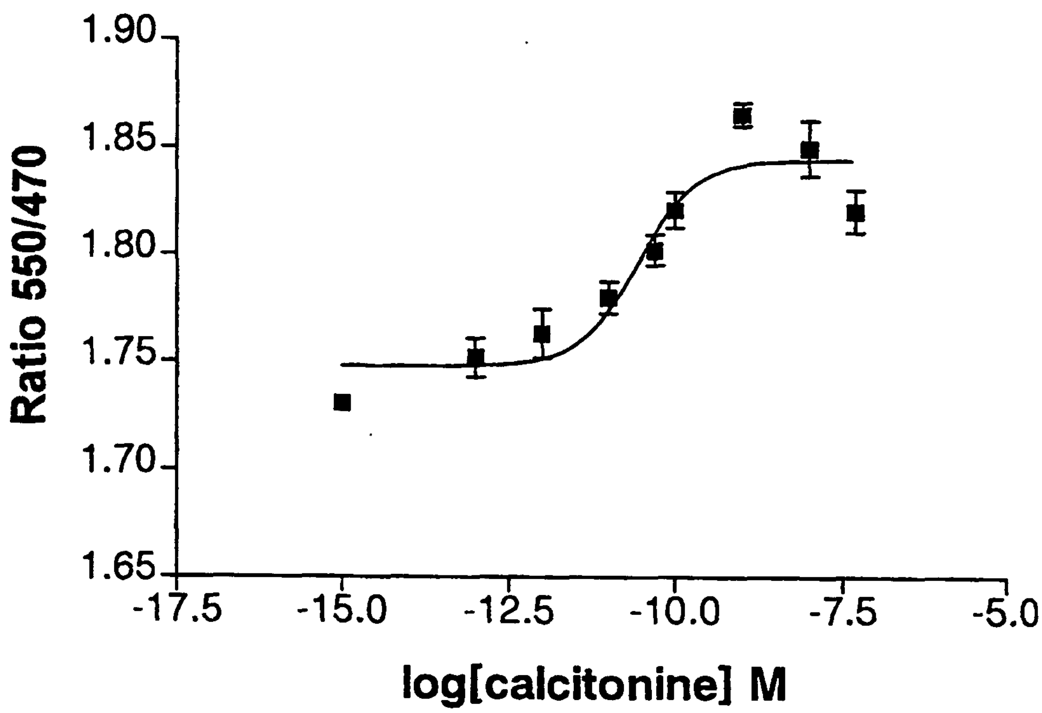
1.90
1.85
1.80
1.75
1.70
1.65
Ratio 550/470
-17.5
-15.0
-12.5
-10.0
-7.5
-5.0
log[calcitonine] M

FIGURE 12

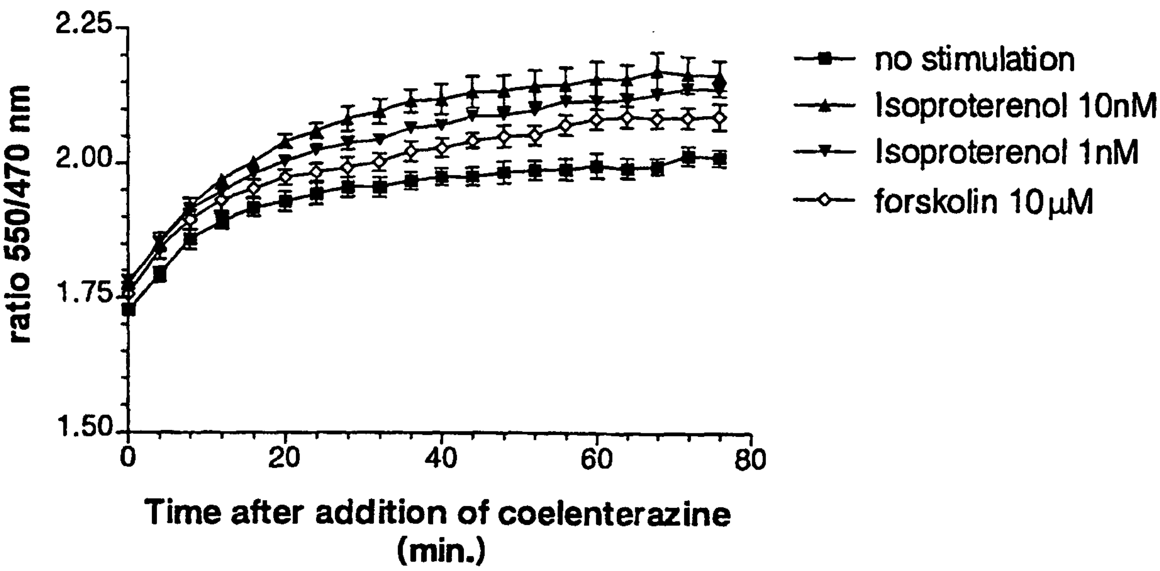
2.25
2.00
1.75
1.50
ratio 550/470 nm
0
20
40
60
80
Time after addition of coelenterazine (min.)
no stimulation
Isoproterenol 10nM
Isoproterenol 1nM
forskolin 10µM

FIGURE 13

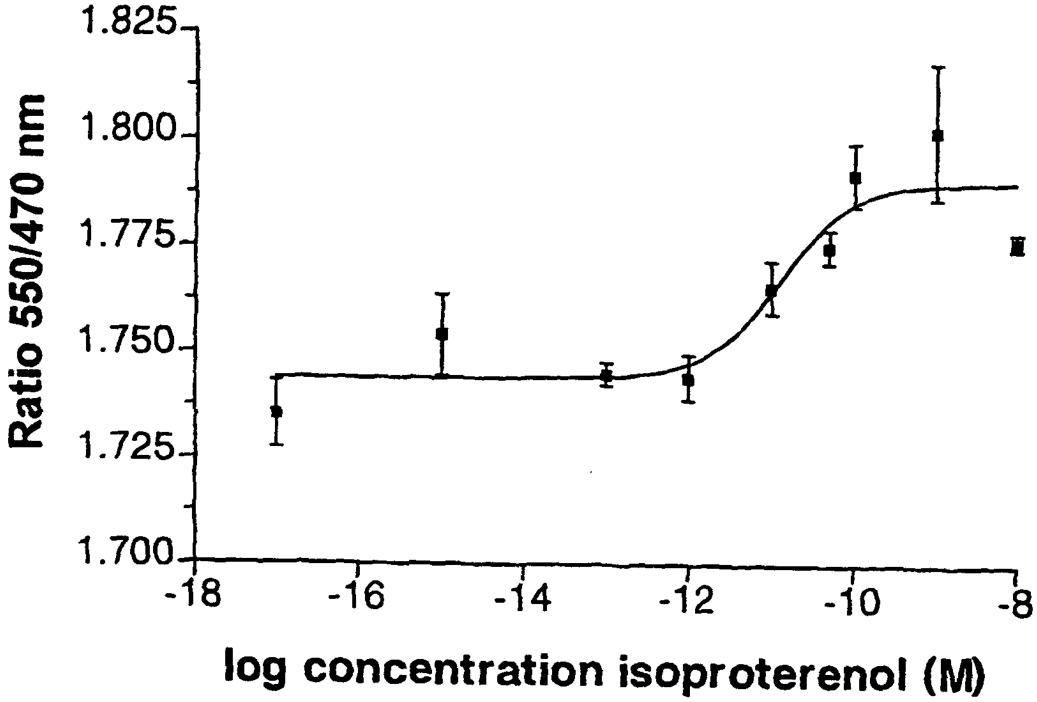
1.825
1.800
1.775
1.750
1.725
1.700
Ratio 550/470 nm
-18
-16
-14
-12
-10
-8
log concentration isoproterenol (M)

FIGURE 14

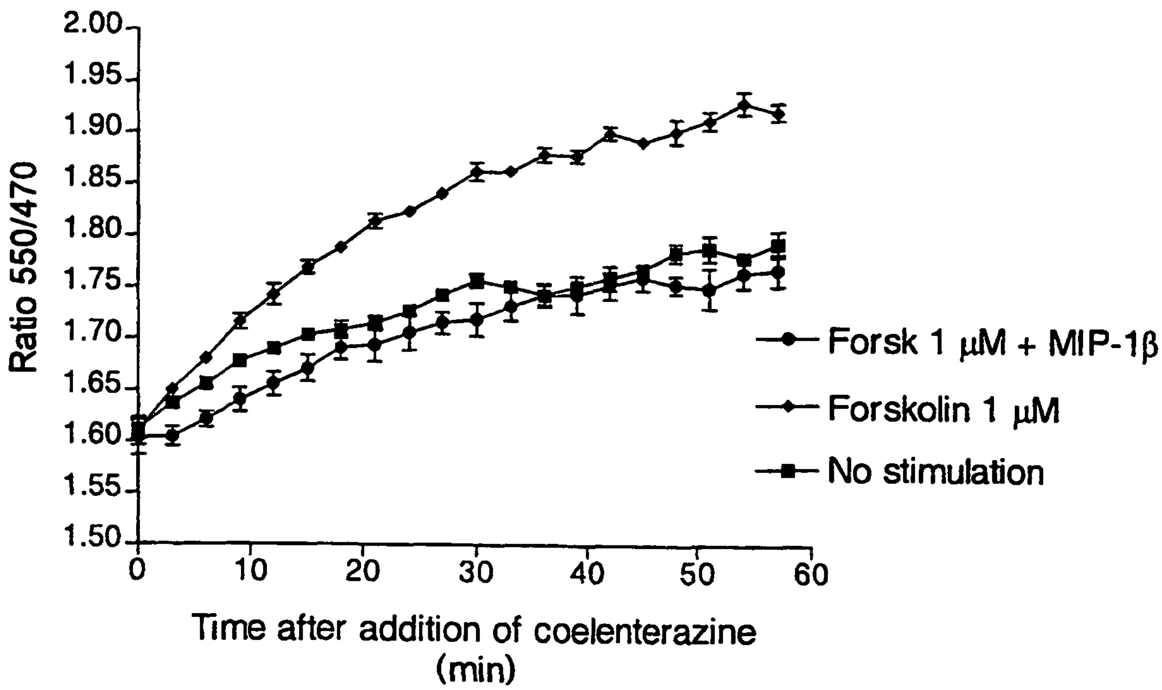
2.00
1.95
1.90
1.85
1.80
1.75
1.70
1.65
1.60
1.55
1.50
Ratio 550/470
0
10
20
30
40
50
60
Time after addition of coelenterazine (min)
Forsk 1 μM + MIP-1β
Forskolin 1 μM
No stimulation

FIGURE 15

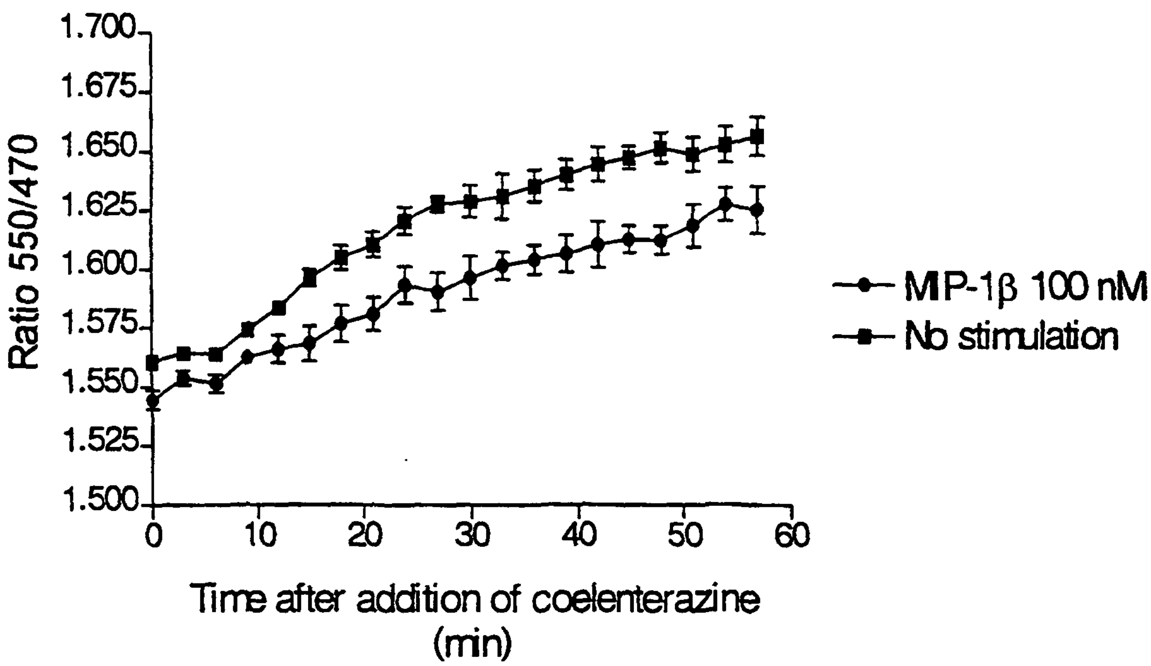
1.700
1.675
1.650
1.625
1.600
1.575
1.550
1.525
1.500
Ratio 550/470
0
10
20
30
40
50
60
Time after addition of coelenterazine
(min)
MIP-1β 100 nM
No stimulation

FIGURE 16

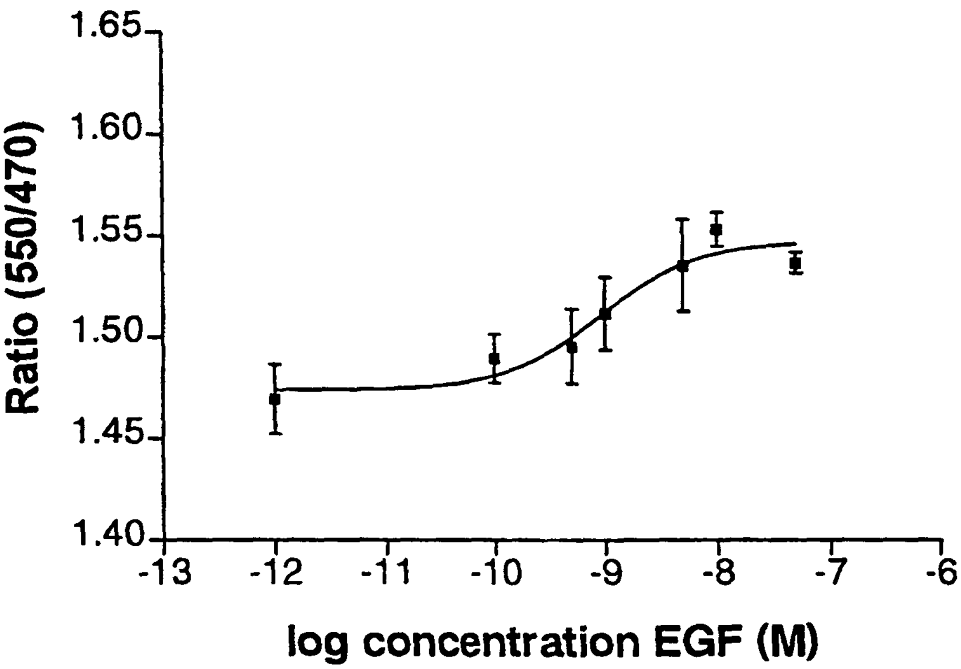
1.65
1.60
1.55
1.50
1.45
1.40
Ratio (550/470)
-13
-12
-11
-10
-9
-8
-7
-6
log concentration EGF (M)

FIGURE 17

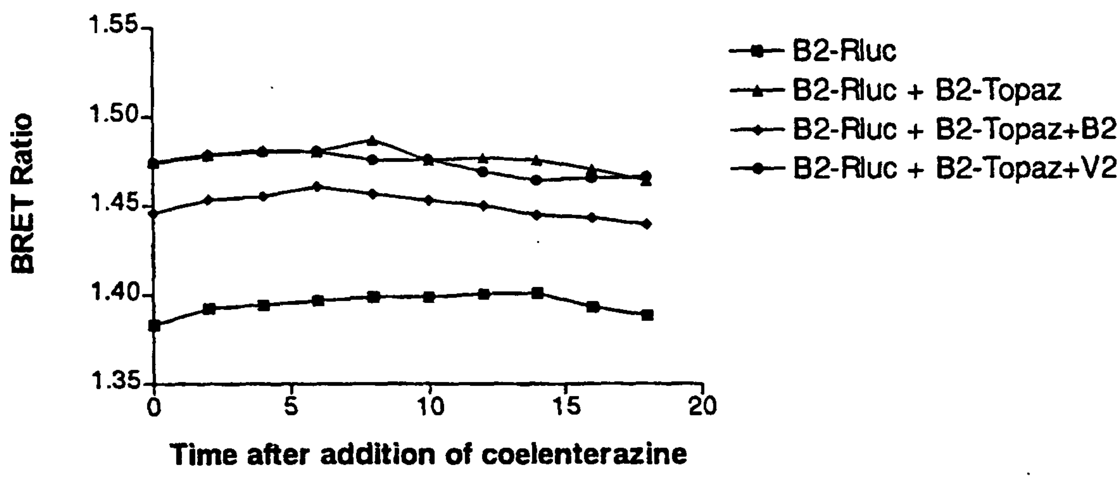
1.55
1.50
1.45
1.40
1.35
BRET Ratio
0
5
10
15
20
Time after addition of coelenterazine
B2-Rluc
B2-Rluc + B2-Topaz
B2-Rluc + B2-Topaz+B2
B2-Rluc + B2-Topaz+V2

FIGURE 18

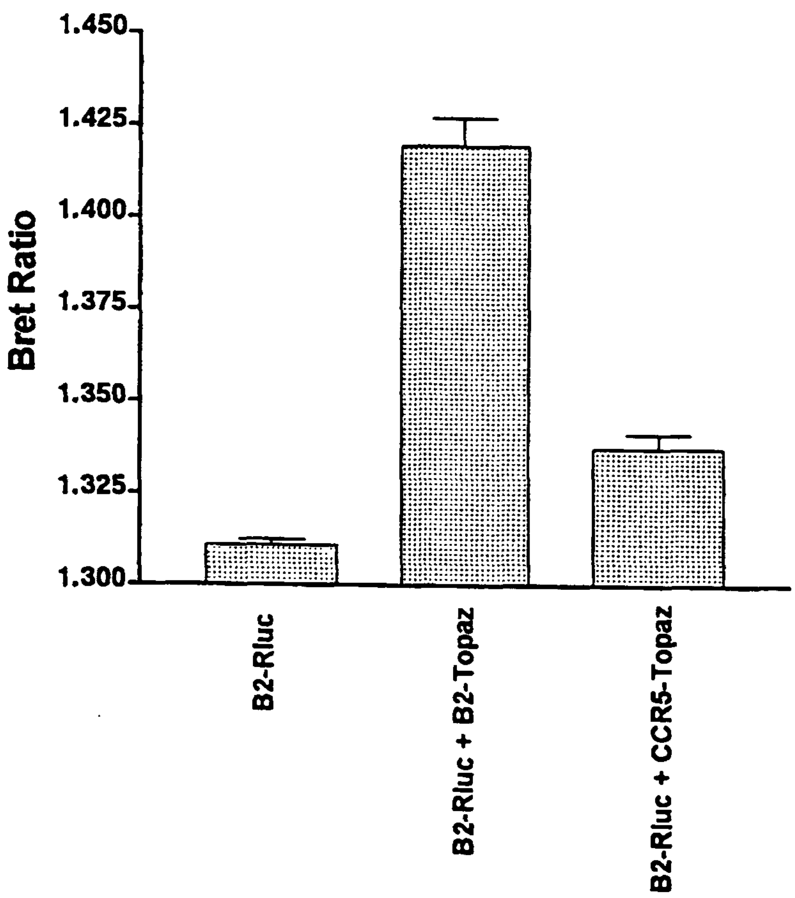
1.450
1.425
1.400
1.375
1.350
1.325
1.300
Bret Ratio
B2-Rluc
B2-Rluc + B2-Topaz
B2-Rluc + CCR5-Topaz

FIGURE 19

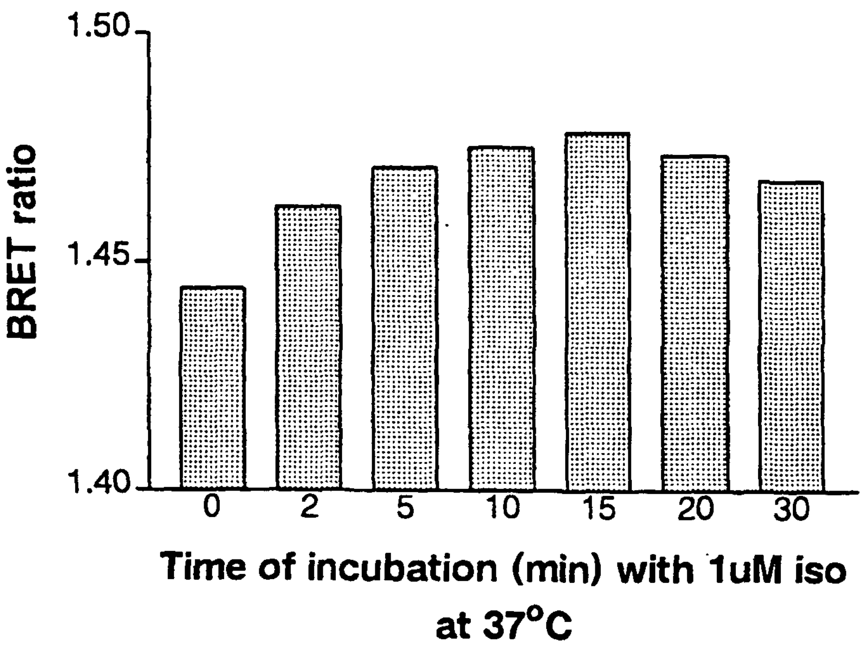
BRET ratio
1.50
1.45
1.40
0
2
5
10
15
20
30
Time of incubation (min) with 1uM iso at 37°C

FIGURE 20

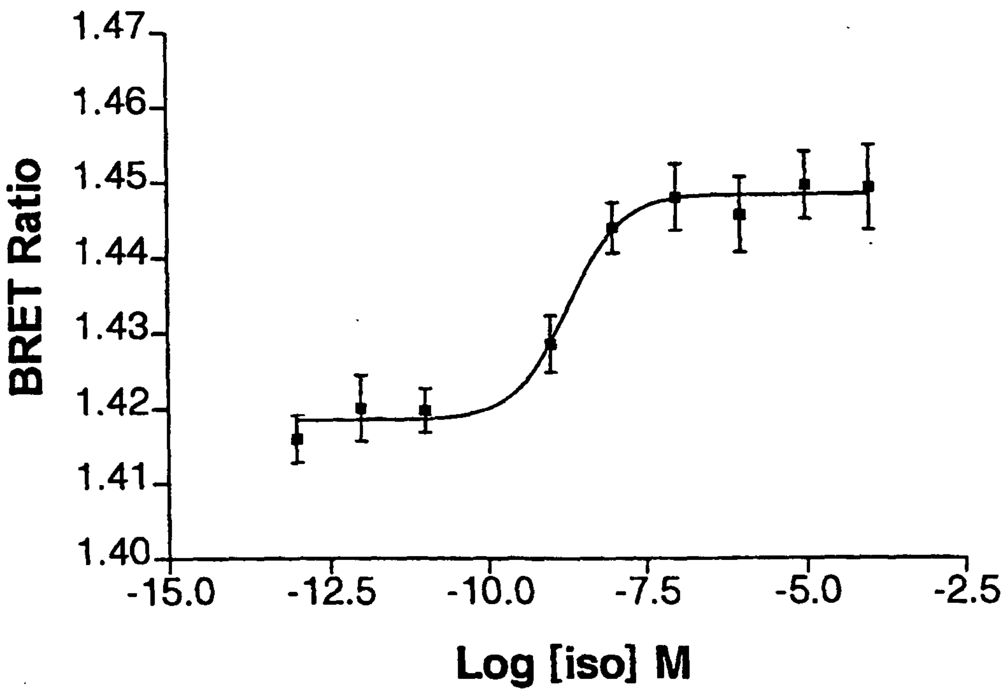
1.47
1.46
1.45
1.44
1.43
1.42
1.41
1.40
BRET Ratio
-15.0
-12.5
-10.0
-7.5
-5.0
-2.5
Log [iso] M

FIGURE 21

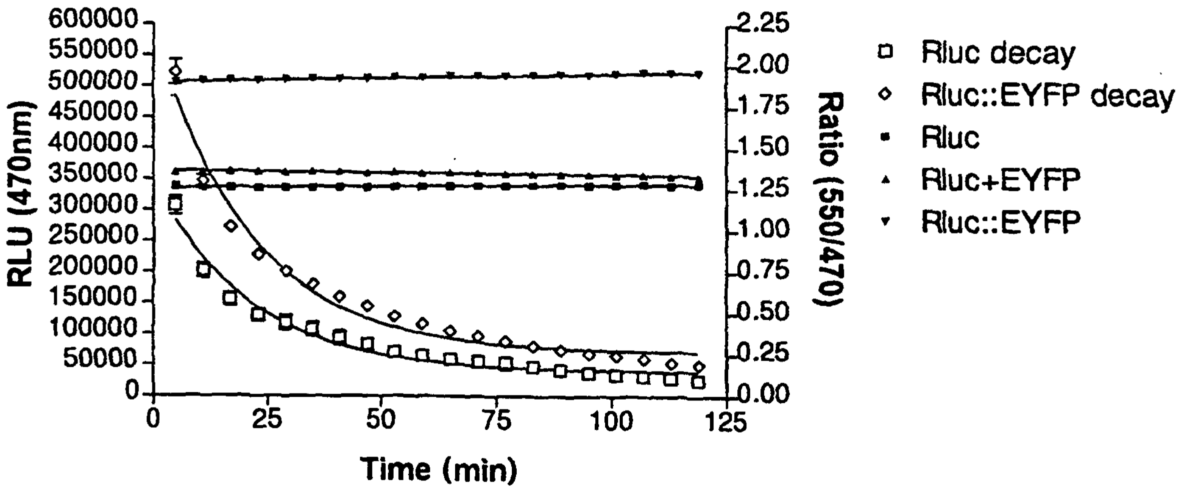
600000
550000
500000
450000
400000
350000
300000
250000
200000
150000
100000
50000
0
RLU (470nm)
2.25
2.00
1.75
1.50
1.25
1.00
0.75
0.50
0.25
0.00
Ratio (550/470)
0
25
50
75
100
125
Time (min)
Rluc decay
Rluc::EYFP decay
Rluc
Rluc+EYFP
Rluc::EYFP

FIGURE 22

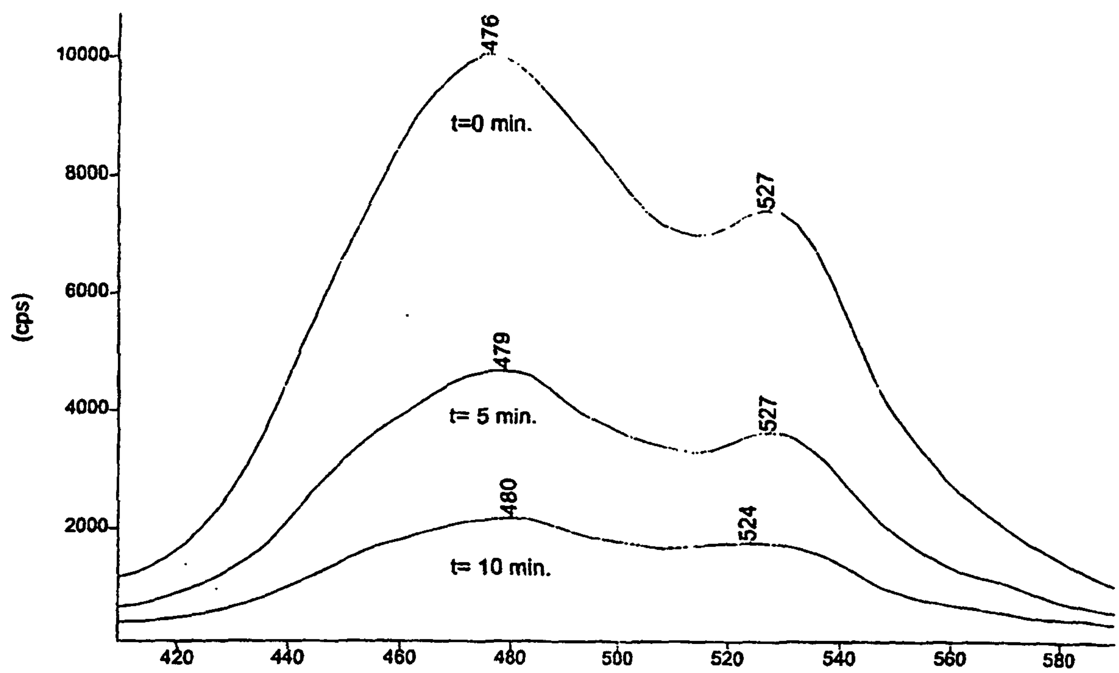


Emission Acquistion

FIGURE 23

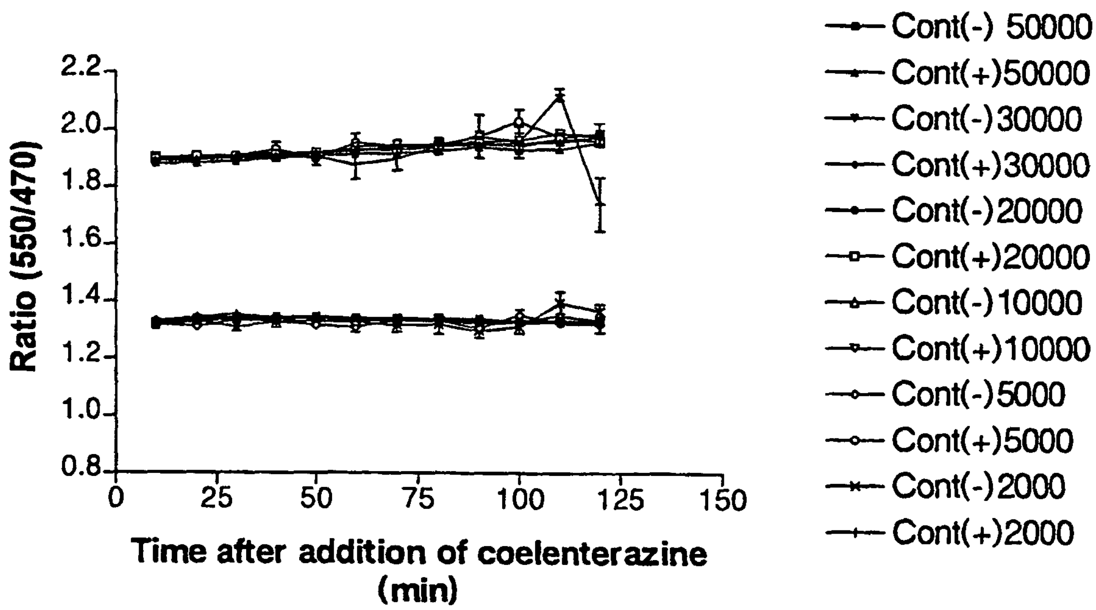
Ratio (550/470)
2.2
2.0
1.8
1.6
1.4
1.2
1.0
0.8
0
25
50
75
100
125
150
Time after addition of coelenterazine (min)
Cont(-) 50000
Cont(+)50000
Cont(-)30000
Cont(+)30000
Cont(-)20000
Cont(+)20000
Cont(-)10000
Cont(+)10000
Cont(-)5000
Cont(+)5000
Cont(-)2000
Cont(+)2000

FIGURE 24

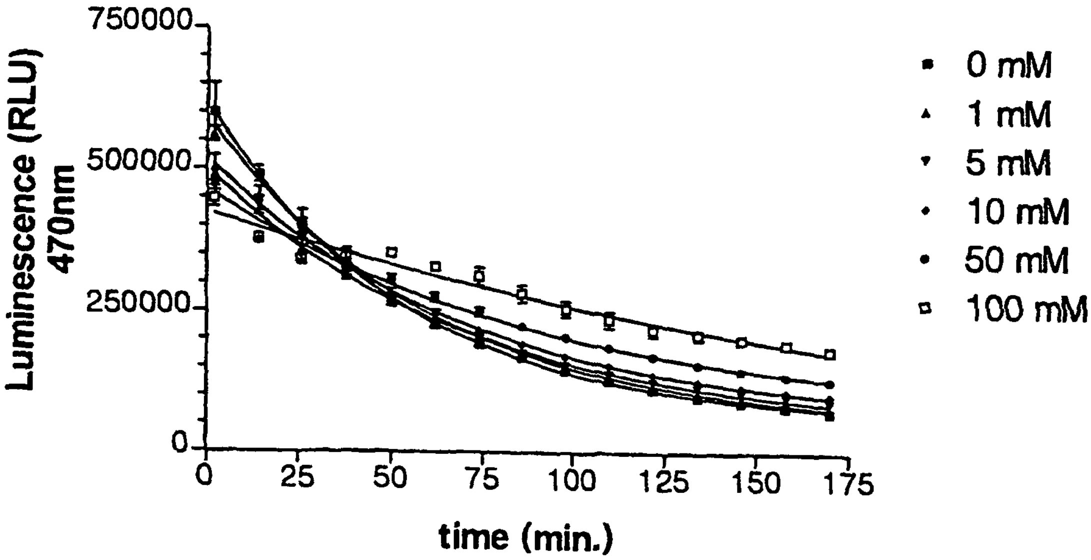
750000
500000
250000
0
Luminescence (RLU) 470nm
0
25
50
75
100
125
150
175
time (min.)
0 mM
1 mM
5 mM
10 mM
50 mM
100 mM

FIGURE 25

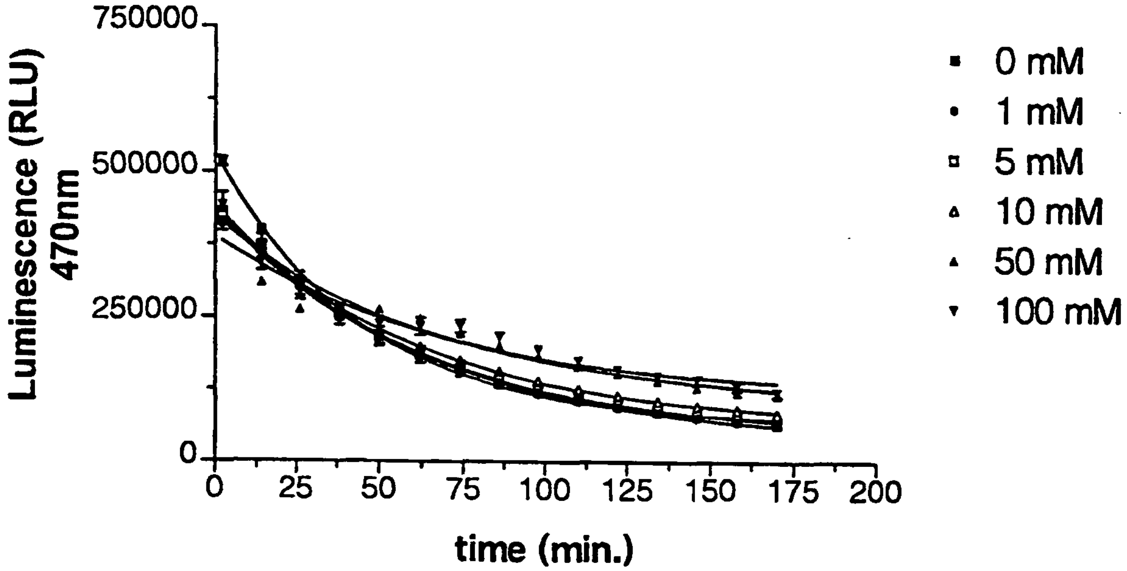

750000
500000
250000
0
Luminescence (RLU) 470nm
0
25
50
75
100
125
150
175
200
time (min.)
0 mM
1 mM
5 mM
10 mM
50 mM
100 mM

FIGURE 26

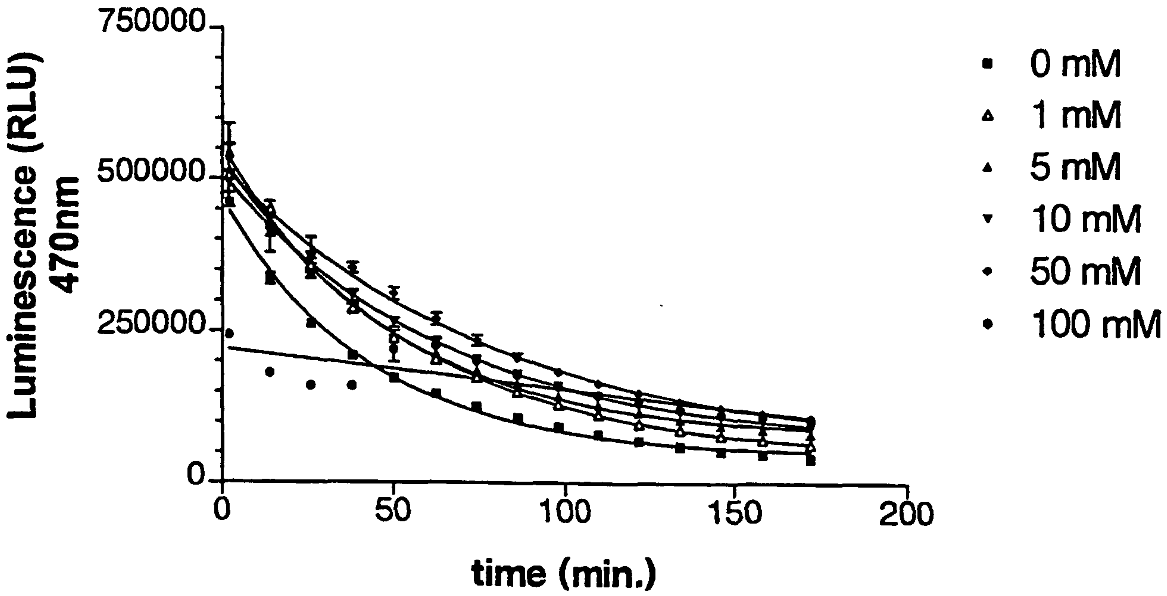

Luminescence (RLU)
470nm
750000
500000
250000
0
0
50
100
150
200
time (min.)
0 mM
1 mM
5 mM
10 mM
50 mM
100 mM

FIGURE 27

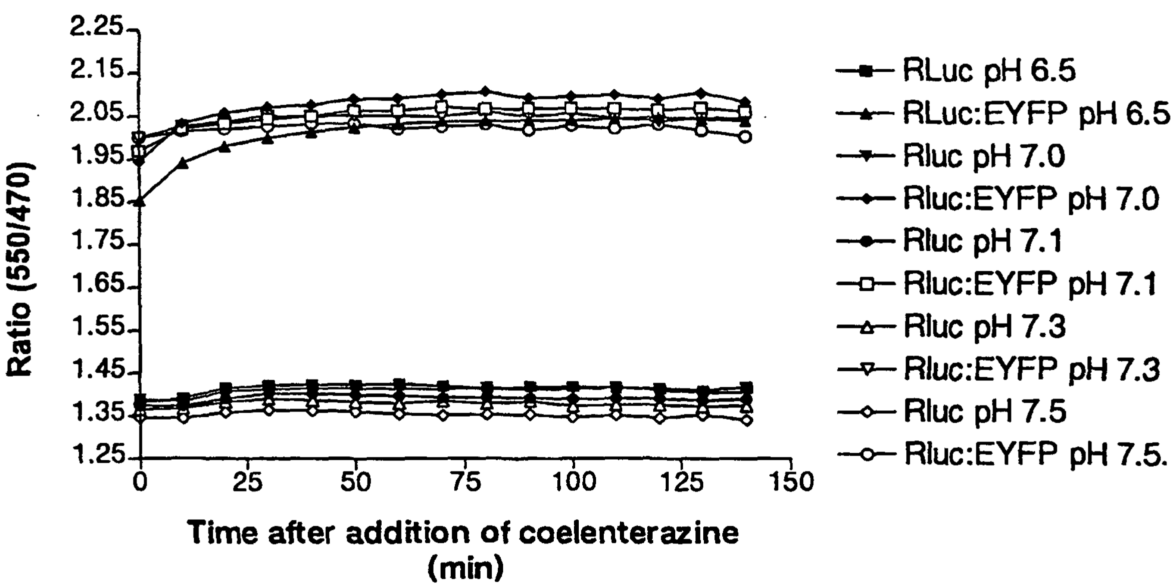
2.25
2.15
2.05
1.95
1.85
1.75
1.65
1.55
1.45
1.35
1.25
Ratio (550/470)
0
25
50
75
100
125
150
Time after addition of coelenterazine (min)
RLuc pH 6.5
RLuc:EYFP pH 6.5
Rluc pH 7.0
Rluc:EYFP pH 7.0
Rluc pH 7.1
Rluc:EYFP pH 7.1
Rluc pH 7.3
Rluc:EYFP pH 7.3
Rluc pH 7.5
Rluc:EYFP pH 7.5.

FIGURE 28

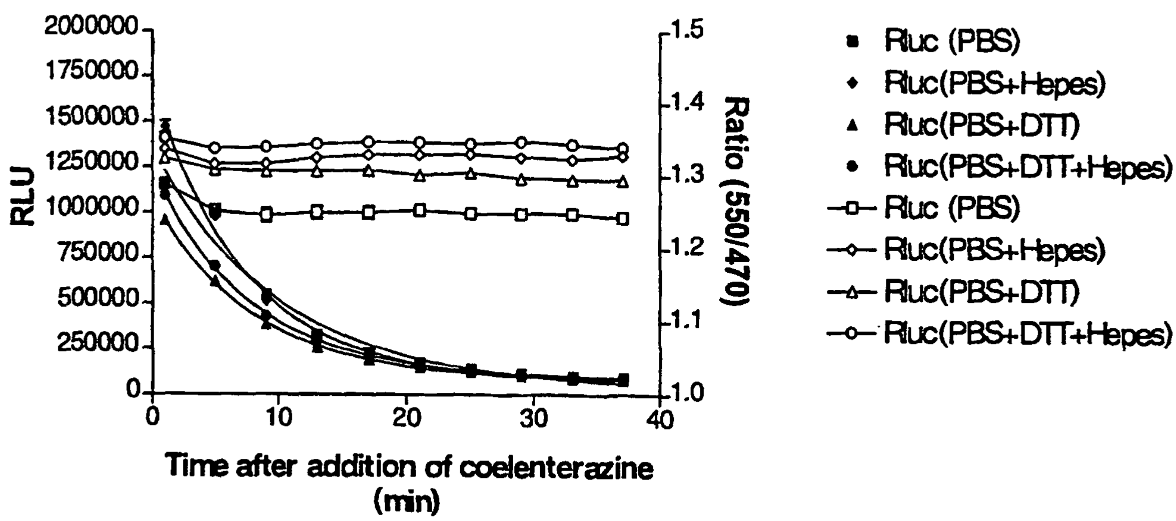

2000000
1750000
1500000
1250000
1000000
750000
500000
250000
0
RLU
0
10
20
30
40
Time after addition of coelenterazine (min)
1.5
1.4
1.3
1.2
1.1
1.0
Ratio (550/470)
Rluc (PBS)
Rluc(PBS+Hepes)
Rluc(PBS+DTT)
Rluc(PBS+DTT+Hepes)
Rluc (PBS)
Rluc(PBS+Hepes)
Rluc(PBS+DTT)
Rluc(PBS+DTT+Hepes)

FIGURE 29

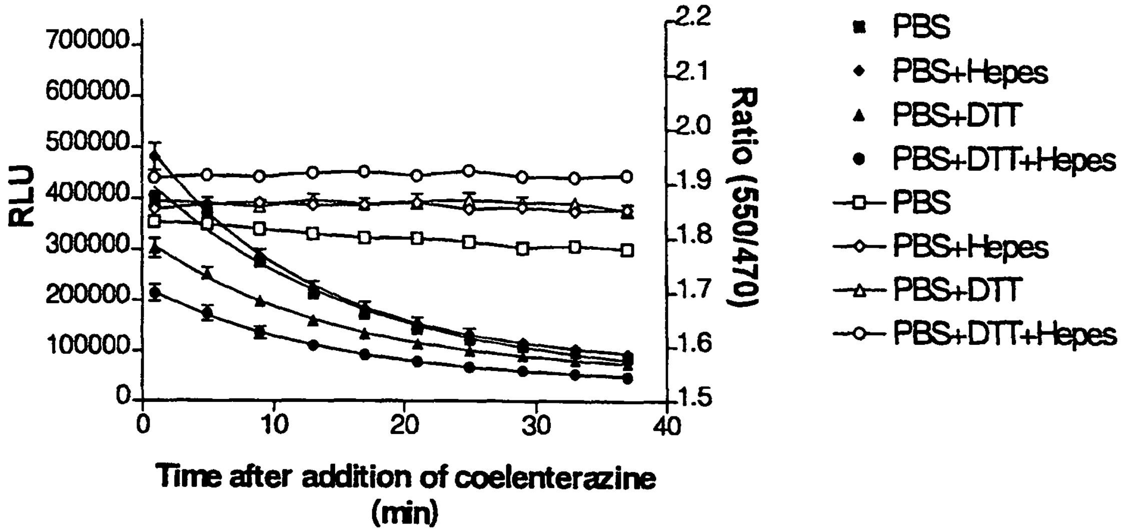
RLU
700000
600000
500000
400000
300000
200000
100000
0
2.2
2.1
2.0
1.9
1.8
1.7
1.6
1.5
Ratio (550/470)
0
10
20
30
40
Time after addition of coelenterazine (min)
PBS
PBS+Hepes
PBS+DTT
PBS+DTT+Hepes
PBS
PBS+Hepes
PBS+DTT
PBS+DTT+Hepes

FIGURE 30

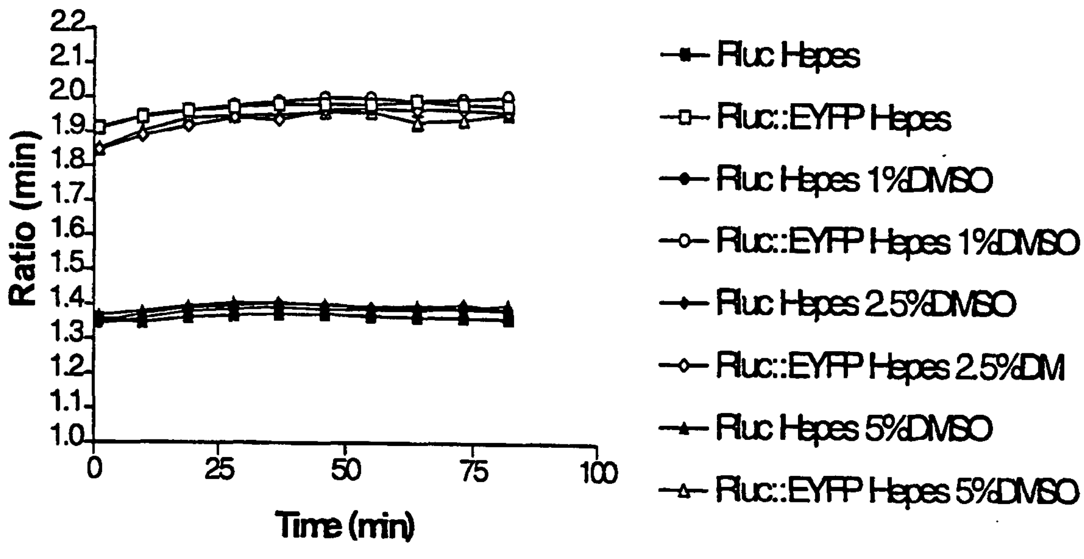

Ratio (min)
2.2
2.1
2.0
1.9
1.8
1.7
1.6
1.5
1.4
1.3
1.2
1.1
1.0
0
25
50
75
100
Time (min)
Rluc Hepes
Rluc::EYFP Hepes
Rluc Hepes 1%DMSO
Rluc::EYFP Hepes 1%DMSO
Rluc Hepes 2.5%DMSO
Rluc::EYFP Hepes 2.5%DM
Rluc Hepes 5%DMSO
Rluc::EYFP Hepes 5%DMSO

FIGURE 31

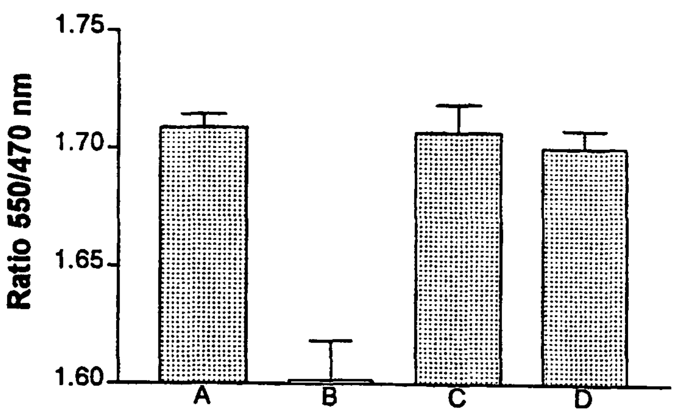


A: no stimulation
B: staurosporine 1µM
C: staurosporine 1µM + caspase-3 inhibitor (2nM)
D: caspase-3 inhibitor (10nM)

FIGURE 32

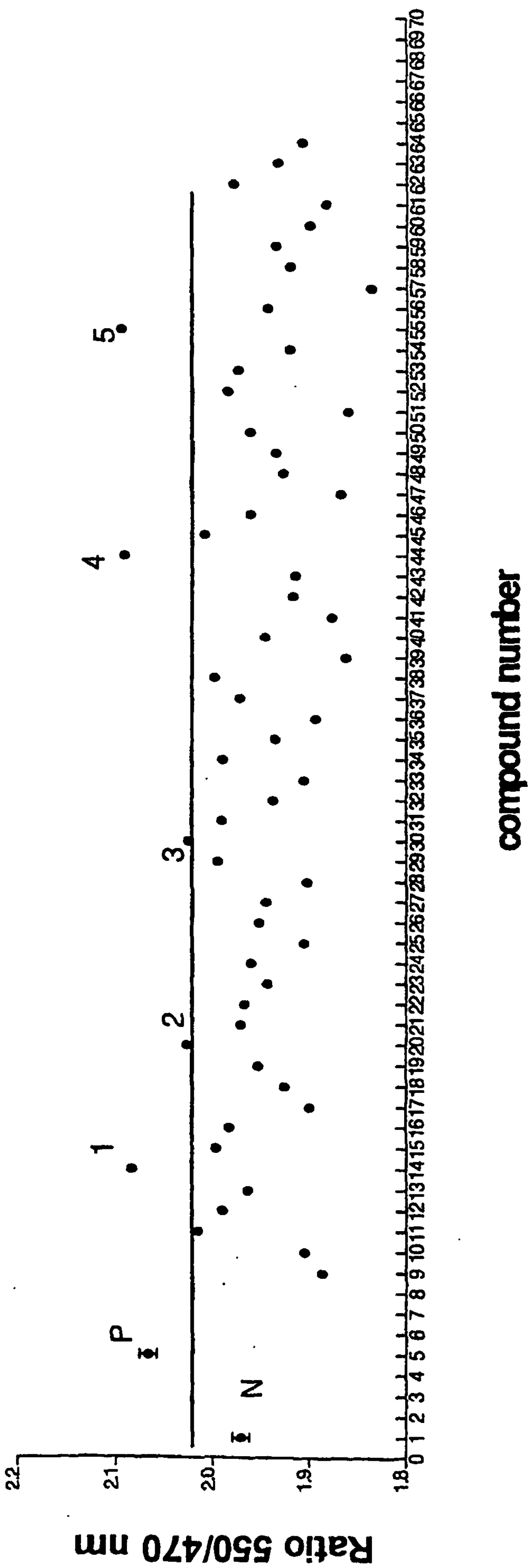

FIGURE 33

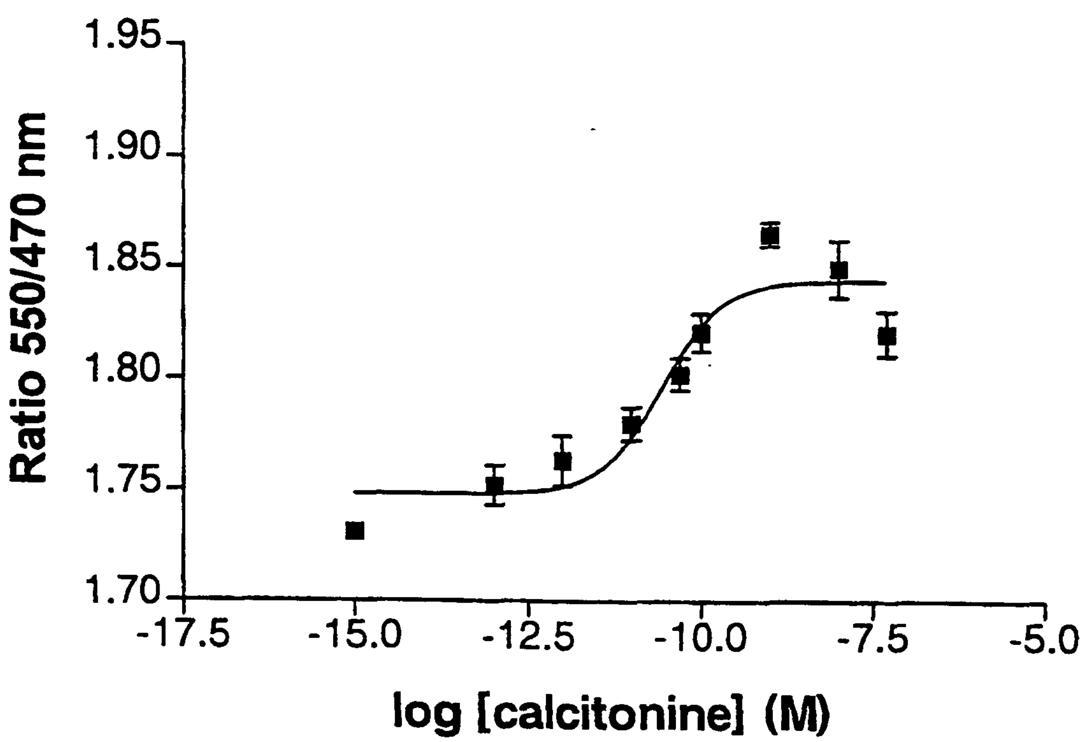
Ratio 550/470 nm
1.95
1.90
1.85
1.80
1.75
1.70
-17.5
-15.0
-12.5
-10.0
-7.5
-5.0
log [calcitonine] (M)

FIGURE 34

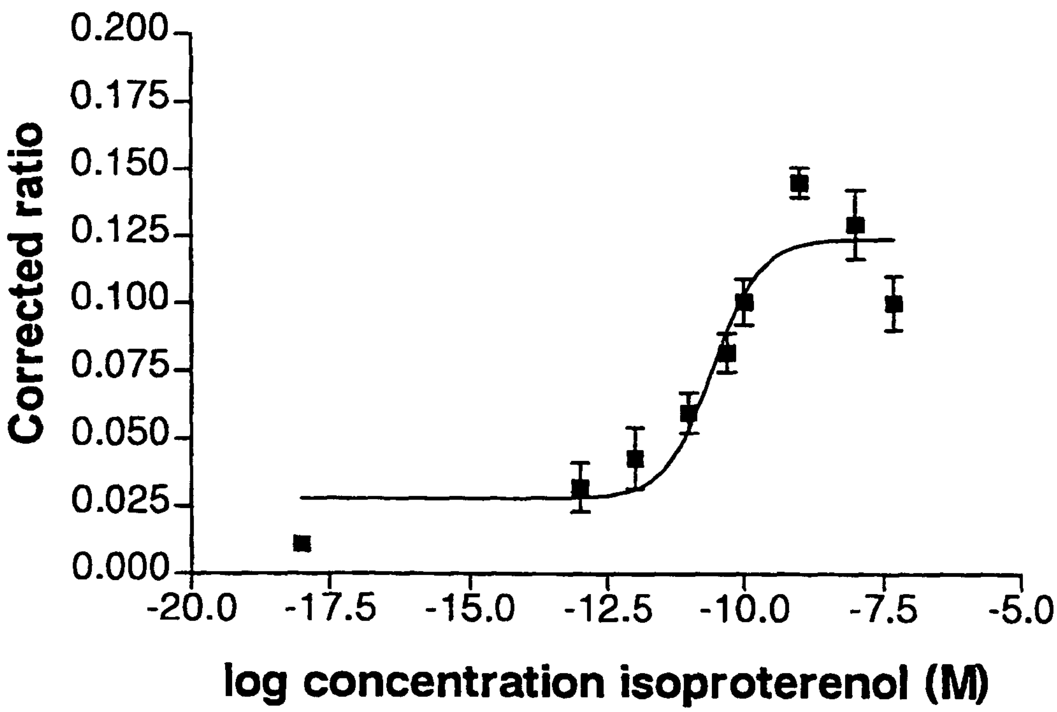
0.200
0.175
0.150
0.125
0.100
0.075
0.050
0.025
0.000
Corrected ratio
-20.0
-17.5
-15.0
-12.5
-10.0
-7.5
-5.0
log concentration isoproterenol (M)

FIGURE 35

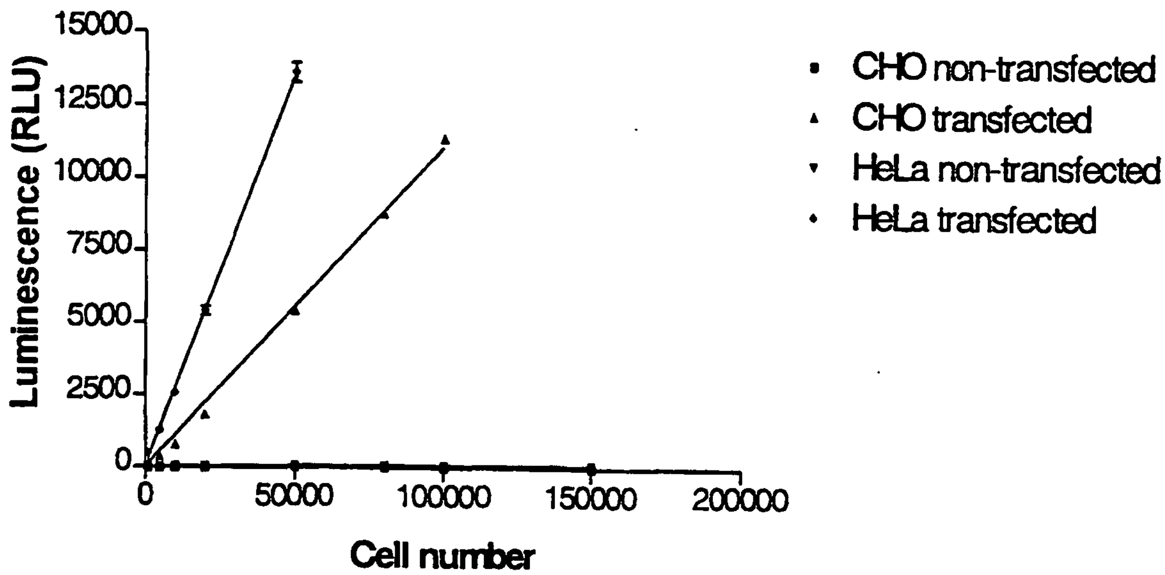
15000
12500
10000
7500
5000
2500
0
Luminescence (RLU)
0
50000
100000
150000
200000
Cell number
CHO non-transfected
CHO transfected
HeLa non-transfected
HeLa transfected

FIGURE 36

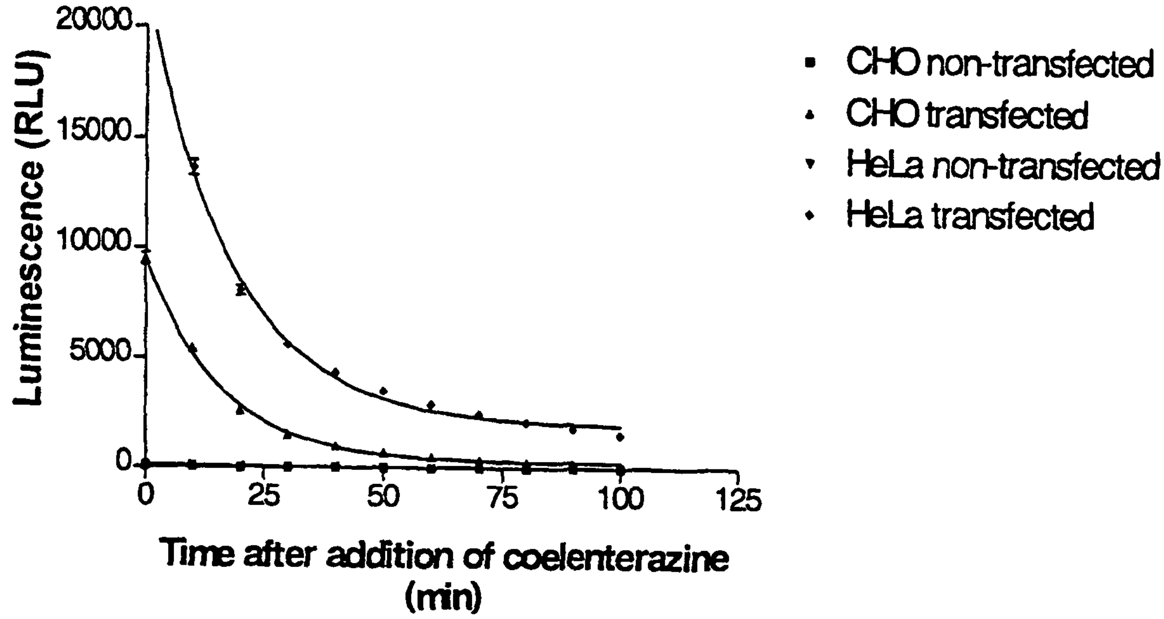

Luminescence (RLU)
20000
15000
10000
5000
0
0
25
50
75
100
125
Time after addition of coelenterazine (min)
CHO non-transfected
CHO transfected
HeLa non-transfected
HeLa transfected

FIGURE 37

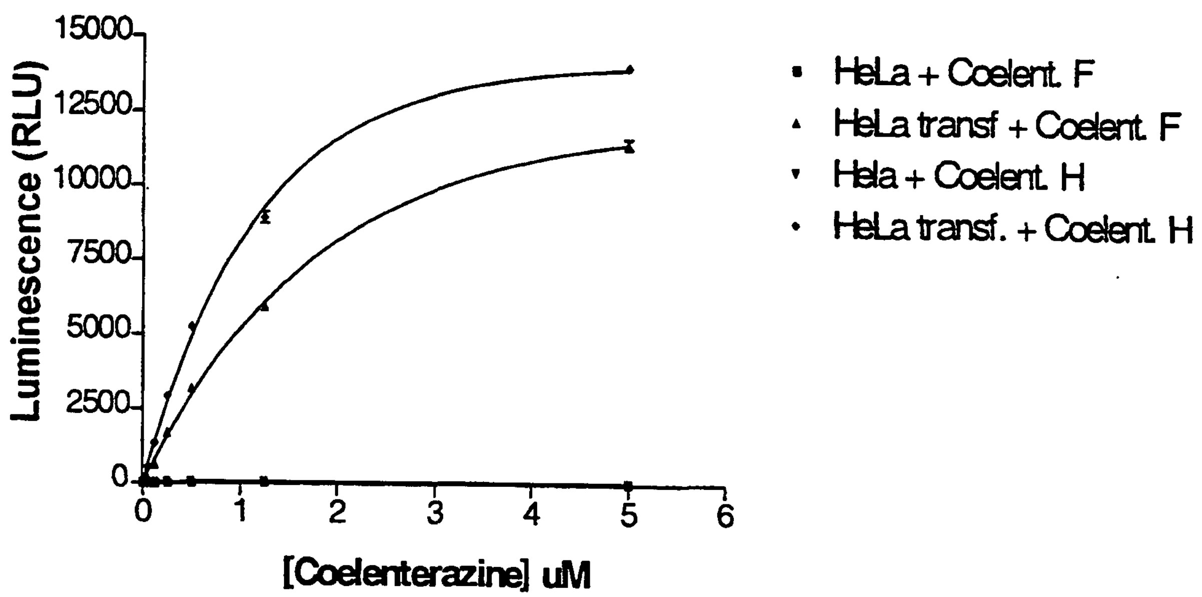
15000
12500
10000
7500
5000
2500
0
Luminescence (RLU)
0
1
2
3
4
5
6
[Coelenterazine] uM
HeLa + Coelent. F
HeLa transf + Coelent. F
Hela + Coelent. H
HeLa transf. + Coelent. H

FIGURE 38

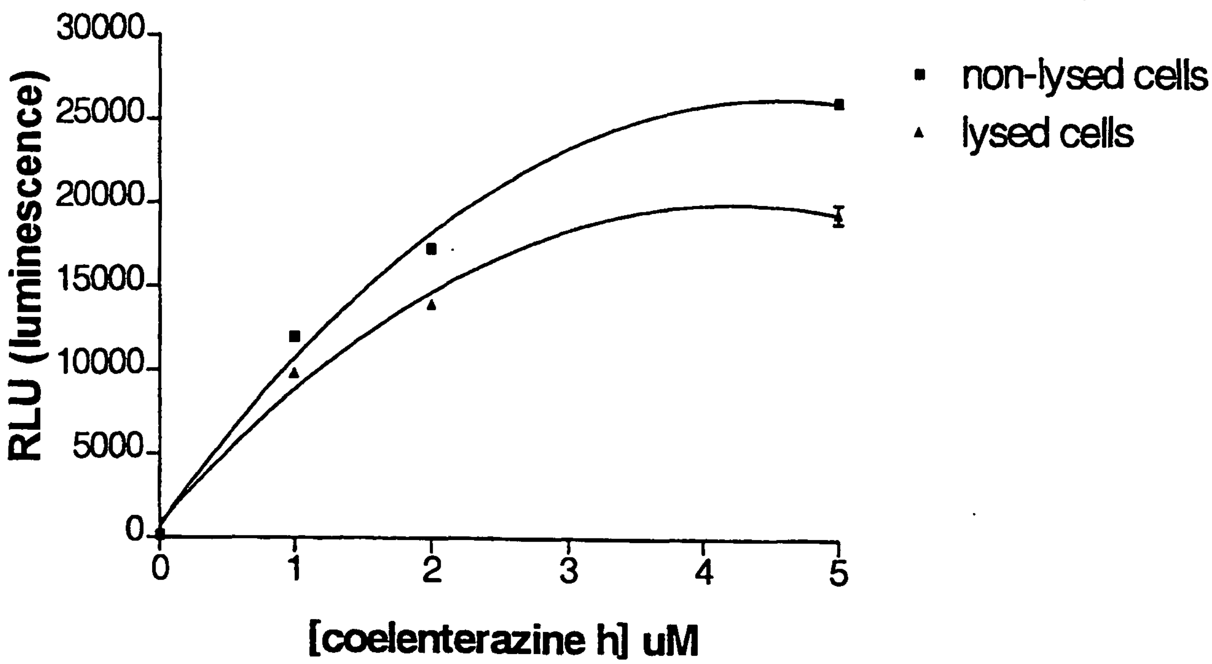
RLU (luminescence)
30000
25000
20000
15000
10000
5000
0
0
1
2
3
4
5
[coelenterazine h] uM
non-lysed cells
lysed cells

FIGURE 39

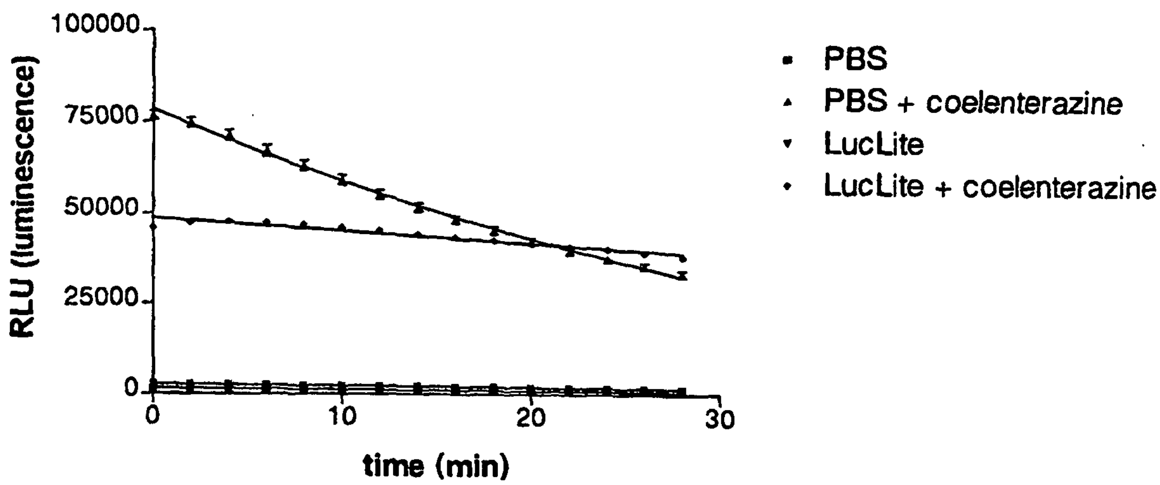
100000
75000
50000
25000
0
RLU (luminescence)
0
10
20
30
time (min)
PBS
PBS + coelenterazine
LucLite
LucLite + coelenterazine

FIGURE 40

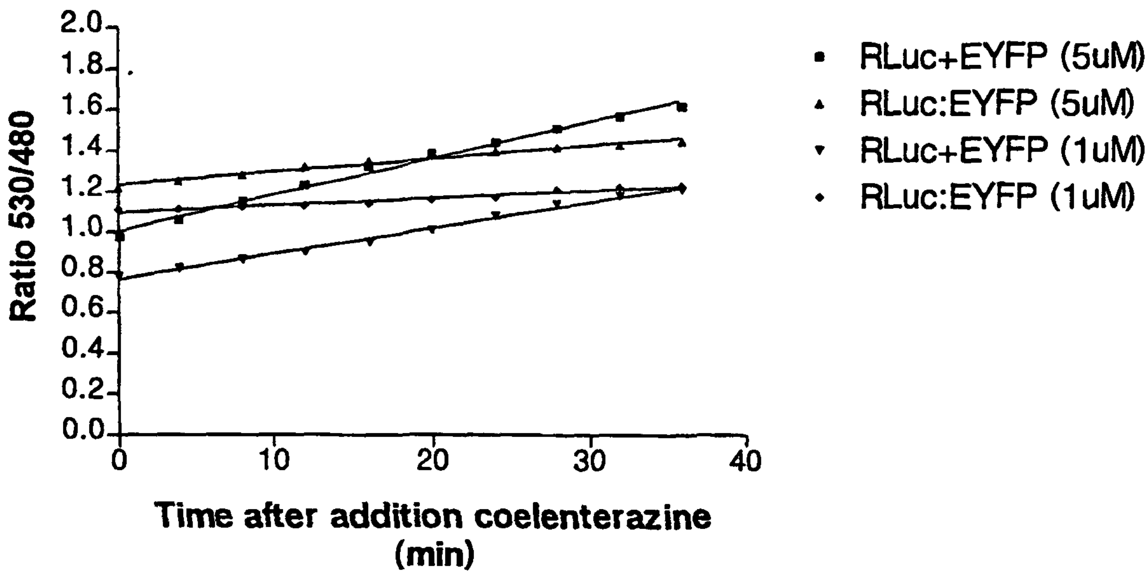
2.0
1.8
1.6
1.4
1.2
1.0
0.8
0.6
0.4
0.2
0.0
Ratio 530/480
0
10
20
30
40
Time after addition coelenterazine (min)
RLuc+EYFP (5uM)
RLuc:EYFP (5uM)
RLuc+EYFP (1uM)
RLuc:EYFP (1uM)

FIGURE 41

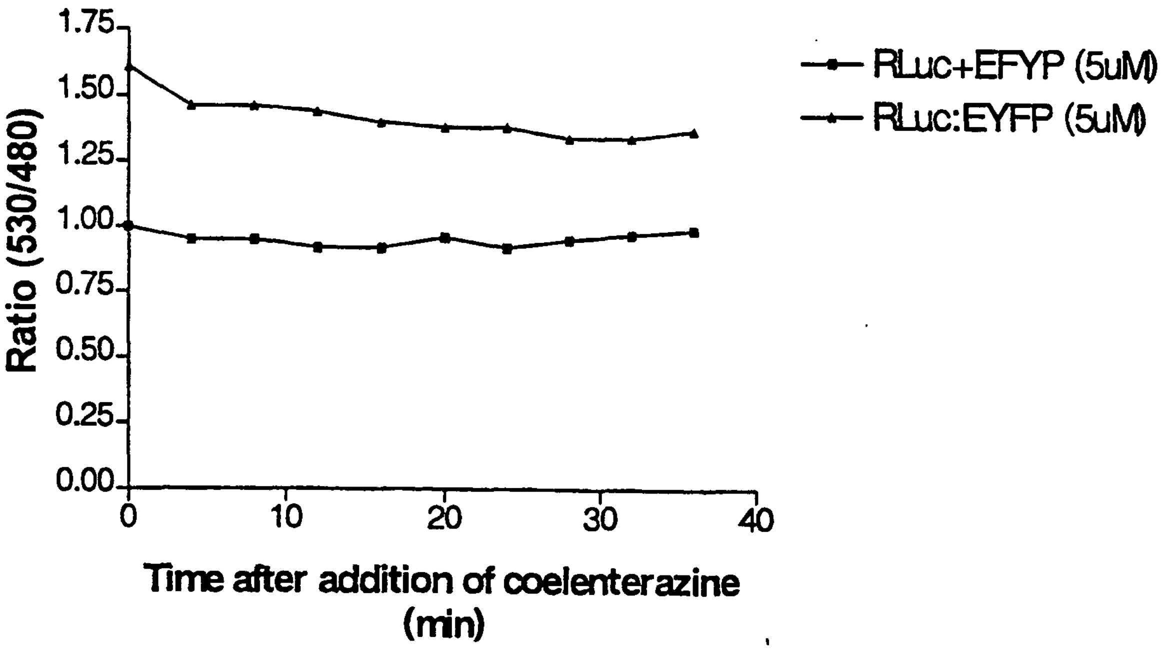

1.75
1.50
1.25
1.00
0.75
0.50
0.25
0.00
Ratio (530/480)
0
10
20
30
40
Time after addition of coelenterazine (min)
RLuc+EFYP (5uM)
RLuc:EYFP (5uM)

FIGURE 42

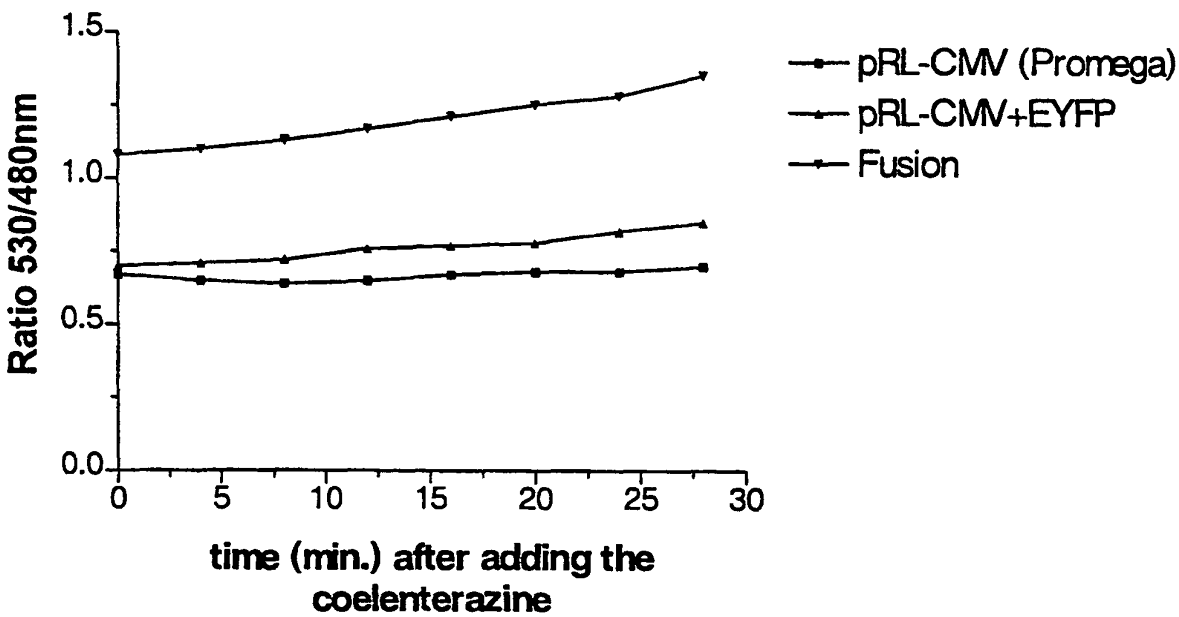
1.5
1.0
0.5
0.0
Ratio 530/480nm
0
5
10
15
20
25
30
time (min.) after adding the coelenterazine
pRL-CMV (Promega)
pRL-CMV+EYFP
Fusion

FIGURE 43

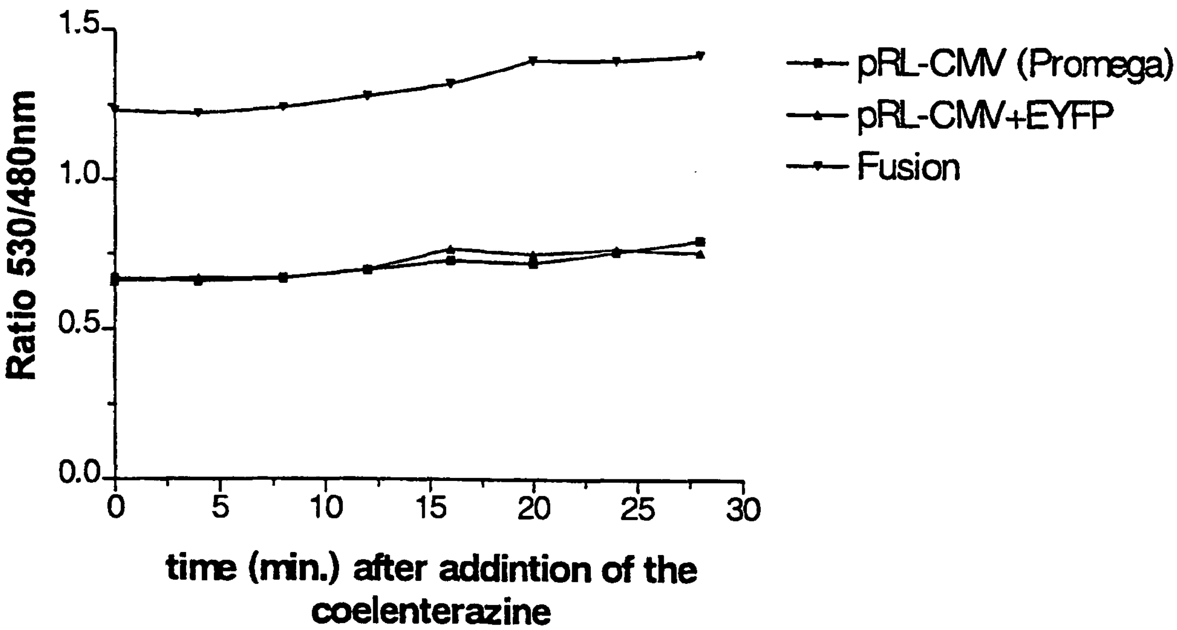
1.5
1.0
0.5
0.0
Ratio 530/480nm
0
5
10
15
20
25
30
time (min.) after addintion of the coelenterazine
pRL-CMV (Promega)
pRL-CMV+EYFP
Fusion

FIGURE 44

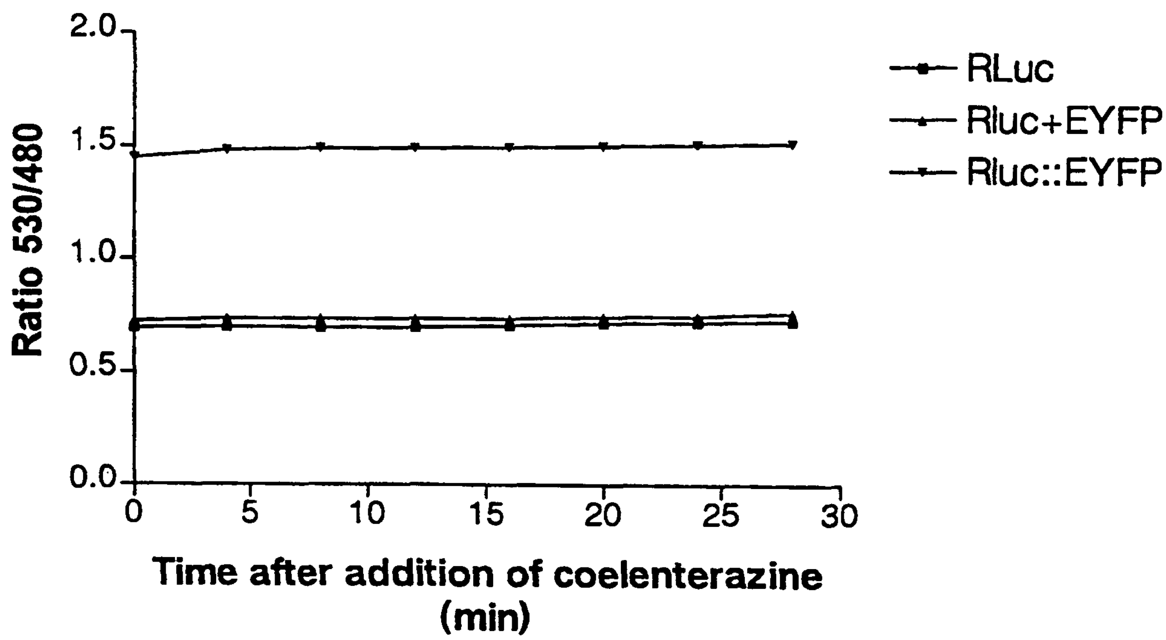
2.0
1.5
1.0
0.5
0.0
Ratio 530/480
0
5
10
15
20
25
30
Time after addition of coelenterazine
(min)
RLuc
Rluc+EYFP
Rluc::EYFP

FIGURE 45

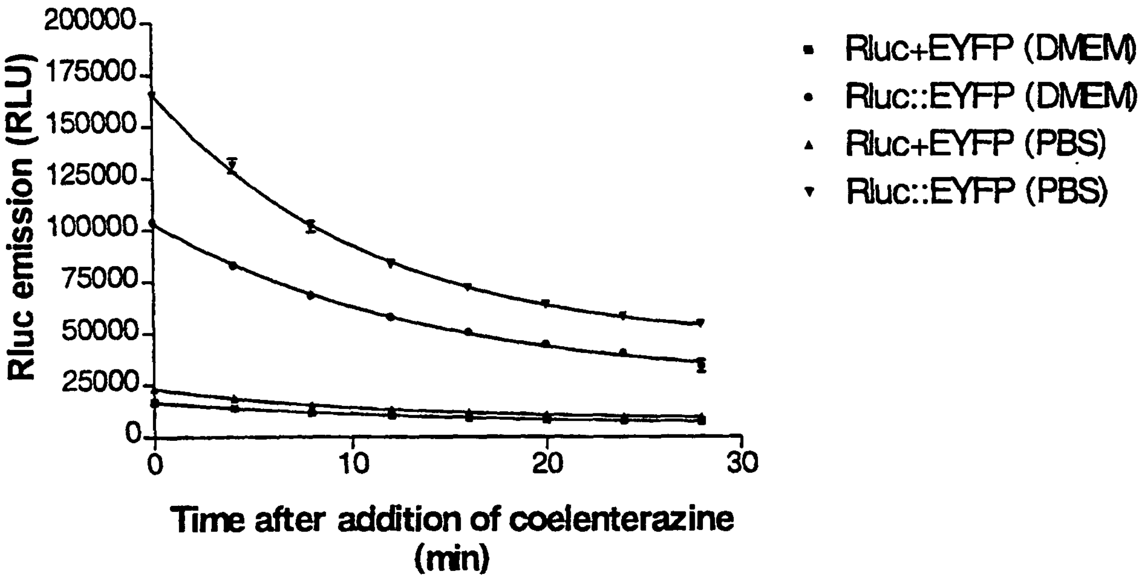
200000
175000
150000
125000
100000
75000
50000
25000
0
Rluc emission (RLU)
0
10
20
30
Time after addition of coelenterazine (min)
Rluc+EYFP (DMEM)
Rluc::EYFP (DMEM)
Rluc+EYFP (PBS)
Rluc::EYFP (PBS)

FIGURE 46

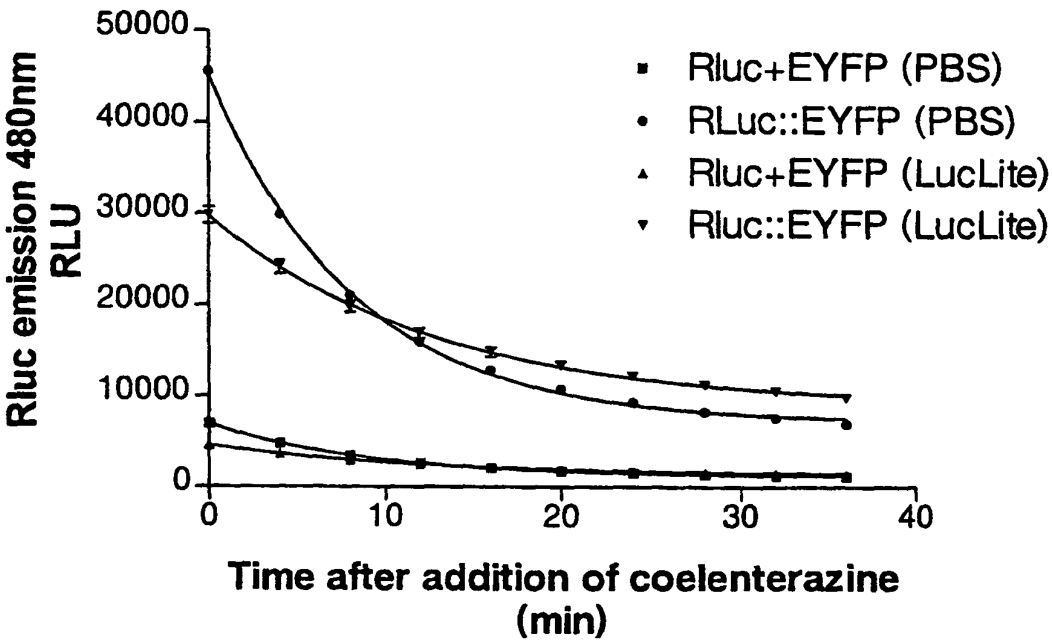
50000
40000
30000
20000
10000
0
0
10
20
30
40
Rluc emission 480nm
RLU
Rluc+EYFP (PBS)
RLuc::EYFP (PBS)
Rluc+EYFP (LucLite)
Rluc::EYFP (LucLite)
Time after addition of coelenterazine
(min)

FIGURE 47

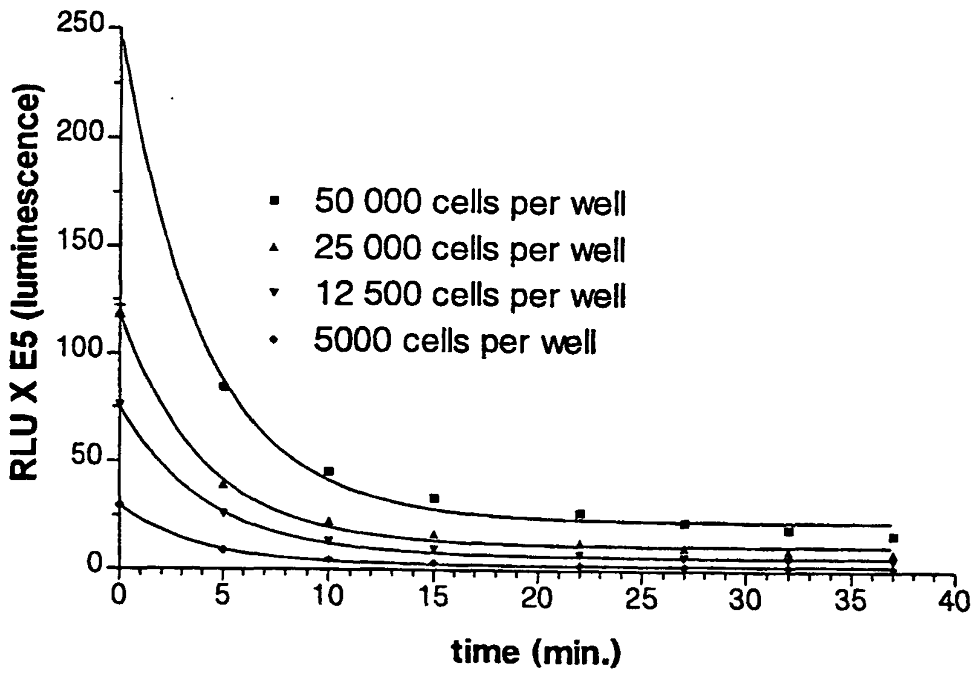
250
200
150
100
50
0
RLU X E5 (luminescence)
50 000 cells per well
25 000 cells per well
12 500 cells per well
5000 cells per well
0
5
10
15
20
25
30
35
40
time (min.)

FIGURE 48

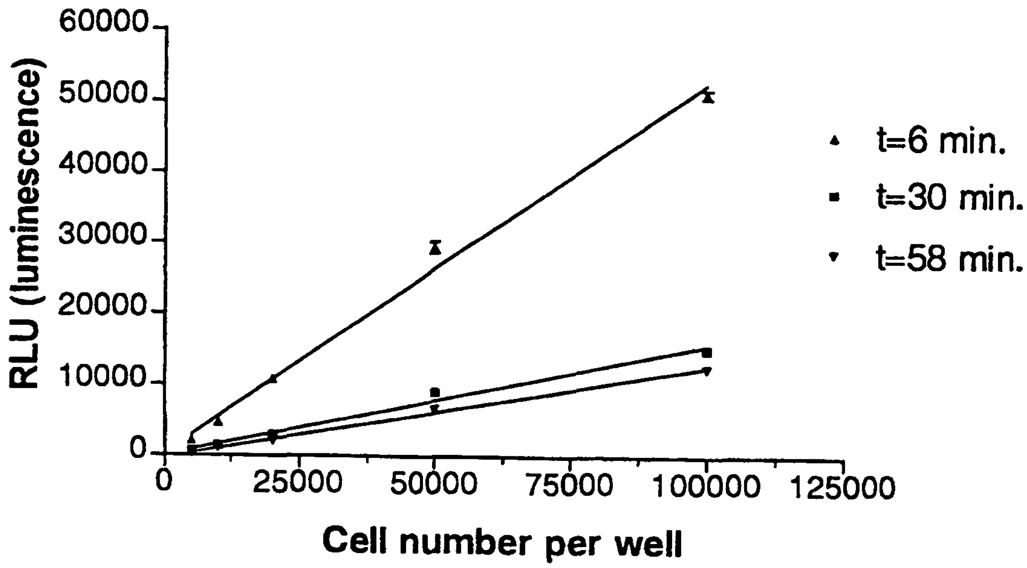
60000
50000
40000
30000
20000
10000
0
RLU (luminescence)
0
25000
50000
75000
100000
125000
Cell number per well
t=6 min.
t=30 min.
t=58 min.

FIGURE 49

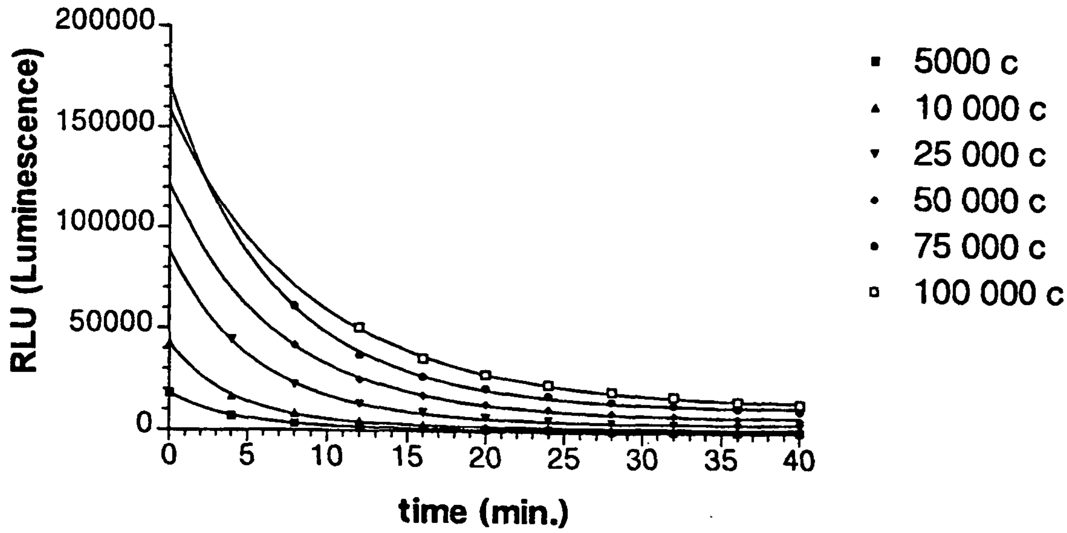
200000
150000
100000
50000
0
RLU (Luminescence)
0
5
10
15
20
25
30
35
40
time (min.)
5000 c
10 000 c
25 000 c
50 000 c
75 000 c
100 000 c

FIGURE 50

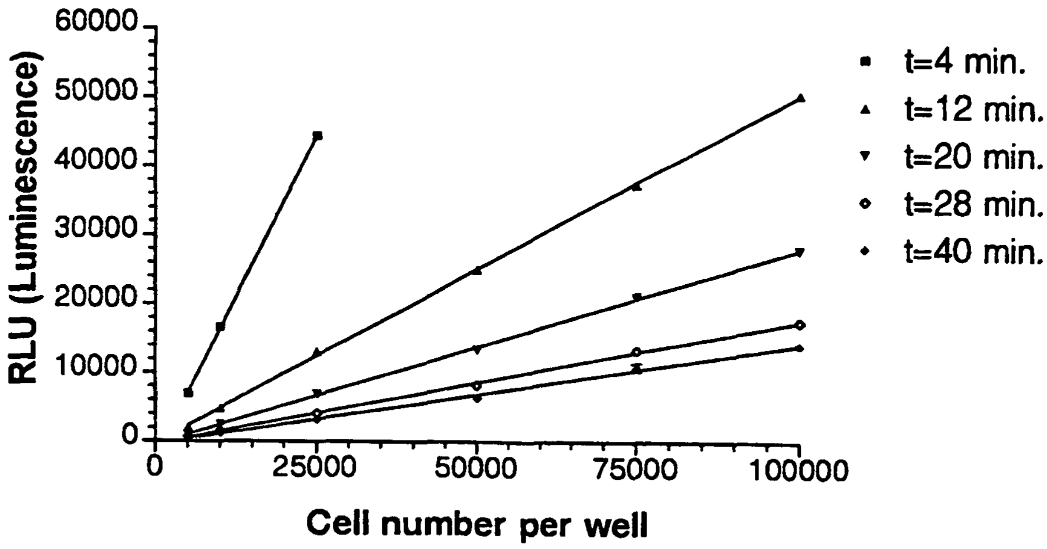
RLU (Luminescence)
60000
50000
40000
30000
20000
10000
0
0
25000
50000
75000
100000
Cell number per well
t=4 min.
t=12 min.
t=20 min.
t=28 min.
t=40 min.

FIGURE 51

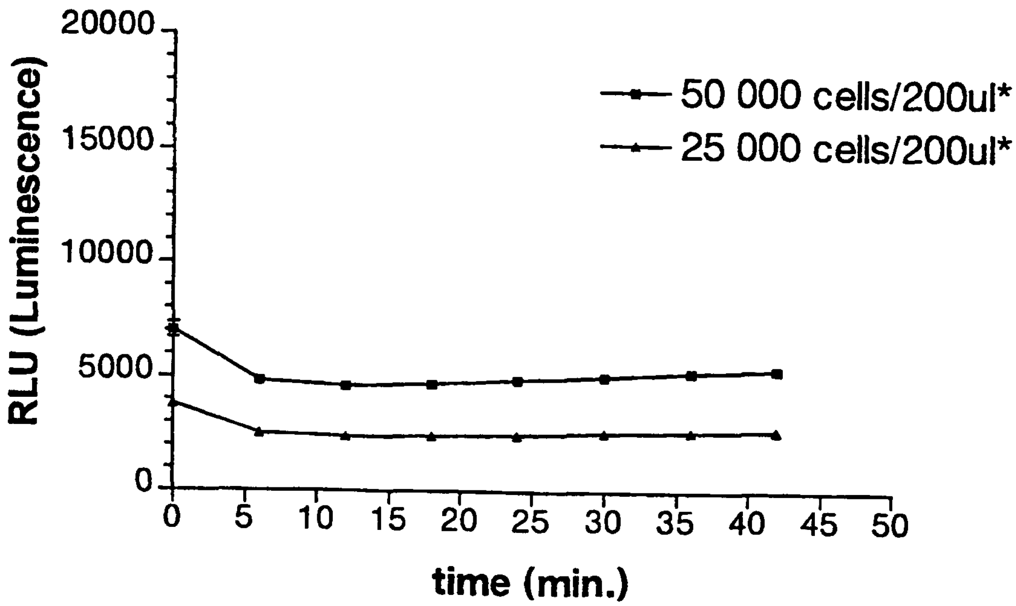
20000
15000
10000
5000
0
RLU (Luminescence)
50 000 cells/200ul*
25 000 cells/200ul*
0
5
10
15
20
25
30
35
40
45
50
time (min.)

FIGURE 52

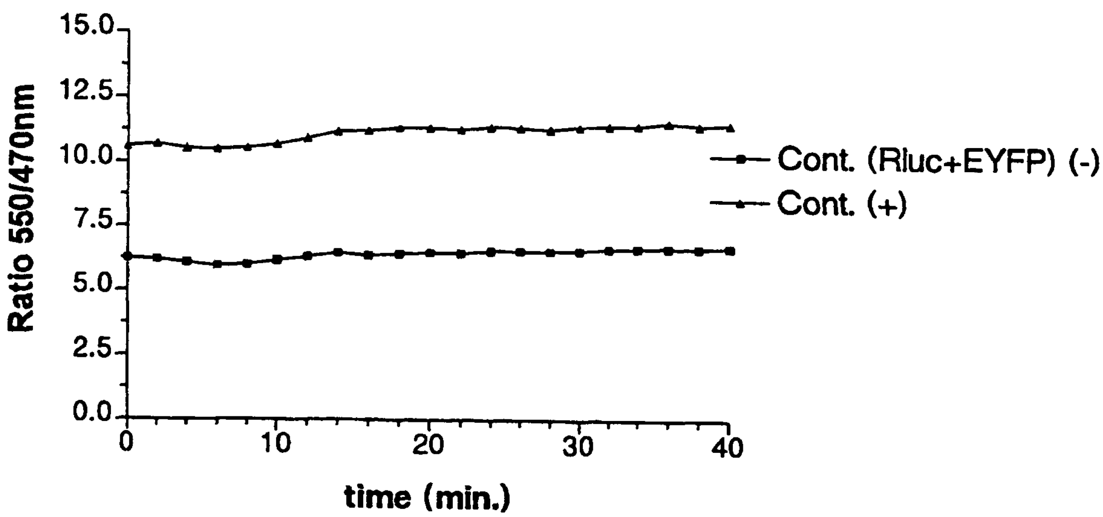
15.0
12.5
10.0
7.5
5.0
2.5
0.0
Ratio 550/470nm
0
10
20
30
40
time (min.)
Cont. (Rluc+EYFP) (-)
Cont. (+)

FIGURE 53

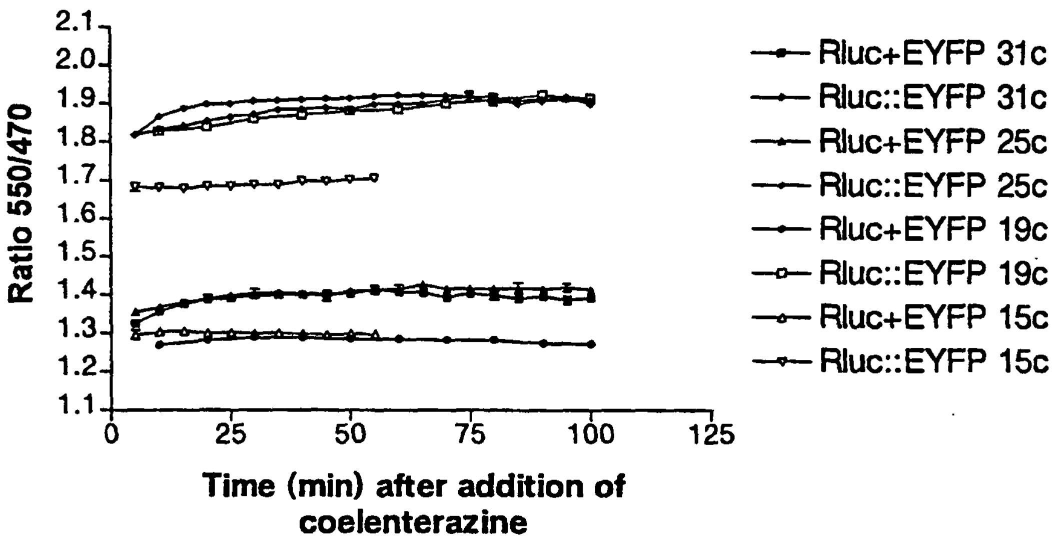
Ratio 550/470
2.1
2.0
1.9
1.8
1.7
1.6
1.5
1.4
1.3
1.2
1.1
0
25
50
75
100
125
Time (min) after addition of coelenterazine
Rluc+EYFP 31c
Rluc::EYFP 31c
Rluc+EYFP 25c
Rluc::EYFP 25c
Rluc+EYFP 19c
Rluc::EYFP 19c
Rluc+EYFP 15c
Rluc::EYFP 15c

FIGURE 54

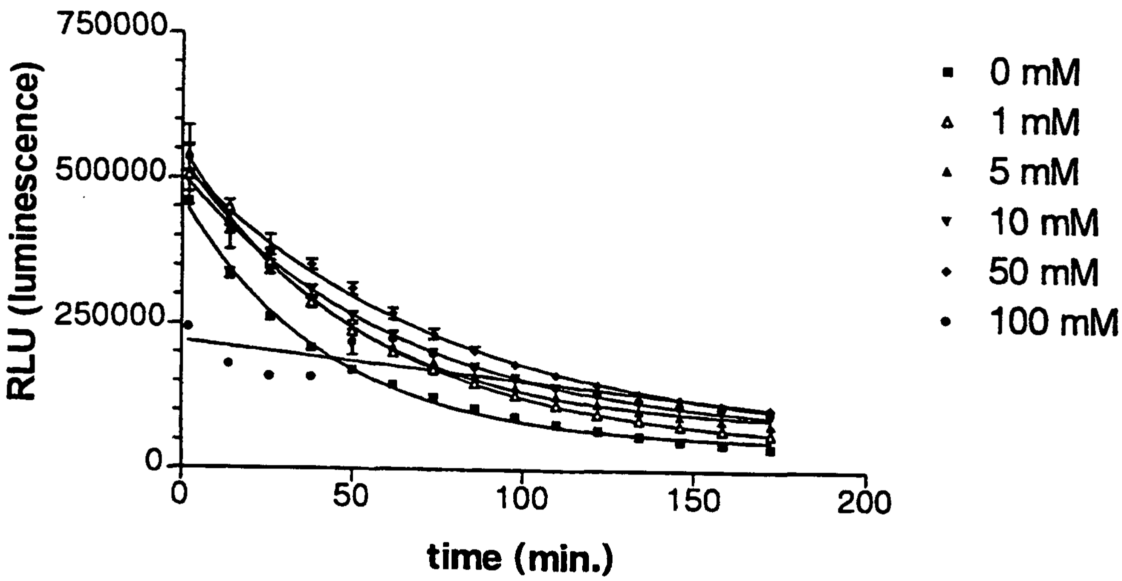
750000
500000
250000
0
RLU (luminescence)
0
50
100
150
200
time (min.)
0 mM
1 mM
5 mM
10 mM
50 mM
100 mM

FIGURE 55

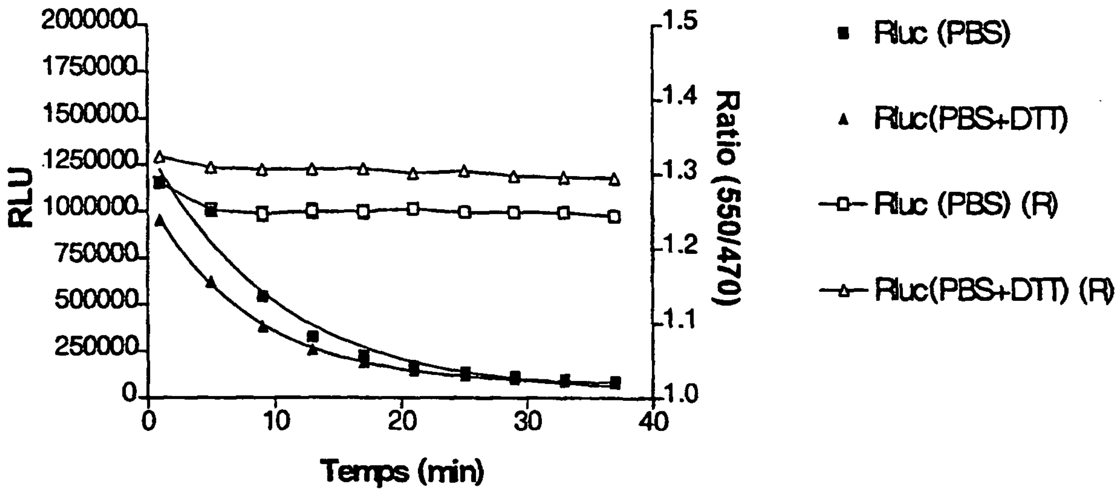
RLU
2000000
1750000
1500000
1250000
1000000
750000
500000
250000
0
0
10
20
30
40
Temps (min)
1.5
1.4
1.3
1.2
1.1
1.0
Ratio (550/470)
Rluc (PBS)
Rluc(PBS+DTT)
Rluc (PBS) (R)
Rluc(PBS+DTT) (R)

FIGURE 56

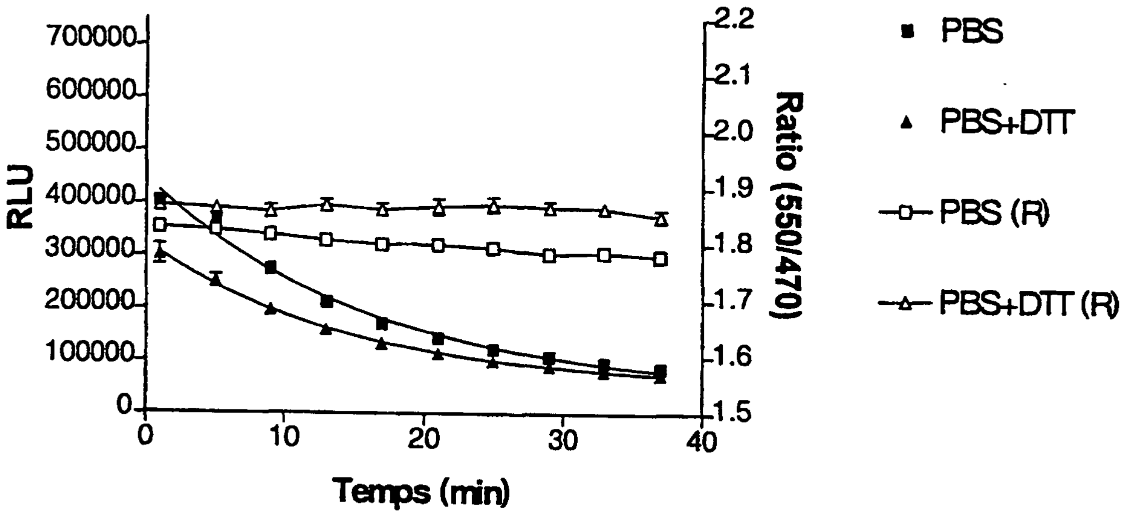
RLU
700000
600000
500000
400000
300000
200000
100000
0
0
10
20
30
40
Temps (min)
2.2
2.1
2.0
1.9
1.8
1.7
1.6
1.5
Ratio (550/470)
PBS
PBS+DTT
PBS (R)
PBS+DTT (R)

FIGURE 57

ATGACTTCGA AAGTTTATGA TCCAGAACAA AGGAAACGGA TGATAACTGG TCCGCAGTGG
TGGGCCAGAT GTAAACAAAT GAATGTTCTT GATTCATTTA TTAATTATTA TGATTCAGAA
AAACATGCAG AAAATGCTGT TATTTTTTTA CATGGTAACG CGGCCTCTTC TTATTTATGG
CGACATGTTG TGCCACATAT TGAGCCAGTA GCGCGGTGTA TTATACCAGA CCTTATTGGT
ATGGGCAAAT CAGGCAAATC TGGTAATGGT TCTTATAGGT TACTTGATCA TTACAAATAT
CTTACTGCAT GGTTTGAACT TCTTAATTTA CCAAAGAAGA TCATTTTTGT CGGCCATGAT
TGGGGTGCTT GTTTGGCATT TCATTATAGC TATGAGCATC AAGATAAGAT CAAAGCAATA
GTTCACGCTG AAAGTGTAGT AGATGTGATT GAATCATGGG ATGAATGGCC TGATATTGAA
GAAGATATTG CGTTGATCAA ATCTGAAGAA GGAGAAAAAA TGGTTTTGGA GAATAACTTC
TTCGTGGAAA CCATGTTGCC ATCAAAAATC ATGAGAAAGT TAGAACCAGA AGAATTTGCA
GCATATCTTG AACCATTCAA AGAGAAAGGT GAAGTTCGTC GTCCAACATT ATCATGGCCT
CGTGAAATCC CGTTAGTAAA AGGTGGTAAA CCTGACGTTG TACAAATTGT TAGGAATTAT
AATGCTTATC TACGTGCAAG TGATGATTTA CCAAAAATGT TTATTGAATC GGACCCAGGA
TTCTTTTCCA ATGCTATTGT TGAAGGTGCC AAGAAGTTTC CTAATACTGA ATTTGTCAAA
GTAAAAGGTC TTCATTTTTC GCAAGAAGAT GCACCTGATG AAATGGGAAA ATATATCAAA
TCGTTCGTTG AGCGAGTTCT CAAAAATGAA CAACGGGCCC GGGATCCCCG GGTACCGGTC
GCCACCATGG TGAGCAAGGG CGAGGAGCTG TTCACCGGGG TGGTGCCCAT CCTGGTCGAG
CTGGACGGCG ACGTAAACGG CCACAAGTTC AGCGTGTCCG GCGAGGGCGA GGGCGATGCC
ACCTACGGCA AGCTGACCCT GAAGTTCATC TGCACCACCG GCAAGCTGCC CGTGCCCTGG
CCCACCCTCG TGACCACCTT CGGCTACGGC CTGCAGTGCT TCGCCCGCTA CCCCGACCAC
ATGAAGCAGC ACGACTTCTT CAAGTCCGCC ATGCCCGAAG GCTACGTCCA GGAGCGCACC
ATCTTCTTCA AGGACGACGG CAACTACAAG ACCCGCGCCG AGGTGAAGTT CGAGGGCGAC
ACCCTGGTGA ACCGCATCGA GCTGAAGGGC ATCGACTTCA AGGAGGACGG CAACATCCTG
GGGCACAAGC TGGAGTACAA CTACAACAGC CACAACGTCT ATATCATGGC CGACAAGCAG
AAGAACGGCA TCAAGGTGAA CTTCAAGATC CGCCACAACA TCGAGGACGG CAGCGTGCAG
CTCGCCGACC ACTACCAGCA GAACACCCCC ATCGGCGACG GCCCCGTGCT GCTGCCCGAC
AACCACTACC TGAGCTACCA GTCCGCCCTG AGCAAAGACC CCAACGAGAA GCGCGATCAC
ATGGTCCTGC TGGAGTTCGT GACCGCCGCC GGGATCACTC TCGGCATGGA CGAGCTGTAC
AAGTAA

FIGURE 58

ATGACTTCGA AAGTTTATGA TCCAGAACAA AGGAAACGGA TGATAACTGG TCCGCAGTGG
TGGGCCAGAT GTAAACAAAT GAATGTTCTT GATTCATTTA TTAATTATTA TGATTCAGAA
AAACATGCAG AAAATGCTGT TATTTTTTTA CATGGTAACG CGGCCTCTTC TTATTTATGG
CGACATGTTG TGCCACATAT TGAGCCAGTA GCGCGGTGTA TTATACCAGA CCTTATTGGT
ATGGGCAAAT CAGGCAAATC TGGTAATGGT TCTTATAGGT TACTTGATCA TTACAAATAT
CTTACTGCAT GGTTTGAACT TCTTAATTTA CCAAAGAAGA TCATTTTTGT CGGCCATGAT
TGGGGTGCTT GTTTGGCATT TCATTATAGC TATGAGCATC AAGATAAGAT CAAAGCAATA
GTTCACGCTG AAAGTGTAGT AGATGTGATT GAATCATGGG ATGAATGGCC TGATATTGAA
GAAGATATTG CGTTGATCAA ATCTGAAGAA GGAGAAAAAA TGGTTTTGGA GAATAACTTC
TTCGTGGAAA CCATGTTGCC ATCAAAAATC ATGAGAAAGT TAGAACCAGA AGAATTTGCA
GCATATCTTG AACCATTCAA AGAGAAAGGT GAAGTTCGTC GTCCAACATT ATCATGGCCT
CGTGAAATCC CGTTAGTAAA AGGTGGTAAA CCTGACGTTG TACAAATTGT TAGGAATTAT
AATGCTTATC TACGTGCAAG TGATGATTTA CCAAAAATGT TTATTGAATC GGACCCAGGA
TTCTTTTCCA ATGCTATTGT TGAAGGTGCC AAGAAGTTTC CTAATACTGA ATTTGTCAAA
GTAAAAGGTC TTCATTTTTC GCAAGAAGAT GCACCTGATG AAATGGGAAA ATATATCAAA
TCGTTCGTTG AGCGAGTTCT CAAAAATGAA CAACGGGCCC GGGATCCGGG CGACGATGAC
GATAAGTTGG CGGTACCGGT CGCCACCATG GTGAGCAAGG GCGAGGAGCT GTTCACCGGG
GTGGTGCCCA TCCTGGTCGA GCTGGACGGC GACGTAAACG GCCACAAGTT CAGCGTGTCC
GGCGAGGGCG AGGGCGATGC CACCTACGGC AAGCTGACCC TGAAGTTCAT CTGCACCACC
GGCAAGCTGC CCGTGCCCTG GCCCACCCTC GTGACCACCT TCGGCTACGG CCTGCAGTGC
TTCGCCCGCT ACCCCGACCA CATGAAGCAG CACGACTTCT TCAAGTCCGC CATGCCCGAA
GGCTACGTCC AGGAGCGCAC CATCTTCTTC AAGGACGACG GCAACTACAA GACCCGCGCC
GAGGTGAAGT TCGAGGGCGA CACCCTGGTG AACCGCATCG AGCTGAAGGG CATCGACTTC
AAGGAGGACG GCAACATCCT GGGGCACAAG CTGGAGTACA ACTACAACAG CCACAACGTC
TATATCATGG CCGACAAGCA GAAGAACGGC ATCAAGGTGA ACTTCAAGAT CCGCCACAAC
ATCGAGGACG GCAGCGTGCA GCTCGCCGAC CACTACCAGC AGAACACCCC CATCGGCGAC
GGCCCCGTGC TGCTGCCCGA CAACCACTAC CTGAGCTACC AGTCCGCCCT GAGCAAAGAC
CCCAACGAGA AGCGCGATCA CATGGTCCTG CTGGAGTTCG TGACCGCCGC CGGGATCACT
CTCGGCATGG ACGAGCTGTA CAAGTAAA

FIGURE 59

ATGACTTCGA AAGTTTATGA TCCAGAACAA AGGAAACGGA TGATAACTGG TCCGCAGTGG
TGGGCCAGAT GTAAACAAAT GAATGTTCTT GATTCATTTA TTAATTATTA TGATTCAGAA
AAACATGCAG AAAATGCTGT TATTTTTTTA CATGGTAACG CGGCCTCTTC TTATTTATGG
CGACATGTTG TGCCACATAT TGAGCCAGTA GCGCGGTGTA TTATACCAGA CCTTATTGGT
ATGGGCAAAT CAGGCAAATC TGGTAATGGT TCTTATAGGT TACTTGATCA TTACAAATAT
CTTACTGCAT GGTTTGAACT TCTTAATTTA CCAAAGAAGA TCATTTTTGT CGGCCATGAT
TGGGGTGCTT GTTTGGCATT TCATTATAGC TATGAGCATC AAGATAAGAT CAAAGCAATA
GTTCACGCTG AAAGTGTAGT AGATGTGATT GAATCATGGG ATGAATGGCC TGATATTGAA
GAAGATATTG CGTTGATCAA ATCTGAAGAA GGAGAAAAAA TGGTTTTGGA GAATAACTTC
TTCGTGGAAA CCATGTTGCC ATCAAAAATC ATGAGAAAGT TAGAACCAGA AGAATTTGCA
GCATATCTTG AACCATTCAA AGAGAAAGGT GAAGTTCGTC GTCCAACATT ATCATGGCCT
CGTGAAATCC CGTTAGTAAA AGGTGGTAAA CCTGACGTTG TACAAATTGT TAGGAATTAT
AATGCTTATC TACGTGCAAG TGATGATTTA CCAAAAATGT TTATTGAATC GGACCCAGGA
TTCTTTTCCA ATGCTATTGT TGAAGGTGCC AAGAAGTTTC CTAATACTGA ATTTGTCAAA
GTAAAAGGTC TTCATTTTTC GCAAGAAGAT GCACCTGATG AAATGGGAAA ATATATCAAA
TCGTTCGTTG AGCGAGTTCT CAAAAATGAA CAACGGGCCC GGGATCCGGC CGACGAGGTG
GACGGCGAAT CCGCGGTACC GGTCGCCACC ATGGTGAGCA AGGGCGAGGA GCTGTTCACC
GGGGTGGTGC CCATCCTGGT CGAGCTGGAC GGCGACGTAA ACGGCCACAA GTTCAGCGTG
TCCGGCGAGG GCGAGGGCGA TGCCACCTAC GGCAAGCTGA CCCTGAAGTT CATCTGCACC
ACCGGCAAGC TGCCCGTGCC CTGGCCCACC CTCGTGACCA CCTTCGGCTA CGGCCTGCAG
TGCTTCGCCC GCTACCCCGA CCACATGAAG CAGCACGACT TCTTCAAGTC CGCCATGCCC
GAAGGCTACG TCCAGGAGCG CACCATCTTC TTCAAGGACG ACGGCAACTA CAAGACCCGC
GCCGAGGTGA AGTTCGAGGG CGACACCCTG GTGAACCGCA TCGAGCTGAA GGGCATCGAC
TTCAAGGAGG ACGGCAACAT CCTGGGGCAC AAGCTGGAGT ACAACTACAA CAGCCACAAC
GTCTATATCA TGGCCGACAA GCAGAAGAAC GGCATCAAGG TGAACTTCAA GATCCGCCAC
AACATCGAGG ACGGCAGCGT GCAGCTCGCC GACCACTACC AGCAGAACAC CCCCATCGGC
GACGGCCCCG TGCTGCTGCC CGACAACCAC TACCTGAGCT ACCAGTCCGC CCTGAGCAAA
GACCCCAACG AGAAGCGCGA TCACATGGTC CTGCTGGAGT TCGTGACCGC CGCCGGGATC
ACTCTCGGCA TGGACGAGCT GTACAAGTAA

# FIGURE 60

ATGGTGAGCA AGGGCGAGGA GCTGTTCACC GGGGTGGTGC CCATCCTGGT CGAGCTGGAC
GGCGACGTAA ACGGCCACAA GTTCAGCGTG TCCGGCGAGG GCGAGGGCGA TGCCACCTAC
GGCAAGCTGA CCCTGAAGTT CATCTGCACC ACCGGCAAGC TGCCCGTGCC CTGGCCCACC
CTCGTGACCA CCTTCGGCTA CGGCCTGCAG TGCTTCGCCC GCTACCCCGA CCACATGAAG
CAGCACGACT TCTTCAAGTC CGCCATGCCC GAAGGCTACG TCCAGGAGCG CACCATCTTC
TTCAAGGACG ACGGCAACTA CAAGACCCGC GCCGAGGTGA AGTTCGAGGG CGACACCCTG
GTGAACCGCA TCGAGCTGAA GGGCATCGAC TTCAAGGAGG ACGGCAACAT CCTGGGGCAC
AAGCTGGAGT ACAACTACAA CAGCCACAAC GTCTATATCA TGGCCGACAA GCAGAAGAAC
GGCATCAAGG TGAACTTCAA GATCCGCCAC AACATCGAGG ACGGCAGCGT GCAGCTCGCC
GACCACTACC AGCAGAACAC CCCCATCGGC GACGGCCCCG TGCTGCTGCC CGACAACCAC
TACCTGAGCT ACCAGTCCGC CCTGAGCAAA GACCCCAACG AGAAGCGCGA TCACATGGTC
CTGCTGGAGT TCGTGACCGC CGCCGGGATC ACTCTCGGCA TGGACGAGCT GTACAAGAGA
TCTATGACCA TGGACTCTGG AGCAGACAAC CAGCAGAGTG GAGATGCTGC TGTAACAGAA
GCTGAAAGTC AACAAATGAC AGTTCAAGCC CAGCCACAGA TTGCCACATT AGCCCAGGTA
TCCATGCCAG CAGCTCATGC GACGTCATCT GCTCCCACTG TAACCTTAGT GCAGCTGCCC
AATGGGCAGA CAGTCCAGGT CCATGGGGTC ATCCAGGCGG CCCAGCCATC AGTTATTCAG
TCTCCACAAG TCCAAACAGT TCAGTCTTCC TGTAAGGACT TAAAAAGACT TTTCTCCGGA
ACTCAGATTT CAACTATTGC AGAAAGTGAA GATTCACAGG AGTCTGTGGA TAGTGTAACT
GATTCCCAAA AACGAAGGGA AATCCTTTCA AGGAGGCCTT CCTACAGGAA AATTTTGAAT
GACTTATCTT CTGATGCACC AGGGGTGCCA AGGATTGAAG AAGAAAAATC AGAAGAAGAG
ACTTCAGCCC CTGCCATCAC CACTGTAACA GTGCCAACCC CGATTTACCA AACTAGCAGT
GGGCAGTATA TTGCCATTAC CCAGGGAGGA GCAATACAGC TGGCTAACAA TGGTACCGAT
GGGGTACAGG GCCTGCAGAC ATTAACCATG ACCAATGCAG CTGCCACTCA GCCGGGTACT
ACCATTCTAC AATATGCACA GACCACTGAT GGACAGCAGA TTCTAGTGCC CAGCAACCAA
GTTGTTGTTC AAGCTGCCTC TGGTGATGTA CAAACATACC AGATTCGCAC AGCACCCACT
AGCACCATTG CCCCTGGAGT TGTTATGGCG TCCTCCCCAG CACTTCCTAC ACAGCCTGCT
GAAGAAGCAG CAAAGCTTGG TACCGAGCTC GGATCCCCCA AGAAGAAGAG GAAGGTGTGA

FIGURE 61

ATGACTTCGA AAGTTTATGA TCCAGAACAA AGGAAACGGA TGATAACTGG TCCGCAGTGG
TGGGCCAGAT GTAAACAAAT GAATGTTCTT GATTCATTTA TTAATTATTA TGATTCAGAA
AAACATGCAG AAAATGCTGT TATTTTTTTA CATGGTAACG CGGCCTCTTC TTATTTATGG
CGACATGTTG TGCCACATAT TGAGCCAGTA GCGCGGTGTA TTATACCAGA CCTTATTGGT
ATGGGCAAAT CAGGCAAATC TGGTAATGGT TCTTATAGGT TACTTGATCA TTACAAATAT
CTTACTGCAT GGTTTGAACT TCTTAATTTA CCAAAGAAGA TCATTTTTGT CGGCCATGAT
TGGGGTGCTT GTTTGGCATT TCATTATAGC TATGAGCATC AAGATAAGAT CAAAGCAATA
GTTCACGCTG AAAGTGTAGT AGATGTGATT GAATCATGGG ATGAATGGCC TGATATTGAA
GAAGATATTG CGTTGATCAA ATCTGAAGAA GGAGAAAAAA TGGTTTTGGA GAATAACTTC
TTCGTGGAAA CCATGTTGCC ATCAAAAATC ATGAGAAAGT TAGAACCAGA AGAATTTGCA
GCATATCTTG AACCATTCAA AGAGAAAGGT GAAGTTCGTC GTCCAACATT ATCATGGCCT
CGTGAAATCC CGTTAGTAAA AGGTGGTAAA CCTGACGTTG TACAAATTGT TAGGAATTAT
AATGCTTATC TACGTGCAAG TGATGATTTA CCAAAAATGT TTATTGAATC GGACCCAGGA
TTCTTTTCCA ATGCTATTGT TGAAGGTGCC AAGAAGTTTC CTAATACTGA ATTTGTCAAA
GTAAAAGGTC TTCATTTTTC GCAAGAAGAT GCACCTGATG AAATGGGAAA ATATATCAAA
TCGTTCGTTG AGCGAGTTCT CAAAAATGAA CAACGGGCCC GGGATCCCCG GGTACCGACA
GGGCAACAGA ATGCCACTTC TTTAAGTAAC CCAAATCCCA TAGACCCCAG CTCCATGCAG
CGAGCCTATG CTGCTCTCGG ACTCCCCTAC ATGAACCAGC CCCAGACGCA GCTGCAGCCT
CAGGTTCCTG GCCAGCAACC AGCACAGCCT CAAACCCACC AGCAGATGAG GACTCTCAAC
CCCCTGGGAA ATAATCCAAT GAACATTCCA GCAGGAGGAA TAACAACAGA TCAGCAGCCC
CCAAACTTGA TTTCAGAATC AGCTCTTCCG ACTTCCCTGG GGGCCACAAA CCCACTGATG
AACGATGGCT CCAACTCTGG TAACATTGGA ACCCTCAGCA CTATACCAAC AGCAGCTCCT
CCTTCTAGCA CCGGTGTAAG GAAAGGCTGG CACGAACATG TCACTCAGGA CCTGCGGAGC
CATCTAGTGC ATAAACTCGT CCAAGCCATC TTCCCAACAC CTGATCCCGC AGCTCTAAAG
GATCGCCGCA TGGAAAACCT GGTAGCCTAT GCTAAGAAAG TGGAAGGGGA CATGTACGAG
TCTGCCAACA GCAGGGATGA ATATTATCAC TTATTAGCAG AGAAAATCTA CAAGATAGCG
GCCGCTCGAG AATTCCCCAA GAAGAAGAGG AAGGTGTAG

FIGURE 62

ATGGTGAGCA AGGGCGAGGA GCTGTTCACC GGGGTGGTGC CCATCCTGGT CGAGCTGGAC
GGCGACGTAA ACGGCCACAA GTTCAGCGTG TCCGGCGAGG GCGAGGGCGA TGCCACCTAC
GGCAAGCTGA CCCTGAAGTT CATCTGCACC ACCGGCAAGC TGCCCGTGCC CTGGCCCACC
CTCGTGACCA CCTTCGGCTA CGGCCTGCAG TGCTTCGCCC GCTACCCCGA CCACATGAAG
CAGCACGACT TCTTCAAGTC CGCCATGCCC GAAGGCTACG TCCAGGAGCG CACCATCTTC
TTCAAGGACG ACGGCAACTA CAAGACCCGC GCCGAGGTGA AGTTCGAGGG CGACACCCTG
GTGAACCGCA TCGAGCTGAA GGGCATCGAC TTCAAGGAGG ACGGCAACAT CCTGGGGCAC
AAGCTGGAGT ACAACTACAA CAGCCACAAC GTCTATATCA TGGCCGACAA GCAGAAGAAC
GGCATCAAGG TGAACTTCAA GATCCGCCAC AACATCGAGG ACGGCAGCGT GCAGCTCGCC
GACCACTACC AGCAGAACAC CCCCATCGGC GACGGCCCCG TGCTGCTGCC CGACAACCAC
TACCTGAGCT ACCAGTCCGC CCTGAGCAAA GACCCCAACG AGAAGCGCGA TCACATGGTC
CTGCTGGAGT TCGTGACCGC CGCCGGGATC ACTCTCGGCA TGGACGAGCT GTACAAGAGA
TCTCCCAAGA ACTACATAGA AATGAAACCA CATCCGTGGT TTTTTGGCAA AATCCCCAGA
GCCAAGGCAG AAGAAATGCT TAGCAAACAG CGGCACGATG GGGCCTTTCT TATCCGAGAG
AGTGAGAGCG CTCCTGGGGA CTTCTCCCTC TCTGTCAAGT TTGGAAACGA TGTGCAGCAC
TTCAAGGTGC TCCGAGATGG AGCCGGGAAG TACTTCCTCT GGGTGGTGAA GTTCAATTCT
TTGAATGAGC TGGTGGATTA TCACAGATCT ACATCTGTCT CCAGAAACCA GCAGATATTC
CTGCGGGACA TAGAACAGGT GCCACAGCAG CCGACATACG TCCAGGCCCT CTGA

FIGURE 63

ATGACTTCGA AAGTTTATGA TCCAGAACAA AGGAAACGGA TGATAACTGG TCCGCAGTGG
TGGGCCAGAT GTAAACAAAT GAATGTTCTT GATTCATTTA TTAATTATTA TGATTCAGAA
AAACATGCAG AAAATGCTGT TATTTTTTTA CATGGTAACG CGGCCTCTTC TTATTTATGG
CGACATGTTG TGCCACATAT TGAGCCAGTA GCGCGGTGTA TTATACCAGA CCTTATTGGT
ATGGGCAAAT CAGGCAAATC TGGTAATGGT TCTTATAGGT TACTTGATCA TTACAAATAT
CTTACTGCAT GGTTTGAACT TCTTAATTTA CCAAAGAAGA TCATTTTTGT CGGCCATGAT
TGGGGTGCTT GTTTGGCATT TCATTATAGC TATGAGCATC AAGATAAGAT CAAAGCAATA
GTTCACGCTG AAAGTGTAGT AGATGTGATT GAATCATGGG ATGAATGGCC TGATATTGAA
GAAGATATTG CGTTGATCAA ATCTGAAGAA GGAGAAAAAA TGGTTTTGGA GAATAACTTC
TTCGTGGAAA CCATGTTGCC ATCAAAAATC ATGAGAAAGT TAGAACCAGA AGAATTTGCA
GCATATCTTG AACCATTCAA AGAGAAAGGT GAAGTTCGTC GTCCAACATT ATCATGGCCT
CGTGAAATCC CGTTAGTAAA AGGTGGTAAA CCTGACGTTG TACAAATTGT TAGGAATTAT
AATGCTTATC TACGTGCAAG TGATGATTTA CCAAAAATGT TTATTGAATC GGACCCAGGA
TTCTTTTCCA ATGCTATTGT TGAAGGTGCC AAGAAGTTTC CTAATACTGA ATTTGTCAAA
GTAAAAGGTC TTCATTTTTC GCAAGAAGAT GCACCTGATG AAATGGGAAA ATATATCAAA
TCGTTCGTTG AGCGAGTTCT CAAAAATGAA CAACGGGCCC GGGATCCCCG GGTACCGATG
AACAAGCTGA GTGGAGGCGG CGGGCGCAGG ACTCGGGTGG AAGGGGGCCA GCTTGGGGGC
GAGGAGTGGA CCCGCCACGG GAGCTTTGTC AATAAGCCCA CGCGGGGCTG GCTGCATCCC
AACGACAAAG TCATGGGACC CGGGGTTTCC TACTTGGTTC GGTACATGGG TTGTGTGGAG
GTCCTCCAGT CAATGCGTGC CCTGGACTTC AACACCCGGA CTCAGGTCAC CAGGGAGGCC
ATCAGTCTGG TGTGTGAGGC TGTGCCGGGT GCTAAGGGGG CGACAAGGAG GAGAAAGCCC
TGTAGCCGCC CGCTCAGCTC TATCCTGGGG AGGAGTAACC TGAAATTTGC TGGAATGCCA
ATCACTCTCA CCGTCTCCAC CAGCAGCCTC AACCTCATGG CCGCAGACTG CAAACAGATC
ATCGCCAACC ACCACATGCA ATCTATCTCA TTTGCATCCG GCGGGGATCC GGACACAGCC
GAGTATGTCG CCTATGTTGC CAAAGACCCT GTGAATCAGA GAGCCTGCCA CATTCTGGAG
TGTCCCGAAG GGCTTGCCCA GGATGTCATC AGCACCATTG GCCAGGCCTT CGAGTTGCGC
TTCAAACAAT ACCTCAGGAA CCCACCCAAA CTGGTCACCC CTCATGACAG GATGGCTGGC
TTTGATGGCT CAGCATGGGA TGAGGAGGAG GAAGAGCCAC CTGACCATCA GTACTATAAT
GACTTCCCGG GGAAGGAACC CCCCTTGGGG GGGGTGGTAG ACATGAGGCT TCGGGAAGGA
GCCGCTCCAG GGGCTGCTCG ACCCACTGCA CCCAATGCCC AGACCCCCAG CCACTTGGGA
GCTACATTGC CTGTAGGACA GCCTGTTGGG GGAGATCCAG AAGTCCGCAA ACAGATGCCA
CCTCCACCAC CCTGTCCAGG CAGAGAGCTT TTTGATGATC CCTCCTATGT CAACGTCCAG
AACCTAGACA AGGCCCGGCA AGCAGTGGGT GGTGCTGGGC CCCCCAATCC TGCTATCAAT
GGCAGTGCAC CCCGGGACCT GTTTGACATG AAGCCCTTCG AAGATGCTCT TCGGGTGCCT
CCACCTCCCC AGTCGGTGTC CATGGCTGAG CAGCTCCGAG GGGAGCCCTG A